(19) Europäisches Patentamt
European Patent Office
Office européen des brevets

(11) EP 4 755 923 A1

(12) EUROPEAN PATENT APPLICATION
published in accordance with Art. 153(4) EPC

(43) Date of publication:
**10.06.2026 Bulletin 2026/24**

(21) Application number: **24850477.1**

(22) Date of filing: **07.03.2024**

(51) International Patent Classification (IPC):
***C08B 37/00*** *(2006.01)* ***A61K 31/715*** *(2006.01)*
***A61P 35/00*** *(2006.01)*

(52) Cooperative Patent Classification (CPC):
**A61K 33/243; A61K 31/716; A61K 36/074; C08B 37/0003; C08B 37/0024;** A61K 2236/13; A61K 2236/15; A61K 2236/17; A61K 2236/331; A61K 2236/51

(86) International application number:
**PCT/CN2024/080443**

(87) International publication number:
**WO 2025/030817 (13.02.2025 Gazette 2025/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **04.08.2023 CN 202310984017**

(71) Applicant: **Shenzhen Yandai Investment Co., Ltd**
**Shenzhen, Guangdong 518000 (CN)**

(72) Inventors:
- **CHEN, Xuemin**
  **Shenzhen, Guangdong 518000 (CN)**
- **HE, Chunming**
  **Shenzhen, Guangdong 518000 (CN)**
- **MO, Guan**
  **Shenzhen, Guangdong 518000 (CN)**
- **CHEN, Jin**
  **Shenzhen, Guangdong 518000 (CN)**
- **LI, Yanjun**
  **Shenzhen, Guangdong 518000 (CN)**
- **CHEN, Yao**
  **Shenzhen, Guangdong 518000 (CN)**
- **CHEN, Fengji**
  **Shenzhen, Guangdong 518000 (CN)**
- **LIANG, Mei**
  **Shenzhen, Guangdong 518000 (CN)**
- **FANG, Zhe**
  **Shenzhen, Guangdong 518000 (CN)**
- **SU, Yuanye**
  **Shenzhen, Guangdong 518000 (CN)**

(74) Representative: **Metida**
**Gyneju str. 16**
**01109 Vilnius (LT)**

# (54) POLYSACCHARIDE COMPOUND HAVING DEFINITE MOLECULAR STRUCTURE AND CAPABLE OF ELIMINATING TOXIC AND SIDE EFFECTS OF CHEMOTHERAPEUTIC DRUGS

(57) This invention pertains to the field of plant extraction and separation technology and provides a polysaccharide compound with a well-defined molecular structure. The polysaccharide compound is suitable for treating patients with advanced cancer (where surgical options are no longer viable, life expectancy is only three to six months, and chemotherapy remains feasible), including the following steps of: S1. dust removal, drying, and crushing of Ganoderma lucidum; S2. mixing with water in a sealed container and extracting an active ingredient under high temperature and high pressure; S3. removing the residues from the concentrated extract containing the active ingredient; and S4. formulating the residue-free concentrate to a specific concentration and isolating polysaccharide with the active ingredient by column chromatography. The polysaccharide with this active ingredient, characterized by its water solubility, is readily absorbed by the human body, offers antitumor effects, and has proven efficacy in the prevention of tumor development in humans. Notably, when used in combination with chemotherapy drugs, the polysaccharide can alleviate toxic side effects caused by the chemotherapy drugs on the human body and has been supported by experimental data showing control and reduction of tumor masses, and reduction and elimination of cancer cells.

EP 4 755 923 A1

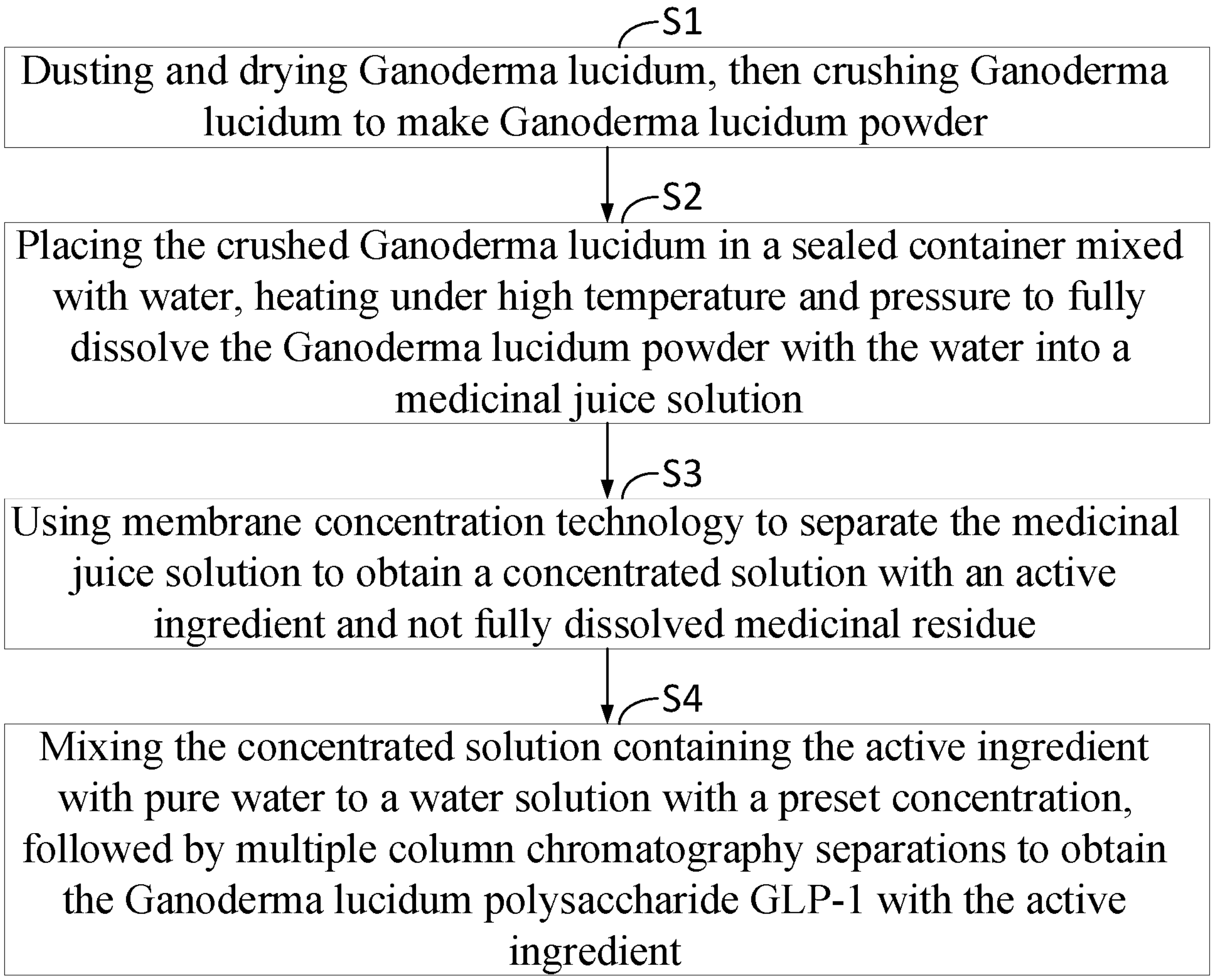
S1
Dusting and drying Ganoderma lucidum, then crushing Ganoderma lucidum to make Ganoderma lucidum powder
S2
Placing the crushed Ganoderma lucidum in a sealed container mixed with water, heating under high temperature and pressure to fully dissolve the Ganoderma lucidum powder with the water into a medicinal juice solution
S3
Using membrane concentration technology to separate the medicinal juice solution to obtain a concentrated solution with an active ingredient and not fully dissolved medicinal residue
S4
Mixing the concentrated solution containing the active ingredient with pure water to a water solution with a preset concentration, followed by multiple column chromatography separations to obtain the Ganoderma lucidum polysaccharide GLP-1 with the active ingredient

Fig. 1

## Description

### Technical Field

The present invention relates to the field of improvements in extraction and separation technology, specifically relating to a method for extracting Ganoderma lucidum polysaccharide GLP-1 and its applications.

### Background

Ganoderma lucidum is a type of fungal plant with a long history of medicinal use in China and Japan. Ganoderma lucidum has a plurality of complex active ingredients, and over 150 compounds have been isolated from it, such as polysaccharides, triterpenes, sterols, alkaloids, furan derivatives, amino peptides, and inorganic elements. Geographic location (longitude and latitude), seed variation, growth environment, and differences in temperature, humidity, and light intensity can significantly affect the content, proportion, and presence of an active ingredient referred to in this invention in Ganoderma lucidum.

### Summary of Invention

The invention provides an improved method and application for extracting polysaccharide compounds with defined molecular structures and pharmacological functions from raw Ganoderma lucidum materials.
This invention aims to address the unmet international challenges of treating patients with advanced cancer, where "patients with advanced cancer" are defined as: those who are no longer surgical candidates, have a life expectancy of only three to six months, and are still eligible for chemotherapy; the "challenges of treating patients" refer to: allowing patients with advanced cancer to regain their appetite quickly (typically within two to three weeks), reducing or stabilizing tumor size, and using in combination with chemotherapy drugs to essentially eliminate the toxic side effects induced by the chemotherapy drugs on the human body. Long-term combination use with the chemotherapy drugs can achieve the objective of substantially eradicating cancer cells or keeping the number of cancer cells within safe limits for high-quality human survival.
Another important function of the active ingredients specified in this invention is the prevention of mutations in normal human cells and the prevention of cancer cell formation.
Furthermore, the active ingredient in this invention, when used in combination with the chemotherapy drugs, exhibit exceptionally good therapeutic effects on lung cancer, liver cancer, and breast cancer, demonstrating a certain degree of broad-spectrum activity.
The invention provides a method for extracting Ganoderma lucidum polysaccharide GLP-1, comprising the steps of:

S1. dusting and drying Ganoderma lucidum, then crushing Ganoderma lucidum to make Ganoderma lucidum powder;
S2. placing the crushed Ganoderma lucidum powder in a sealed container mixed with water, heating under high temperature and pressure to fully dissolve the Ganoderma lucidum powder with the water into a medicinal juice solution;
S3. using membrane concentration technology to separate the medicinal juice solution to obtain a concentrated solution with an active ingredient and not fully dissolved medicinal residue; and
S4. mixing the concentrated solution containing the active ingredient with pure water to a water solution with a preset concentration, followed by multiple column chromatography separations to obtain the Ganoderma lucidum polysaccharide GLP-1 with the active ingredient.

A further aspect of the invention: In step S2, the mixture of the Ganoderma lucidum powder and the water in the sealed container is fully stirred and heated at a high temperature to 105-200°C, with boiling time lasting for 2-6 h, during which the internal pressure in the sealed container gradually increases as the heating temperature rises, forming a high-temperature and high-pressure environment within the sealed container.
A further aspect of the invention: In step S2, the mixture of the Ganoderma lucidum powder and the water in the sealed container is heated at a high temperature to 105-170°C, with boiling time lasting for 3-6 h, during which the internal pressure in the sealed container gradually increases as the heating temperature rises, forming the high-temperature and high-pressure environment within the sealed container.
A further aspect of the invention: In step S4, the concentrated liquid containing the active ingredient is mixed with the pure water at a concentration ratio of 1:2 to 1:5.
A further aspect of the invention: In step S3, Ganoderma lucidum residue is removed from the extracted water

solution containing the active ingredient by using the membrane concentration technology, obtaining a concentrated liquid or paste containing the active ingredient.

A further aspect of the invention: In step S1, the Ganoderma lucidum is rinsed with clean water to remove surface dust and dried at 105°C, and the dried Ganoderma lucidum is crushed, with the crushed Ganoderma lucidum powder being larger than 60 mesh.

A further aspect of the invention: In step S2, the mixed liquid in the sealed container is heated at a high temperature to 105°C, 110°C, 115°C, 120°C, 125°C, 130°C, 135°C, 140°C, 145°C, 150°C, 155°C, 160°C, 165°C, 170°C, 175°C, 180°C, 185°C, 190°C, 195°C, or 200°C, with boiling times of 2 h, 2.5 h, 3 h, 3.5 h, 4 h, 4.5 h, 5 h, 5.5 h, or 6 h, during which the internal pressure in the sealed container gradually increases as the heating temperature rises, forming the high-temperature and high-pressure environment within the sealed container.

The invention also provides the Ganoderma lucidum polysaccharide GLP-1, wherein the structural formula of the Ganoderma lucidum polysaccharide GLP-1 is

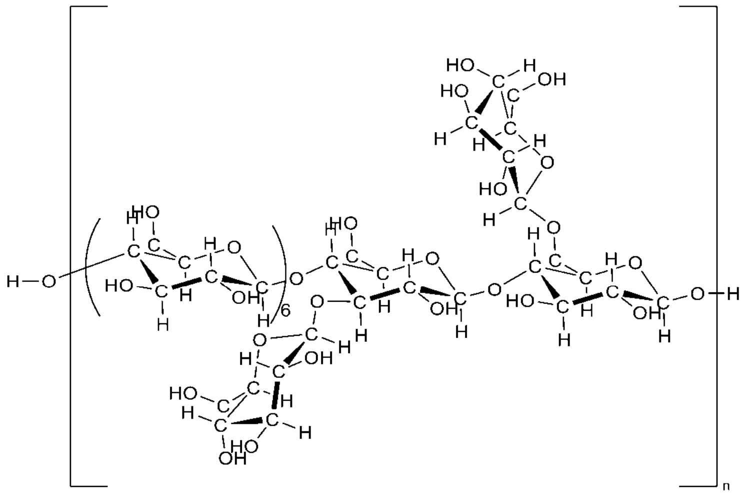

;

the molecular formula is $(C_{60}H_{100}O_{50})n$, where n = 17-25.

A further aspect of the invention: n is 17, 18, 19, 20, 21, 22, 23, 24, or 25.

This invention also provides the application of the Ganoderma lucidum polysaccharide GLP-1. The Ganoderma lucidum polysaccharide GLP-1 is characterized by its water solubility, is readily absorbed by the human body, offers antitumor effects, and has proven efficacy in the prevention of tumor development in humans. Notably, when used in combination with chemotherapy drugs, it can alleviate toxic side effects caused by the chemotherapy drugs on the human body, control and reduce tumor masses, and reduce and eliminate cancer cells.

The beneficial effects of this invention include a simple extraction process, high polysaccharide yield, low production cost, and ease of industrialization. The resultant Ganoderma lucidum polysaccharide GLP-1 is highly soluble and readily absorbed by the human body, thereby exerting its antitumor, antioxidant, and anti-aging effects.

Brief Description of the Drawings

Figure 1: Flowchart of the method for extracting Ganoderma lucidum polysaccharide GLP-1, provided by an embodiment of the invention.

Figure 2: Schematic diagram of lgMp-RT (peak molecular weight) calibration curves provided by an embodiment of the invention.

Figure 3: Schematic diagram of lgMp-RT (weight-average molecular weight) calibration curvew provided by an embodiment of the invention.

Figure 4: Schematic diagram of lgMp-RT (number-average molecular weight) calibration curves provided by an embodiment of the invention.

Figure 5: Schematic diagram of the molecular weight spectrum of the sample provided by an embodiment of the

invention.

Figure 6: Schematic diagram 1 of the ion chromatography of mixed standard 16 sugars, provided by an embodiment of the invention.

Figure 7: Schematic diagram 2 of the ion chromatography of mixed standard 16 sugars, provided by an embodiment of the invention.

Figure 8: GCMS chromatogram of the sample (PMAA) provided by an embodiment of the invention.

Figure 9: Schematic diagram 1 of the analysis results of permethylated alditol acetate (PMAA) of polysaccharide, provided by an embodiment of the invention.

Figure 10: Schematic diagram 2 of the analysis results of permethylated alditol acetate (PMAA) of polysaccharide, provided by an embodiment of the invention.

Figure 11: Schematic diagram 3 of the analysis results of permethylated alditol acetate (PMAA) of polysaccharide, provided by an embodiment of the invention.

Figure 12: Schematic diagram 4 of the analysis results of permethylated alditol acetate (PMAA) of polysaccharide, provided by an embodiment of the invention.

Figure 13: Schematic diagram of the hydrogen spectrum provided by an embodiment of the invention.

Figure 14: Schematic diagram of the carbon spectrum provided by an embodiment of the invention.

Figure 15: Schematic diagram of the Dept135 spectrum provided by an embodiment of the invention.

Figure 16: Schematic diagram of HH-COSY provided by an embodiment of the invention.

Figure 17: Schematic diagram of HSQC provided by an embodiment of the invention.

Figure 18: HMBC spectrum provided by an embodiment of the invention.

Figure 19: Schematic diagram of NOESY provided by an embodiment of the invention.

Figure 20: Schematic diagram of the effect of Ganoderma lucidum polysaccharide GLP-1 combined with chemotherapy on the tumor volume in LLC-bearing mice, provided by an embodiment of the invention.

Figure 21: Schematic diagram of the effect of Ganoderma lucidum polysaccharide GLP-1 combined with chemotherapy on the tumor pathology in LLC-bearing mice (×200), provided by an embodiment of the invention.

Figure 22: Schematic diagram of the effect of Ganoderma lucidum polysaccharide GLP-1 combined with chemotherapy on the renal pathology in LLC-bearing mice (×200), provided by an embodiment of the invention.

Figure 23: Schematic diagram of the effect of Ganoderma lucidum polysaccharide GLP-1 combined with chemotherapy on the gastric pathology in LLC-bearing mice (×200), provided by an embodiment of the invention.

Figure 24: Schematic diagram of the effect of Ganoderma lucidum polysaccharide GLP-1 combined with chemotherapy on the spleen pathology in LLC-bearing mice (×200), provided by an embodiment of the invention.

Figure 25: Schematic diagram of the effect of Ganoderma lucidum polysaccharide GLP-1 combined with radiotherapy on the tumor volume in LLC-bearing mice, provided by an embodiment of the invention.

Figure 26: Schematic diagram of the effect of Ganoderma lucidum polysaccharide GLP-1 combined with radiotherapy on the thymus tissue pathology in LLC-bearing mice (×200), provided by an embodiment of the invention.

Figure 27: Schematic diagram of the effect of Ganoderma lucidum polysaccharide GLP-1 combined with chemotherapy on the spleen tissue pathology in LLC-bearing mice (×200), provided by an embodiment of the invention.

Figure 28: Schematic diagram of the effect of Ganoderma lucidum polysaccharide GLP-1 combined with chemotherapy on the nasal tissue pathology in LLC-bearing mice (×200), provided by an embodiment of the invention.

Figure 29: Schematic diagram of the effect of Ganoderma lucidum polysaccharide GLP-1 combined with chemotherapy on the gastric tissue pathology in LLC-bearing mice (×200), provided by an embodiment of the invention.

Figure 30: Schematic diagram of the effect of Ganoderma lucidum polysaccharide GLP-1 combined with chemotherapy on the renal tissue pathology in LLC-bearing mice (×200), provided by an embodiment of the invention.

Figures 31, 32, 33, and 34: Schematic diagrams of the effect of Ganoderma lucidum polysaccharide GLP-1 combined with chemotherapy on H22-bearing mice, provided by an embodiment of the invention.

Figures 35, 36, 37, and 38: External appearance schematic diagrams of the effect of Ganoderma lucidum polysaccharide GLP-1 combined with chemotherapy on H22-bearing mice, provided by an embodiment of the invention.

Figures 39, 40, 41 and 42: Schematic diagrams of the effect of Ganoderma lucidum polysaccharide GLP-1 combined with chemotherapy on 4T1 breast tumor-bearing mice, provided by an embodiment of the invention.

Figures 43, 44, 45, and 46: External appearance schematic diagrams of the effect of Ganoderma lucidum polysaccharide GLP-1 combined with chemotherapy on 4T1 breast tumor-bearing mice, provided by an embodiment of the invention.

Figures 47, 48, 49, 50, 51, and 52 Schematic diagrams of the effect of Ganoderma lucidum polysaccharide GLP-1 combined with cisplatin on LLC cells, provided by an embodiment of the invention.

## Detailed Description of the Embodiments

Embodiments of the present invention are described in detail below, and examples of the embodiments are shown in the drawings, wherein the same or similar reference numerals denote the same or similar elements or elements having the same or similar functions throughout. The embodiments below described by reference to the drawings are exemplary and are intended for the purpose of explaining the invention and should not be construed as limiting the scope of the invention.

As shown in Figure 1, the present invention provides a flowchart for the method for extracting the Ganoderma lucidum polysaccharide GLP-1, described in detail as follows:

In step S1, harvested Ganoderma lucidum, either raw or having undergone preliminary processing, is washed with clean water in washing equipment to remove surface dust. After dust removal, the Ganoderma lucidum is transferred to drying equipment for drying at a temperature of 105°C. The dried Ganoderma lucidum is then placed in a crusher to be broken down into Ganoderma lucidum powder. The Ganoderma lucidum powder is sieved, particles of the Ganoderma lucidum powder larger than 60 mesh are transferred to the next process, while particles of the Ganoderma lucidum powder smaller than 60 mesh are returned to the crusher for further crushing. This process is repeated multiple times until the crushed Ganoderma lucidum powder meets specified requirements.
In step S2, the Ganoderma lucidum powder that meets the requirements is mixed with pure water and placed in a sealed container. The sealed container is heated at high temperatures. As the temperature rises, the internal pressure of the sealed container gradually increases, creating a high-temperature and high-pressure environment that facilitates the thorough dissolution of the Ganoderma lucidum powder with the water to form a mixed solution. The sealed container is heated to a temperature between 105°C and 200°C, with a boiling time of 2-6 h, preferably between 105°C and 170°C, for 3-6 h. More preferably, the heating occurs at temperatures of 105°C, 110°C, 115°C, 120°C, 125°C, 130°C, 135°C, 140°C, 145°C, 150°C, 155°C, 160°C, 165°C, 170°C, 175°C, 180°C, 185°C, 190°C, 195°C, or 200°C, with a boiling time of 2 h, 2.5 h, 3 h, 3.5 h, 4 h, 4.5 h, 5 h, 5.5 h, or 6 h, ensuring full dissolution of the mixture.
In step S3, the water solution extracted, containing the active ingredient, is centrifuged to remove residues. The supernatant is concentrated using a membrane concentration to obtain a concentrated liquid.
In step S4, the concentrated liquid containing the active ingredient is prepared at a specific concentration, separated by column chromatography, to obtain Ganoderma lucidum polysaccharide GLP-1 with the active ingredient.

This method offers a simple extraction process, high polysaccharide yield, low production costs, and simple operation.

The invention also provides the Ganoderma lucidum polysaccharide GLP-1, wherein the structural formula of the Ganoderma lucidum polysaccharide GLP-1 is

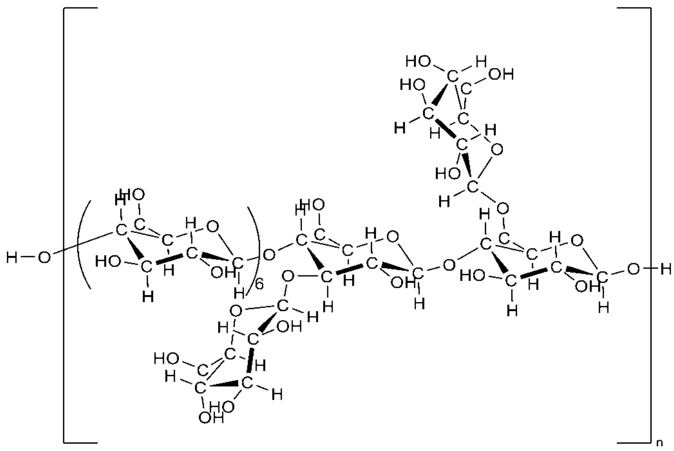

;

the molecular formula is $(C_{60}H_{100}O_{50})n$, where n = 17-25.

n is 17, 18, 19, 20, 21, 22, 23, 24, or 25.

Following the acquisition of the Ganoderma lucidum polysaccharide GLP-1 with the aforementioned structure, assay experiments were conducted and the following results are reported herein.

I. Molecular Weight Determination

1. Experimental Objective

To determine the molecular weight and purity of the polysaccharide using HPGPC.

2. Experimental Materials

2.1 Equipment

| Equipment name | Manufacturer | Model |
|---|---|---|
| High-performance liquid chromatography | Shimadzu | LC-10A |
| Differential refractometer | Shimadzu | RI-10A |
| BRT105-104-102 tandem gel permeation chromatography column | BoRui Saccharide | BRT105-104-102 (8 × 300 mm) |
| Electronic scale | Sartorius | CPA225D |
| Centrifuge | Eppendorf | Eppendorf5424 |
| Pipette | Sartorius | 200 uL, 1,000 uL |

2.2 Materials

| Reagent | Manufacturer | Batch No. | Catalog No. | Grade | Shelf life |
|---|---|---|---|---|---|
| NaCl | ACROS | A0356762 | 139725000 | ACROS | 2022 |

2.3 Standards

| Standard | Manufacturer | Batch No. | Storage conditions | Purity | Shelf life |
|---|---|---|---|---|---|
| Dextranstandards1152 | Yuanye Bio-Technology | A16A8L41850 | Seal and store | 99% | 2 years |
| 5000 | Sigma | 102084138 | Seal and store | ≥99% | 2 years |
| 11600 | Sigma | 102136543 | Seal and store | 99% | 2 years |
| 23800 | Sigma | 102124529 | Seal and store | ≥97% | 2 years |
| 48600 | Sigma | 102104509 | Seal and store | ≥99% | 2 years |
| 80900 | Sigma | 102108375 | Seal and store | >98% | 2 years |
| 148000 | Sigma | 102089360 | Seal and store | >98% | 2 years |
| 273000 | Sigma | 102110878 | Seal and store | 98% | 2 years |
| 409800 | Sigma | 102124507 | Seal and store | >98% | 2 years |
| 667800 | Sigma | 102104510 | Seal and store | >98% | 2 years |
| Molecular weight is measured in Daltons (Da). | | | | | |

3. Experimental Procedure

3.1 Reagent Preparation

| Reagent name | Preparation method | Storage conditions | Shelf life |
|---|---|---|---|
| 0.05M NaCl solution | Precisely prepared, filtered through a 0.45-μm membrane, degassed by sonication for 10 min | RT | 1 month |

3.2 Preparation of Sample and Standard Solutions

Samples and standards were precisely weighed to prepare a 5 mg/mL solution, which was centrifuged at 12,000 rpm for 10 min, and the supernatant was filtered through a 0.22-μm micropore filter. Then the sample was transferred to a 1.8-mL sample vial.

3.3 Chromatographic Method

Column: BRT105-104-102 tandem gel permeation chromatography column (8 × 300 mm); Mobile phase: 0.05M NaCl solution; Flow rate: 0.6 mL/min; Column temperature: 40°C; Injection volume: 20 μL; Detector: Differential refractometer RI-10A.

4. Experimental Results

As shown in Figures 2-4, calibration curves for lgMp-RT (peak molecular weight), lgMw-RT (weight-average molecular weight), and lgMn-RT (number-average molecular weight) were obtained.

The equation of the calibration curves for lgMp-RT is: $y = -0.1802x + 11.661$ $R2 = 0.9928$;
The equation of the calibration curves for lgMw-RT is: $y = -0.1926x + 12.241$ $R2 = 0.9965$;
The equation of the calibration curves for lgMn-RT is: $y = -0.1783x + 11.506$ $R2 = 0.9911$;

Using the standard curves, a formula was derived to calculate the molecular weight of each sample. The molecular weight chromatograms for the samples are shown in Figure 5, with the results detailed in the table below.

| Sample ID | RT (min) | lgMp | lgMw | lgMn | Mp | Mw | Mn | Peak Area Ratio (%) |
|---|---|---|---|---|---|---|---|---|
| | 39.67 | 4.5 | 4.6 | 4.4 | 32544 | 39862 | 27092 | 100 |
| Note: The peak at 46.5 min corresponds to the mobile phase. | | | | | | | | |

II. Monosaccharide Composition Determination Experiment

1. Experimental Objective

To determine the monosaccharide composition using an ion chromatograph.

2. Experimental Principle

This method is based on the electrochemical activity of sugar molecules and their ionization in strong alkaline solutions. Sugar compounds are weak acids with a pKa greater than 11. In high pH eluents, they partially or fully exist as anions. Efficient anion exchange and separation of sugar compounds is achieved, which leverages differences in ion exchange due to variations in pKa of different sugars, as well as differences in hydrophobic interactions between some sugars and the anion exchange resin. Detection is then accomplished by measuring the current produced by the oxidation of hydroxyl groups in the sugar molecules at a gold electrode surface.

3. Experimental Materials

3.1 Equipment

| Equipment name | Manufacturer | Model |
|---|---|---|
| Ion chromatograph | ThermoFisher | ICS5000 |
| Electric thermostatic blast drying oven | LICHEN | 101-1BS |
| Nitrogen blower | LICHEN | UGC-24M |
| Electronic scale | Sartorius | BS 210 S |
| Centrifuge | ThermoFisher | D-37520 |
| Pipette | DRAGONLAB | 19050983 |

3.2 Reagents

| Reagent | Manufacturer | Batch No. | Catalog No. | Grade |
|---|---|---|---|---|
| Trifluoroacetic acid | ACROS | A0356762 | 139725000 | AR |
| 50% sodium hydroxide solution | Alfa Aesar | Z21E036 | 33382 | GR |
| Sodium acetate | ThermoFishe | 191126 | 059326 | GR |

3.3 Standards

| Standard | Manufacturer | Batch No. | Storage conditions | Purity |
|---|---|---|---|---|
| Mannose | Bo Rui Saccharide | C17D9H77586 | Seal and store | AR |
| Rhamnose | Bo Rui Saccharide | H10S9Z69863 | Seal and store | AR |

(continued)

| Standard | Manufacturer | Batch No. | Storage conditions | Purity |
|---|---|---|---|---|
| Galacturonic acid | Bo Rui Saccharide | K02A9B66077 | Seal and store | AR |
| Galactose | Bo Rui Saccharide | E1927035 | Seal and store | AR |
| Glucose | Bo Rui Saccharide | Q18F10N80946 | Seal and store | AR |
| Glucuronic acid | Bo Rui Saccharide | K14M10S82777 | Seal and store | AR |
| Arabinose | Bo Rui Saccharide | S15A10G85850 | Seal and store | AR |
| Xylose | Bo Rui Saccharide | A22S6X3606 | Seal and store | AR |
| Fucose | Bo Rui Saccharide | X29D7Y27768 | Seal and store | AR |
| Glucosamine hydrochloride | Bo Rui Saccharide | A22S6X3606 | Seal and store | AR |
| N-acetyl-D-glucosamine | Bo Rui Saccharide | A21J8X40372 | Seal and store | AR |
| D-fructose | Bo Rui Saccharide | J01J10R89818 | Seal and store | AR |
| D-ribose | Bo Rui Saccharide | H26F10Z81556 | Seal and store | AR |
| Galactosamine hydrochloride | Bo Rui Saccharide | B01J8S37079 | Seal and store | AR |
| L-guluronic acid | Bo Rui Saccharide | S200115AG1 | Seal and store | ≥98% |
| D-mannuronic acid | Bo Rui Saccharide | S200108AM1 | Seal and store | ≥98% |

4. Experimental Methods

4.1 Reagent Preparation

| Reagent name | Preparation method | Storage conditions |
|---|---|---|
| 15 mM NaOH solution | 2.4 g of 50% NaOH solution, 2 L of water | RT |
| 15 mM NaOH & 100 mM NaOAc solution | 1.2 g of 50% NaOH solution, 8.2 g of NaOAc, 1 L of water | RT |

4.2 Preparation and Calculation Method for Standard Solutions

Standard stock solutions were prepared using 16 different monosaccharide standards (fucose, rhamnose, arabinose, galactose, glucose, xylose, mannose, fructose, ribose, galacturonic acid, glucuronic acid, glucosamine hydrochloride, galactosamine hydrochloride, N-acetyl-D-glucosamine, guluronic acid, and mannuronic acid).

Concentration standards of each monosaccharide standard solution were accurately prepared and used as a mixed standard. The mass of different monosaccharides was determined using an absolute quantification method and the molar ratios were calculated based on the molar mass of each monosaccharide.

4.3 Sample Preparation

5 mg of the sample was accurately weighed in an ampule. 2 mL of 3M TFA was added and hydrolyzed at 120°C for 3 h. The acid hydrolysate was accurately transferred to a tube and evaporated to dryness under nitrogen. 5 mL water was added, vortexed to mix, and then 50 μL was taken and added to 950 μL of deionized water, and centrifuged at 12,000 rpm for 5 min. The supernatant was transferred for IC analysis.

4.4 Chromatographic Method

Column: Dionex CarbopacTM PA20 (3 × 150 mm); Mobile phase: A: H2O; B: 15 mM NaOH; C: 15 mM NaOH & 100 mM NaOAc; Flow rate: 0.3 mL/min; Injection volume: 5 μL; Column temperature: 30°C; Detector: Electrochemical detector.

4.5 Standard Series

| No. | Name | ppm | Name | RT | Area |
|---|---|---|---|---|---|
| 1 | Fucose | 5 | Fuc | 5.659 | 18.741 |
| 2 | Galactosamine hydrochloride | 3 | GalN | 10.084 | 23.888 |
| 3 | Rhamnose | 5 | Rha | 10.475 | 10.717 |
| 4 | Arabinose | 3.7 | Ara | 11.092 | 16.035 |
| 5 | Glucosamine hydrochloride | 5 | GlcN | 12.367 | 31.057 |
| 6 | Galactose | 5 | Gal | 13.767 | 17.597 |
| 7 | Glucose | 5 | Glc | 15.484 | 20.442 |
| 8 | N-acetyl-D-glucosamine 5 | 5 | GlcNAc | 16.792 | 13.652 |
| 9 | Xylose | 5 | Xyl | 17.834 | 22.737 |
| 10 | Mannose | 5 | Man | 18.117 | 14.734 |
| 11 | Fructose | 15 | Fru | 20.534 | 12.857 |
| 12 | Ribose | 10 | Rib | 22.484 | 26.868 |
| 13 | Galacturonic acid | 5 | GalA | 45.125 | 8.815 |
| 14 | Guluronic acid | 10 | GulA | 45.950 | 20.824 |
| 15 | Glucuronic acid | 5 | GlcA | 48.509 | 11.689 |
| 16 | Mannuronic acid | 10 | ManA | 50.992 | 22.847 |

$$C\ (\text{standard}) / A\ (\text{standard}) = C\ (\text{sample}) / A\ (\text{sample})$$

5. Experimental Results

Mixed standard: Solvent peaks at 2.0 min for sodium hydroxide and at 41 min for sodium acetate, as shown in Figure 6.

| Name | RT | Molar Ratio |
|---|---|---|
| Fucose | 5.959 | 0.000 |
| Galactosamine hydrochloride | 10.817 | 0.000 |
| Rhamnose | 11.334 | 0.000 |
| Arabinose | 11.792 | 0.000 |
| Glucosamine hydrochloride | 13.384 | 0.000 |
| Galactose | 14.609 | 0.000 |
| Glucose | 16.809 | 0.887 |
| N-acetyl-D-glucosamine | 18.55 | 0.000 |
| Xylose | 19.209 | 0.000 |
| Mannose | 19.825 | 0.000 |
| Fructose | 22.259 | 0.000 |
| Ribose | 24.309 | 0.000 |
| Galacturonic acid | 44.592 | 0.000 |
| Guluronic acid | 45.184 | 0.000 |

(continued)

| Name | RT | Molar Ratio |
|---|---|---|
| Glucuronic acid | 47.317 | 0.113 |
| Mannuronic acid | 50.2 | 0.000 |

III. Experiment on the Determination of Polysaccharide Linkage

1. Experimental Objective

To determine the linkage patterns of polysaccharide samples through derivatization such as methylation by GC-MS analysis.

2. Experimental Materials

2.1 Equipment

| Equipment name | Manufacturer | Model |
|---|---|---|
| Rotary evaporator | Zhengzhou Greatwall Scientific Industrial and Trade Co., Ltd. | R-1001VN |
| Nitrogen blower | LICHEN | UGC-24M |
| Magnetic stirrer | DLAB | MS7-H550-Pro |
| Vacuum drying oven | LICHEN | 101-1BS |
| Gas chromatograph-mass spectrometer | Agilent | 6890-5973 |

2.2 Reagents

| Reagent | Manufacturer | Batch No. | Catalog No. | Grade |
|---|---|---|---|---|
| Trifluoroacetic acid | ACROS | A0356762 | 139725000 | AR |
| Methyl iodide | Adamas | P1345479 | 01111630 | AR |
| Sodium borohydride | Aldrich | MKCD7945 | 205591 | AR |
| Ethyl acetate | Vokai | 08050003 | 40065982 | AR |
| Acetic anhydride | HUSHI | 20170314 | 10000318 | AR |
| Perchloric acid | Aldrich | SHBF7833V | 311421 | AR |
| Acetic acid | Fisher | 156174 | A35-500 | AR |
| Methanol | Merck | 10941735810 | 67-56-1 | AR |
| Sodium hydroxide | HUSHI | 20150429 | 10019718 | AR |
| Dimethyl sulfoxide | Adamas | P1265087 | 759270 | AR |
| Sodium hydride | Adamas | P1306059 | 81778A | AR |
| Methylation kit | Borui Saccharide | BRT-2020JJH | BRT-JJH | AR |

3. Experimental Methods

3.1 Reagent Preparation

| Reagent name | Preparation method | Storage conditions |
|---|---|---|
| 3M trifluoroacetic acid | 1V trifluoroacetic acid + 3V water | Store in refrigerator at 5°C |
| Sodium hydride dry powder | 60% sodium hydride washed with hexane | Store dry at room temperature |
| Sodium borodeuteride and | 20 mg + 20 mM NaOH solution | Seal and store |
| sodium hydroxide solution | | |
| 20% acetic acid methanol solution | 1V glacial acetic acid + 4V water | Store in refrigerator at 5°C |
| Polysaccharide methylation kit | A: anhydrous alkaline solution | Store in refrigerator at 5°C |
| | B: methyl iodide solution | |

3.2 Sample Methylation

The sample underwent methylation, hydrolysis, and acetylation, followed by GC-MS analysis, which was compared with the standard mass spectral library.

2-3 mg of the polysaccharide sample was weighed and placed in a glass reaction vial, 1 mL of anhydrous DMSO was added, and methylation reagent A was added rapidly. The solution was sealed and dissolved under ultrasonication, then methylation reagent B was added. The reaction was allowed at 30°C in a magnetic stirring water bath for 60 min. Finally, 2 mL of ultrapure water was added to stop the methylation reaction.

The methylated polysaccharide was taken, 1 mL of 2M trifluoroacetic acid (TFA) was added, and hydrolyzed for 90 min. The rotary evaporator was used to dry. 2 mL of double-distilled water was added to the residue, which was reduced with 60 mg of sodium borohydride for 8 h, neutralized with glacial acetic acid, and evaporated using the rotary evaporator. Then, it was dried in a 101°C oven, then 1 mL of acetic anhydride was added for acetylation and reacted at 100°C for 1 h, and cooled. Then 3 mL of toluene was added, vacuum concentrated to dry, and this process was repeated 4-5 times to remove excess acetic anhydride.

The acetylated product was dissolved in 3 mL of $CH_2Cl_2$ and transferred to a separatory funnel. A small amount of distilled water was added and shaken thoroughly, then the upper aqueous layer was removed. This process was repeated four times. The $CH_2Cl_2$ layer was dried with an adequate amount of anhydrous sodium sulfate, brought to a volume of 10 mL, and placed in a vial for liquid analysis. The acetylated sample was analyzed using a Shimadzu GCMS-QP 2010 gas chromatograph-mass spectrometer;

GC-MS Conditions: RXI-5 SIL MS column 30 m × 0.25 mm × 0.25 μm; Temperature program: started at 120°C, increased at 3°C/min to 250°C, held for 5 min; Injector temperature at 250°C, detector temperature at 250°C, carrier gas was helium, flow rate was 1 mL/min.

4. Experimental Results

The GC-MS chromatogram of the sample (PMAA) is shown in Figure 8.

The permethylated alditol acetate (PMAA) analysis of the polysaccharide is presented in the following table and Figures 11-14.

| RT | Methylated sugar | Mass fragments (m/z) | Molar ratio | Type of linkage |
|---|---|---|---|---|
| 17.253 | 2, 3, 4, 6-$Me_4$-Glcp | 45, 71, 87, 101, 117, 129, 145, 161, 205 | 0.162 | Glcp-(1→ |
| 21.918 | 2, 3, 6-$Me_3$-Glcp | 45, 87, 99, 101, 113, 117, 129, 143, 161, 173, 233 | 0.619 | →4)-Glcp-(1→ |
| 24.82 | 2, 6-$Me_2$-Glcp | 45, 69, 87, 117, 129, 143, 185 | 0.102 | →3, 4)-Glcp-(1→ |
| 27.129 | 2, 3-$Me_2$-Glcp | 45, 71, 85, 87, 99, 101, 117, 127, 159, 201 | 0.118 | →4, 6)-Glcp-(1→ |
| | Glcp-(1→ | | | |

IV. NMR Spectral Analysis and Interpretation

1. Experimental Materials and Equipment
Deuterium oxide (D2O, 99.9%) and deuterated acetone as internal standard; freeze-dryer, Bruker 600M Nuclear Magnetic Resonance (NMR) spectrometer;
2. Experimental Procedure
50 mg of the polysaccharide sample was weighed and dissolved in 0.5 mL of deuterium oxide followed by freeze-drying. The lyophilized powder was redissolved in 0.5 mL of deuterium oxide and freeze-drying was continued. The process was repeated to ensure complete exchange of labile hydrogens. Subsequently, the sample was dissolve in 0.5 mL of deuterium oxide, and 1H NMR, 13C NMR, DEPT135 one-dimensional and two-dimensional spectral measurements were performed at 25°C using a 600 MHz NMR spectrometer.
3. Experimental Results

The hydrogen spectrum signals were primarily concentrated between 3.0 and 5.5 ppm. The signals for sugar ring protons were between δ3.2-4.0 ppm, with main terminal group protons peaks at δ4.43, 4.45, 4.47, and 4.66, primarily distributed in the 4.3-5.5 ppm region as shown in Figure 13.

Carbon spectral analysis in $^{13}C$ NMR (201 MHz, $D_2O$): The NMR carbon spectrum signals were mainly concentrated between 60-120 ppm. Observations of the carbon spectrum indicated main anomeric carbon signal peaks at δ103.81, 103.82, 103.88, and 104.13, primarily between δ93-105. Other notable signal peaks were at δ76.68, 74.08, 82.67, 76.62, 70.19, 74.15, 71.56, 80.14, 76.67, 61.66, 74.75, 85.63, 77.17, 76.97, 62.03, 74.76, 74.35, 77.31, 69.61, and 62.04 ppm. Based on monosaccharide composition results, the polysaccharide is composed of glucose, indicating the polysaccharide is primarily glucan. This is depicted in Figure 14.

Dept135 spectral analysis showed inverted peaks at 70.19, 61.66, 62.03, and 62.04 ppm, indicative of chemical shifts for C6, as shown in Figure 15.

Figures 16-19 present HSQC spectra where the anomeric carbon signal at δ104.13 corresponds to anomeric hydrogen signal at δ4.66. HH-COSY identifies H1-2 signal at 4.66/3.3; H2-3 signal at 3.3/3.42; H3-4 signal at 3.42/3.66. We deduce H1, H2, H3, and H4 as δ4.66, 3.3, 3.42, and 3.66 respectively, corresponding to δ104.13, 74.76, 74.35, and 77.31. HH-COSY identifies H6b-6a signal at 3.81/3.62; H6a-5 signal at 3.62/3.46. We deduce H6b, H6a, and H5 as δ3.81, 3.62, and 3.46 respectively, corresponding to C6 and C5 at δ62.04 and 74.54, respectively. Therefore, this signal is attributed to the glycosidic bond β-Glcp-(1→.

Further HSQC observations show anomeric carbon signal at δ103.81, corresponding to anomeric hydrogen signal at δ4.45. HH-COSY identifies H1-2 signal at 4.45/3.25; H2-3 signal at 3.25/3.42; H3-4 signal at 3.42/3.64. We deduce H1, H2, H3, and H4 as δ4.45, 3.25, 3.42, 3.64 respectively, corresponding to C1-4 at δ103.81, 76.68, 74.08, and 82.67. Combining Dept135 with HSQC, signals at δ3.78 and 4.13 are attributed to H6a,b peaks, corresponding to C6 at 70.19 ppm. In the HMBC spectrum, H6b intersects with C5 and C4 at 3.78/76.62 and 3.78/82.67, thus C5 is 76.62 ppm. HSQC corresponding H5 is at 3.82 ppm. Therefore, this signal is attributed to the glycosidic bond →4,6)-β-D-Glcp-(1→.

HSQC spectra show the anomeric carbon signal at δ103.88 corresponds to anomeric hydrogen signal at δ4.47. HH-COSY identifies H1-2 signal at 4.47/3.47; H2-3 signal at 3.47/3.65. We deduce H1, H2, and H3 as δ4.47, 3.47, and 3.65 respectively, corresponding to C1-3 at δ103.88, 74.75, and 85.63. In the HMBC spectrum, 85.63/3.44 and 76.97/3.71 are attributed to C3/H5 and C5/H4. Combining Dept135 with HSQC, signals at δ3.65 and 3.83 are attributed to H6a,b peaks, corresponding to C6 at 62.03 ppm. Therefore, this signal is attributed to the glycosidic bond →3,4)-β-D-Glcp-(1→.

HSQC spectra show the anomeric carbon signal at δ103.82 corresponds to anomeric hydrogen signal at δ4.43. HH-COSY identifies H1-2 signal at 4.43/3.28; H2-3 signal at 3.28/3.44. We deduce H1, H2, and H3 as δ4.43, 3.28, and 3.44 respectively, corresponding to C1-3 at δ103.82, 74.15, and 76.82. C4 shifts to the lower field, and C4 is attributed to 80.14 ppm. Combining Dept135 with HSQC, signals at δ3.68 and 3.84 are attributed to H6a,b peaks, corresponding to C6 at 61.66 ppm. Therefore, this signal is attributed to the glycosidic bond →4)-β-D-Glcp-(1→.

Using similar patterns and combining HMBC and NOESY, all glycosidic bond signals are assigned as shown in the following table: Hydrogen and Carbon Signal Attribution

| Glycosyl residues | H1/C1 | H2/C2 | H3/C3 | H4/C4 | H5/C5 | H6a/C6 | H6b |
|---|---|---|---|---|---|---|---|
| β-D-Glcp-(1→ | 4.66 | 3.56 | 3.42 | 3.41 | 3.57 | 3.85 | 3.67 |
| | 103.89 | 71.56 | 74.55 | 69.23 | 76.29 | 62.01 | |
| →4,6)-β-D-Glcp-(1→ | 4.45 | 3.25 | 3.42 | 3.64 | 3.82 | 4.15 | 3.79 |
| | 103.81 | 74.08 | 76.68 | 82.67 | 76.62 | 70.19 | |
| →4)-β-D-Glcp-(1→ | 4.43 | 3.28 | 3.44 | 3.64 | 3.55 | 3.84 | 3.68 |

(continued)

| Glycosyl residues | H1/C1 | H2/C2 | H3/C3 | H4/C4 | H5/C5 | H6a/C6 | H6b |
|---|---|---|---|---|---|---|---|
| | 103.82 | 74.15 | 76.82 | 82.14 | 76.67 | 62.07 | |
| →3,4)-β-D-Glcp-(1→ | 4.47 | 3.47 | 3.68 | 3.71 | 3.44 | 3.76 | 3.91 |
| | 103.88 | 74.75 | 85.63 | 77.17 | 76.97 | 61.47 | |

Main Chain Analysis:

From the HMBC spectra, based on the one-dimensional and two-dimensional NMR spectra, we have attributed the signals of the glycosidic bonds in the polysaccharide: The anomeric carbon of the glycosidic bond →4)-β-D-Glcp-(1→ shows correlation signal peaks with its own H4, indicating the presence of a →4)-β-D-Glcp-(1→4)-β-D-Glcp-(1→ linkage.

The anomeric hydrogen of the glycosidic bond →4)-β-D-Glcp-(1→ shows correlation signal peaks with the C4 of →3,4)-β-D-Glcp-(1→, indicating the presence of a →4)-β-D-Glcp-(1→3,4)-β-D-Glcp-(1→ linkage.

The anomeric hydrogen of the glycosidic bond →3,4)-β-D-Glcp-(1→ shows correlation signal peaks with the C4 of →4,6)-β-D-Glcp-(1→, indicating the presence of a →3,4)-β-D-Glcp-(1→4,6)-β-D-Glcp-(1→ linkage.

Branch Chain Analysis:

The anomeric hydrogen of the glycosidic bond β-D-Glcp-(1→ shows correlation peaks with the C3 of →3,4)-β-D-Glcp-(1→, indicating the presence of a β-D-Glcp-(1→3,4)-β-D-Glcp-(1→ linkage.

The anomeric carbon of the glycosidic bond β-D-Glcp-(1→ shows correlation peaks with the H6 of →4,6)-β-D-Glcp-(1→, indicating the presence of a β-D-Glcp-(1→4,6)-β-D-Glcp-(1→ linkage.

Based on the above, we can deduce that the main chain of the polysaccharide is the β-1,4 glucan, with β-D-Glcp-(1→ linked to the main chain through an O-3 bond of →3,4)-β-D-Glcp-(1→ and an O-6 bond of →4,6)-β-DGlcp-(1→. The condensed structural formula is as follows.

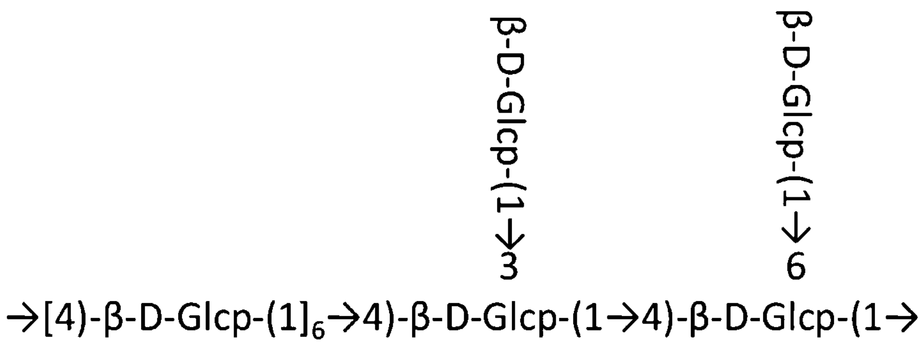

This invention also provides the application of the Ganoderma lucidum polysaccharide GLP-1. The Ganoderma lucidum polysaccharide GLP-1 is characterized by its water solubility, is readily absorbed by the human body, offers antitumor effects, and has proven strong efficacy in the prevention of tumor development in humans. Notably, when used in combination with chemotherapy drugs, it can alleviate toxic side effects caused by the chemotherapy drugs on the human body, control and reduce tumor masses, and reduce and eliminate cancer cells.

The Ganoderma lucidum polysaccharide GLP-1 is used in medications to inhibit tumor metastasis or to enhance human immune function.

**Experimental Study on the Antitumor Effect of Ganoderma Lucidum Polysaccharide GLP-1 Combined with Chemotherapy on LLC-Bearing Mice and Study Data**

Experimental Objective

To study the antitumor effect of Ganoderma lucidum polysaccharide GLP-1 on lung cancer-bearing mice which are prepared by implanting LLC tumors in the right axilla of C57 mice. This study aims to provide experimental evidence for clinical studies of GLP-1.

**Experimental Materials**

**Test Sample**

Ganoderma lucidum polysaccharide GLP-1, batch number: ALM20200518Al, provided by Shenzhen Aolimei Oncology Medical Technology Co., Ltd.

**Positive Control**

Kanglaite soft capsules, batch number: 20200802, Zhejiang Kanglaite Pharmaceutical Co., Ltd.; cisplatin injection, batch number: 601210104, Jiangsu Hansoh Pharmaceutical Co., Ltd.

**Laboratory Animals**

80 SPF-grade male C57 mice, weighing 12-15 g, provided by Hunan SJA Laboratory Animal Co., Ltd. Laboratory animal production license number: SCXK (Xiang) 2019-0004; laboratory animal quality certification number: 430727211103153225. The animals were housed in Barrier Environment Laboratory D of Hunan Puruima Pharmaceutical Research Center Co., Ltd., under the laboratory animal use license number: SYXK (Xiang) 2020-0015.

**Main Reagents**

0.9% sodium chloride injection, batch number: 21071401C, Hunan Kangyuan Pharmaceutical Co., Ltd.; ALP assay kit, batch number: 201223, Maccura Biotechnology; TP assay kit, batch number: 105467, ALB assay kit, batch number: 105468, ALT assay kit, batch number: 105475, AST assay kit, batch number: 104446, TBIL assay kit, batch number: 105465, GGT assay kit, batch number: 104447, all manufactured by Wako Pure Chemical Industries, Ltd. of Japan.

**Main Instruments**

S10 Portable High-Speed Disperser, Scientz; AR223CN Electronic Scale, Ohaus Instruments (Changzhou) Co., Ltd.; LABOSPECT003 Automatic Biochemical Analyzer, Hitachi, Japan; TDZ5-WS Benchtop Multi-Tube Automatic Balance Centrifuge, Hunan Kaida Industrial Development Co., Ltd.; ME2002E Electronic Scale, Mettler-Toledo Instruments (Shanghai) Co., Ltd.; Digital Caliper, Yongkang Zhengfeng Hardware Co., Ltd.; Flow Cytometer, BD Biosciences; ASP200S Fully Automatic Tissue Dehydrator, ASP300S Fully Automatic Tissue Dehydrator, TP1020 Fully Automatic Dehydrator, HI1210 Slide Spreader, HI1220 Slide Dryer, RM2235 Paraffin Microtome, EG1150H+C Tissue Embedding Station, AutoStainer XL Automatic Slide Stainer + CV5030 Automatic Cover Slipper, Leica, Germany; BX43 Biological Microscope + MD50 Digital Imaging System, CX31 Biological Microscope, Olympus, Japan.

**Experimental Methods**

LLC cells in logarithmic growth phase at $1 \times 10^7$/mL were inoculated at 0.2 mL into the right axilla of 8 normal male C57 mice. Tumor growth was monitored, and tumor-bearing mice were prepared. Once the tumor volume in tumor-bearing mice exceeded 200 $mm^3$, the tumor tissue was aseptically harvested to prepare a tissue cell suspension. This was mixed at a 1:1 (V:V) ratio with culture medium to create a tumor tissue homogenate. This homogenate was injected into the right axilla of 62 male C57 mice at 0.2 mL per mouse. When the tumor volume in these tumor-bearing mice exceeded 100 $mm^3$, mice with well-grown tumors without ulceration were selected and randomized into groups based on tumor volume as follows: model control group, cisplatin group (4 mg/kg), Kanglaite soft capsule group (1,404 mg/kg), cisplatin + Kanglaite soft capsule group (4 + 1,404 mg/kg), cisplatin + GLP-1 low-dose group (4 + 130 mg/kg), and cisplatin + GLP-1 high-dose group (4 + 1,170 mg/kg), with 8 mice per group. Additionally, 8 mice were selected as a normal control group. Cisplatin was administered every three days in the cisplatin group and the cisplatin combination groups, Kanglaite daily, and GLP-1 daily, with dosing volumes of 20 mL/kg (oral gavage) and 10 mL/kg (intraperitoneal injection), respectively. Treatment was administered continuously for 27 days. After the last dose, blood was collected from the orbital plexus for analysis of blood WBC, RBC, liver and kidney functions (ALT, AST, BUN, CRE), and CD4+, CD8+ levels. The spleen, thymus, and tumors were weighed to calculate the organ-to-body weight ratio. Bone marrow smears were prepared, and histopathological analyses were conducted on tumors, spleen, stomach, and kidneys.

**Dosage Design**

Based on preliminary study findings, Ganoderma lucidum polysaccharide GLP-1 was administered at a low dose of 130 mg/kg and a high dose of 1,170 mg/kg. The doses for each respective drug administered in this experiment are

shown in Table 1, and the clinical intended dose was used in equivalent proportions.

The proposed clinical dose for Kanglaite soft capsules is 0.45 g per capsule, 6 capsules per dose, 4 doses per day, which totals 10.8 g per day. When converted to an equivalent mouse dose based on body surface area, it is calculated as 10.8 g/day × 0.0026 / 0.02 kg = 1,404 mg/kg. This study used the proposed clinical dose as a basis for the experiment.

Rationale for cisplatin dosage design: Based on the clinical dosage of cisplatin, which should not exceed 100 $mg/m^2$ per person per day, and considering the tolerance of mice to cisplatin, a dose of 4 mg/kg has been selected as the administration dosage.

Table 1: Experimental groups and dosage design

| Group | Dose (mg/kg) | Route of administration | Frequency of administration |
|---|---|---|---|
| Normal control group | - | Oral gavage | Once daily |
| Model control group | - | Intraperitoneal injection | Once every 2 days |
| Cisplatin group | 4 | Intraperitoneal injection | Once every 3 days |
| Kanglaite soft capsule group | 1404 | Oral gavage | Once daily |
| Cisplatin + Kanglaite soft capsule group | 4+1404 | Intraperitoneal injection + oral gavage | Cisplatin, once every 3 days; Kanglaite soft capsule, once daily |
| Cisplatin + GLP-1 low-dose group | 4+130 | Intraperitoneal injection + oral gavage | Cisplatin, once every 3 days: GLP-1, once daily |
| Cisplatin + GLP-1 high-dose group | 4+1170 | Intraperitoneal injection + oral gavage | Cisplatin, once every 3 days: GLP-1, once daily |

**Test Indicators**

**Efficacy Indicators**

**Evaluation of quality of life and survival time in animals:** Throughout the experimental process, detailed records were kept for each group of animals regarding weight, mortality, mental state, fur color, smoothness of fur, and fecal form, including incidences of diarrhea (if any).

**Tumor volume measurement:** Tumor dimensions (length and width) were measured with a caliper every three days post-dose, and the tumor volume was calculated as follows: tumor volume (TV) = 1 / 2 × a (length) × $b^2$ (width). Relative tumor volume (RTV) = $TV_t$ / $TV_0$, where $TV_0$ is the tumor volume at the time of administration, and $TV_t$ is the tumor volume at each measurement. Relative tumor growth rate T/C (%) = $T_{RTV}$ / $C_{RTV}$ × 100%, where $T_{RTV}$ is the RTV for the treatment group and $C_{RTV}$ is the RTV for the control group. Tumor growth inhibition rate (%) = (model control group tumor weight - treatment group tumor weight) / model control group tumor weight × 100%. A tumor growth inhibition rate (%) of < 40% is considered ineffective; a tumor growth inhibition rate (%) of ≥ 40% with $P \leq 0.05$ is considered effective.

**Spleen and thymus organ coefficients:** After the last dose, spleen, thymus, and tumor weights are measured, and organ coefficients are calculated. organ coefficient (%) = organ weight / fasting body weight × 100%.

**Potentiation and Detoxification**

**Hematological tests:** Blood samples for routine blood tests (WBC and RBC) and liver and kidney function tests (BUN and CRE) were collected from the orbital plexus after the last dose.

**Bone marrow examination:** After the last dose, bone marrow was collected for smear tests, and changes in bone marrow cells were observed in each group of mice.

**Histopathology examination:** After the last dose, tissues from tumors, spleens, stomachs, and kidneys were collected for H&E staining to observe histopathological changes.

**Blood CD4+ and CD8+ content measurement:** After the last dose, lymphocyte subtypes CD4+ and CD8+ in the blood were measured using flow cytometry.

**Data Processing and Statistical Analysis**

Significant figures of the study data were rounded according to the nearest whole number. Statistical analysis was conducted in accordance with the center's SOP, using SPSS software. Measurement data are expressed as mean ± standard deviation (x ± s), and normality and homogeneity of variance were tested using Leven's test. If not statistically significant (P > 0.05), one-way ANOVA was used for statistical analysis. If ANOVA was statistically significant (P ≤ 0.05),

the LSD test (parametric method) was used for comparison analysis. If the variance was not homogeneous ($P \leq 0.05$), the Kruskal-Wallis test was employed. If the Kruskal-Wallis test was statistically significant ($P \leq 0.05$), the Dunnett's Test (non-parametric method) was used for comparison analysis. Statistical significance was determined at $\alpha = 0.05$, where $P \leq 0.05$ indicates statistical significance and $P \leq 0.01$ indicates highly significant differences.

## Experimental Results

### General Clinical Observation and Mortality in Animals

Prior to administration, the mice exhibited normal activity and gait. In the model control group, mice 2M06 and 2M03 died on January 20, 2022 and January 21, 2022, respectively. In the Kanglaite soft capsule group, mouse 4M01 died on January 19, 2022. In the cisplatin + Kanglaite soft capsule group, mice 5M01 and 5M04 died on January 14, 2022 and January 22, 2022, respectively. In the cisplatin + GLP-1 high-dose group, mouse 7M08 died on January 09, 2022.

The mortality rates for each group were 0%, 25.0%, 0%, 12.5%, 25.0%, 0%, and 12.5%, respectively.

Table 2: Statistics on the number of surviving animals and mortality rate in each group

| Group | Dose (mg/kg) | Number of animals | Number of deaths | Number of surviving animals | Mortality rate (%) |
|---|---|---|---|---|---|
| Normal control group | - | 8 | 0 | 8 | 0 |
| Model control group | - | 8 | 2 | 6 | 25.0 |
| Cisplatin group | 4 | 8 | 0 | 8 | 0 |
| Kanglaite soft capsule group | 1404 | 8 | 1 | 7 | 12.5 |
| Cisplatin + Kanglaite soft capsule group | 4+1404 | 8 | 2 | 6 | 25.0 |
| Cisplatin + GLP-1 low-dose group | 4+130 | 8 | 0 | 8 | 0 |
| Cisplatin + GLP-1 high-dose group | 4+1170 | 8 | 1 | 7 | 12.5 |

### Effect of Ganoderma Lucidum Polysaccharide GLP-1 Combined with Chemotherapy on Body Weight of LLC-Bearing Mice

As shown in Table 3, compared to the normal control group, the body weight of the mice in the model control group significantly decreased after administration on D4 ($P \leq 0.05$). Compared to the model control group, the body weight of mice in the cisplatin group and cisplatin + Kanglaite soft capsule group significantly decreased after administration from D7 to D25 ($P \leq 0.01$); the body weight of mice in the cisplatin + GLP-1 low-dose and high-dose groups significantly decreased after administration from D4 to D25 ($P \leq 0.05$ or $P \leq 0.01$); the body weight of mice in the Kanglaite soft capsule group significantly decreased after administration from D10 to D19.

Compared to the cisplatin group, the body weight of mice in the Kanglaite soft capsule group significantly increased after administration from D7 to D25 ($P \leq 0.05$ or $P \leq 0.01$); the body weight of mice in the cisplatin + GLP-1 high-dose group significantly decreased after administration on D7 ($P \leq 0.05$). Compared to the Kanglaite soft capsule group, the body weight of mice in the cisplatin + Kanglaite soft capsule group and GLP-1 low-dose and high-dose groups significantly decreased after administration from D7 to D25 ($P \leq 0.05$ or $P \leq 0.01$). There was no significant statistical difference between groups compared to the cisplatin + Kanglaite soft capsule group.

Table 3: Effect of Ganoderma lucidum polysaccharide GLP-1 combined with chemotherapy on body weight of LLC-bearing mice ($\bar{x} \pm s$)

| Group | Weight (g) | | | | | | | | |
|---|---|---|---|---|---|---|---|---|---|
| | D1 | D4 | D7 | D10 | D13 | D16 | D19 | D22 | D25 |
| Normal control group | 15.2 ± 0.6 | **16.8 ± 0.8** | 17.6 ± 1.0 | 17.9 = 0.6 | 19.7 ± 0.8 | 21.0 ± 1.1 | 22.1 ± 1.5 | 21.4 ± 1.1 | 22.1 ± 1.2 |
| Model control group | 14.3 ± 1.1 | **15.6 ± 1.2⁺** | 17.1 ± 1.5 | 17.9 ± 1.6 | 19.3 ± 1.3 | 20.9 ± 1.2 | 21.9 ± 1.0 | 21.7 ± 1.0 | 22.4 ± 2.0 |
| Cisplatin group | 14.1 ± 0.7 | 14.5 ± 11 | **14.1 ± 1.7**** | **13.5 ± 1.5**** | **13.0 ± 1.9**** | **13.8 ± 2.3**** | **13.9 ± 2.3**** | **13.9 ± 2.0**** | **14.0 ± 2.3**** |

(continued)

| Group | Weight (g) | | | | | | | | |
|---|---|---|---|---|---|---|---|---|---|
| | D1 | D4 | D7 | D10 | D13 | D16 | D19 | D22 | D25 |
| Kanglaite soft capsule group | 13.4 ± 1.5 | 14.8 ± 1.5 | **16.1 ± $1.5^{\#\#}$** | **15.6 ± $1.7^{**\#}$** | **169 ± $2.1^{*\#\#}$** | **183 ± $1.7^{*\#\#}$** | **18.8 ± $2.1^{**\#\#}$** | **20.2 ± $1.5^{\#\#}$** | **215 ± $1.4^{\#\#}$** |
| Cisplatin + Kanglaite soft capsule group | 14.7 ± 0 7 | 14.6 ± 0.7 | **13.8 ± $0.7^{**\&\&}$** | **13.4 ± $1.3^{**\&\&}$** | **13.2 ± $1.6^{**\&\&}$** | **13.7 ± $1.7^{**\&\&}$** | **13.9 ± $1.6^{**\&\&}$** | **13.5 ± $1.7^{**\&\&}$** | **14.0 ± $1.5^{**\&\&}$** |
| Cisplatin + GLP-1 low-dose group | 14.0 ± 1.4 | **13.9 ± 1.4**** | **13.9 ± $2.1^{**\&\&}$** | **12.7 ± $2.0^{**\&\&}$** | **12.5 ± $2.3^{**\&\&}$** | **12.9 ± $2.8^{**\&\&}$** | **13.5 ± $33^{**\&\&}$** | **13.6 ± $3.6^{**\&\&}$** | **13.7 ± $3.7^{**\&\&}$** |
| Cisplatin + GLP-1 high-dose group | 14.5 ± 1.3 | **14.3 ± 1.0*** | **12.4 ± $0.9^{***\&\&}$** | **12.1 ± $1.4^{**\&\&}$** | **11.9 ± $1.4^{**\&\&}$** | **12.5 ± $1.5^{**\&\&}$** | **13.1 ± $2.0^{**\&\&}$** | **12.5 ± $1.9^{**\&\&}$** | **12.6 ± $2.3^{**\&\&}$** |

Note: Compared to the normal control group, $^{+}P \leq 0.05$; compared to the model control group, $^{*}P \leq 0.05$, $^{**}P \leq 0.01$; compared to the cisplatin group, $^{\#}P \leq 0.05$, $^{\#\#}P \leq 0.01$; compared to the Kanglaite soft capsule group, $^{\&}P \leq 0.05$, $^{\&\&}P \leq 0.01$ compared to the cisplatin + Kanglaite soft capsule group, $^{\star}P \leq 0.05$, $^{\star\star}P \leq 0.01$.

**Effect of Ganoderma Lucidum Polysaccharide GLP-1 Combined with Chemotherapy on Tumor Volume and Relative Tumor Volume in LLC-Bearing Mice**

As shown in Table 4, compared to the model control group, the tumor volume of mice in the cisplatin + GLP-1 low-dose and high-dose groups significantly decreased after administration from D10 to D25 (P ≤ 0.05 or P ≤ 0.01), the tumor volume of mice in the cisplatin group significantly decreased after administration on D10 and from D16 to D25 (P ≤ 0.05 or P ≤ 0.01), and the tumor volume of mice in the cisplatin + Kanglaite soft capsule group significantly decreased after administration from D13 to D25 (P ≤ 0.05 or P ≤ 0.01). There was no significant difference in tumor volume in the Kanglaite soft capsule group. Compared to the cisplatin group, the tumor volume of mice in the cisplatin + GLP-1 high-dose group significantly decreased after administration from D22 to D25 (P ≤ 0.05), and the tumor volume of mice in the Kanglaite soft capsule group significantly increased after administration from D7 to D25 (P ≤ 0.05 or P ≤ 0.01). Compared to the Kanglaite soft capsule group, the tumor volume of mice in the cisplatin + GLP-1 low-dose and high-dose groups and the Kanglaite soft capsule group significantly decreased after administration from D13 to D25 (P ≤ 0.05 or P ≤ 0.01).

As shown in Table 5, compared to the model control group, the relative tumor volume of mice in the cisplatin group, the cisplatin + Kanglaite soft capsule group, and the cisplatin + GLP-1 low-dose and high-dose groups significantly decreased after administration from D13 to D25 (P ≤ 0.05 or P ≤ 0.01). There was no statistically significant difference in relative tumor volume in the relative tumor volume group. Compared to the cisplatin + Kanglaite soft capsule group, the tumor volume of mice in the cisplatin + GLP-1 high-dose group significantly decreased after administration on D19 and D25 (P ≤ 0.05), with no statistical significance in the other groups.

Table 4: Effect of Ganoderma lucidum polysaccharide GLP-1 combined with chemotherapy on tumor volume in LLC-Bearing Mice ($\bar{x} \pm s$)

| Group | Tumor volume ($mm^3$) | | | | | | | | |
|---|---|---|---|---|---|---|---|---|---|
| | D1 | D4 | D7 | D10 | D13 | D16 | D19 | D22 | D25 |
| Model control group | 94.63 ± 20.97 | 178.51 ± 57.95 | 19.72 ± 12.58 | 26.4 ± 18.02 | 42.45 ± 20.82 | 145.78 ± 62.96 | 218.08 ± 69.66 | 3025.26 ± 804.69 | 4173.23 ± 1009.80 |
| Cisplatin group | 94.99 ± 11.16 | 150.58 ± 34.33 | 8.91 ± 2.56 | **6.51 ± 4.74*** | 27.36 ± 24.4 | **36.09 ± 27.67**** | **71.00 ± 49.93**** | **824.01 ± 435.48**** | **976.79 ± 427.46**** |
| Kanglaite soft capsule group | 94.81 ± 13.17 | 146.63 ± 27.20 | **31.53 ± $12.76^{\#}$** | **17.13 ± $3.68^{\#}$** | **33.8 ± $14.59^{\#}$** | **106.86 ± $26.94^{\#}$** | **164.23 ± $33.41^{\#}$** | **2559.02 ± $439.54^{\#}$** | **4270.10 ± $883.45^{\#\#}$** |

(continued)

| Group | Tumor volume (mm³) | | | | | | | | |
|---|---|---|---|---|---|---|---|---|---|
| | D1 | D4 | D7 | D10 | D13 | D16 | D19 | D22 | D25 |
| Cisplatin + Kanglaite soft capsule group | 95.88 ± 14.41 | 172.38 ± 31.19 | 18.53 ± 6.71 | 10.34 ± 6.25 | 2.56 ± 1.08**& | 4.21 ± 2.78**&& | 18.59 ± 9.44**&& | 218.87 ± 120.00**&& | 781.75 ± 520.71**&& |
| Cisplatin + GLP-1 low-dose group | 94.18 ± 14.29 | 132.37 ± 28.84 | 9.41 ± 3.50 | 6.22 ± 6.22* | 2.26 ± 1.39**& | 6.24 ± 3.33**&& ± | 24.21 ± 17.01**&& | 400.76 ± 191.51**&& | 656.02 ± 291.28**&& |
| Cisplatin + GLP-1 high-dose group | 94.89 ± 14.51 | 124.62 ± 13.92 | 14.97 ± 7.15 | 6.72 ± 4.54* | 0.28 ± 0.28**& | 1.18 ± 1.18**&& | 1.74 ± 1.35**&&★ | 54.23 ± 33.08**#&& | 32.60 ± 16.57**#&&★ |

Note: Compared to the model control group, *P ≤ 0.05, **P ≤ 0.01; compared to the cisplatin group, #P ≤ 0.05, ##P ≤ 0.01; compared to the Kanglaite soft capsule group, &P ≤ 0.05, &&P ≤ 0.01; compared to the cisplatin + Kanglaite soft capsule group, ★P ≤ 0.05, ★★P ≤ 0.01.

Table 5: Effect of Ganoderma lucidum polysaccharide GLP-1 combined with chemotherapy on relative tumor volume in LLC-Bearing Mice ($\bar{x} \pm s$)

| Group | Relative tumor volume | | | | | | | |
|---|---|---|---|---|---|---|---|---|
| | D4 | D7 | D10 | D13 | D16 | D19 | D22 | D25 |
| Model control group | 2.26 ± 2.05 | 0.22 ± 0.43 | 0.33 ± 0.62 | 0.57 ± 0.79 | 1.81 ± 2.36 | 2.63 ± 2.77 | 34.34 ± 29.91 | 51.36 ± 28.59 |
| Cisplatin group | 1.84 ± 1.42 | 0.10 ± 0.07 | 0.09 ± 0.20 | 0.40 ± 1.04* | 0.51 ± 1.18* | 0.97 ± 2.12* | 10.62 ± 18.36* | 12.10 ± 17.51* |
| Kanglaite soft capsule group | 2.03 ± 1.89 | 0.53 ± 0.79 | 0.22 ± 0.15 | 0.42 ± 0.54 | 1.20 ± 0.93 | 1.83 ± 1.15 | 29.27 ± 11.91 | 48.39 ± 17.21 |
| Cisplatin + Kanglaite soft capsule group | 2.32 ± 1.75 | 0.31 ± 0.39 | 0.22 ± 0.36 | 0.04 ± 0.08** | 0.06 ± 0.09** | 0.41 ± 0.86* | 2.51 ± 3.38** | 8.02 ± 13.07* |
| Cisplatin + GLP-1 low-dose group | 1.65 ± 0.99 | 0.11 ± 0.09 | 0.08 ± 0.21 | 0.02 ± 0.05** | 0.06 ± 0.09** | 0.19 ± 0.41** | 3.77 ± 5.62** | 5.79 ± 7.08** |
| Cisplatin + GLP-1 high-dose group | 1.76 ± 1.33 | 0.2 ± 0.29 | 0.09 ± 0.17 | 0.00 ± 0.01** | 0.01 ± 0.03** | 0.01 ± 0.03** | 0.49 ± 0.65** | 0.40 ± 0.61 ** |

Note: Compared to the model control group, *P ≤ 0.05, **P ≤ 0.01.

**Effect of Ganoderma Lucidum Polysaccharide GLP-1 Combined with Chemotherapy on Relative Tumor Growth Rate (T/C) in LLC-Bearing Mice**

As shown in Table 6, the T/C ratios for mice in the cisplatin + GLP-1 low-dose from D7 to D25 after administration were: 50.0%, 21.2%, 3.5%, 3.3%, 7.2%, 11.0%, and 11.3%, all values ≤ 60.0%. For the cisplatin + GLP-1 high-dose group, the T/C ratios from D10 to D25 were: 27.3%, 0.0%, 0.6%, 0.4%, 1.4%, and 0.8%, all values ≤ 60.0%. For the cisplatin group, T/C ratios from D7 to D10 and D16 to D25 were: 45.5%, 27.3%, 28.2%, 36.9%, 30.9%, and 23.6%, all values ≤ 60.0%. For the cisplatin + Kanglaite soft capsule group, T/C ratios from D13 to D25 were: 7.3%, 3.3%, 15.6%, 7.3%, and 15.6%, all values ≤ 60.0%.

Table 6: Effect of Ganoderma lucidum polysaccharide GLP-1 combined with chemotherapy on relative tumor growth rate in LLC-Bearing Mice ($\bar{x}\pm s$)

| Group | Relative tumor growth rate (%) | | | | | | | |
|---|---|---|---|---|---|---|---|---|
| | D4 | D7 | D10 | D13 | D16 | D19 | D22 | D25 |
| Model control group | -- | -- | -- | -- | -- | -- | -- | -- |
| Cisplatin group | 81.4 | **45.5** | **27.3** | 70.2 | **28.2** | **36.9** | **30.9** | **23.6** |
| Kanglaite soft capsule group | 89.8 | 240.9 | 66.7 | 73.7 | 66.3 | 69.6 | 85.3 | 94.2 |
| Cisplatin + Kanglaite soft capsule group | 102.7 | 140.9 | 63.6 | **7.0** | **3.3** | **15.6** | **7.3** | **15.6** |
| Cisplatin + GLP-1 low-dose group | 73.0 | **50.0** | **21.2** | **3.5** | **3.3** | **7.2** | **11.0** | **11.3** |
| Cisplatin + GLP-1 high-dose group | 77.9 | 90.9 | **27.3** | **0.0** | **0.6** | **0.4** | **1.4** | **0.8** |

**Effect of Ganoderma Lucidum Polysaccharide GLP-1 Combined with Chemotherapy on Organ Coefficients and Tumor Growth Inhibition Rates in LLC-Bearing Mice**

As shown in Table 7, compared to the normal control group, the spleen and tumor coefficients in the model control group significantly increased ($P \leq 0.01$) and the thymus coefficient significantly decreased ($P \leq 0.01$). Compared to the model control group, the spleen, thymus, and tumor coefficients in both the cisplatin group and the cisplatin + GLP-1 low-dose group significantly decreased ($P \leq 0.05$ or $P \leq 0.01$), and the spleen and tumor coefficients in the cisplatin + GLP-1 high-dose capsule group significantly decreased ($P \leq 0.01$). Compared to the cisplatin group, the tumor coefficient of mice in the cisplatin + GLP-1 high-dose group significantly decreased ($P \leq 0.05$), and the spleen and tumor coefficients of mice in the Kanglaite soft capsule group significantly increased ($P \leq 0.01$). Compared to the Kanglaite soft capsule group, the spleen and tumor coefficients in the cisplatin + GLP-1 low-dose and high-dose groups significantly decreased ($P \leq 0.01$). There was no significant statistical difference in the organ-to-body weight ratios between groups compared to the cisplatin + Kanglaite soft capsule group. The tumor growth inhibition rate in the Kanglaite soft capsule group was -6.8%, all values < 40.0%; for the cisplatin group, cisplatin + Kanglaite soft capsule group, GLP-4 low-dose and high-dose groups, the tumor growth inhibition rates were 61.6%, 78.3%, 77.6%, and 96.7% respectively, all values ≥ 40.0%.

Table 7: Effect of Ganoderma lucidum polysaccharide GLP-1 combined with chemotherapy on organ coefficients and tumor growth inhibition rates in LLC-bearing mice ($\bar{x}\pm s$)

| Group | Spleen coefficient (%) | Thymus coefficient (%) | Tumor coefficient (%) | Tumor growth inhibition rate (%) |
|---|---|---|---|---|
| Normal control group | 0.368 ± 0.009 | 0.286 ± 0.018 | 0.000 ± 0.000 | |
| Model control group | **1.336 ± 0.280$^{++}$** | **0.128 ± 0.042$^{++}$** | **19.581 ± 3.559$^{++}$** | / / |
| Cisplatin group | **0.456 ± 0.102$^{**}$** | **0.044 ± 0.020$^{*}$** | **7.528 ± 2.567$^{**}$** | **61.6** |
| Kanglaite soft capsule group | **1.307 ± 0.195$^{\#\#}$** | **0.102 ± 0.034** | **20.918 ± 3.245$^{\#\#}$** | -6.8 |
| Cisplatin + Kanglaite soft capsule group | **0.396 ± 0.064$^{**\&\&}$** | **0.053 ± 0.014$^{*}$** | **4.247 ± 2.338$^{**\&\&}$** | **78.3** |
| Cisplatin + GLP-1 low-dose group | **0.446 ± 0.095$^{**\&\&}$** | **0.052 ± 0.022$^{*}$** | **4.395 ± 1.563$^{**\&\&}$** | **77.6** |
| Cisplatin + GLP-1 high-dose group | **0.283 ± 0.026$^{**\&\&}$** | **0.064 ± 0.017** | **0.651 ± 0.353$^{**\#\&\&}$** | **96.7** |

Note: Compared to the normal control group, $^{++}P \leq 0.01$; compared to the model control group, $^{*}P \leq 0.05$, $^{**}P \leq 0.01$; compared to the cisplatin group, $^{\#}P \leq 0.05$, $^{\#\#}P \leq 0.01$; compared to the Kanglaite soft capsule group, $^{\&}P \leq 0.05$, $^{\&\&}P \leq 0.01$; compared to the cisplatin + Kanglaite soft capsule group, $^{\star}P \leq 0.05$, $^{\star\star}P \leq 0.01$.

In Figure 20: 1: Model control group; 2: Cisplatin group; 3: Kanglaite soft capsule group; 4: Cisplatin + Kanglaite soft capsule group; 5: Cisplatin + GLP-1 low-dose group, 6: Cisplatin + GLP-1 high-dose group

**Effect of Ganoderma Lucidum Polysaccharide GLP-1 Combined with Chemotherapy on Blood Biochemical Indicators in LLC-Bearing Mice**

As shown in Table 8, compared to the normal control group, the blood WBC, ALT, AST, and BUN levels in the mice of the model control group significantly increased ($P \leq 0.05$ or $P \leq 0.01$), and the RBC level significantly decreased ($P \leq 0.05$). Compared to the model control group, the blood WBC and AST levels in the mice of the cisplatin + GLP-1 high-dose group significantly decreased ($P \leq 0.05$ or $P \leq 0.01$), the blood WBC, ALT, and AST levels in the mice of the cisplatin + GLP-1 low-dose group significantly decreased ($P \leq 0.05$), and the blood WBC level in the mice of the cisplatin group and the cisplatin + Kanglaite soft capsule group significantly decreased ($P \leq 0.05$). Compared to the cisplatin group, the blood RBC level in the mice of the cisplatin + GLP-1 high-dose group significantly increased ($P \leq 0.05$). Compared to the Kanglaite soft capsule group, the blood WBC level in the mice of the cisplatin + GLP-1 high-dose group significantly decreased ($P \leq 0.05$), and the blood WBC level in the mice of the cisplatin + Kanglaite soft capsule group significantly decreased ($P \leq 0.05$). Compared to the cisplatin + Kanglaite soft capsule group, the blood WBC level in the mice of the cisplatin + GLP-1 low-dose group significantly increased ($P \leq 0.05$), with no statistical significance in other groups.

Table 8: Effect of Ganoderma lucidum polysaccharide GLP-1 combined with chemotherapy on blood biochemical indicators in LLC-bearing mice ($\bar{x} \pm s$)

| Group | WBC ($10^9$/L) | RBC ($10^{12}$/L) | ALT (U/L) | AST (U/L) | BUN (mmol/L) | CRE (umol/L) |
|---|---|---|---|---|---|---|
| Normal control group | 7.65 ± 0.71 | 9.71 ± 0.17 | 33.44 ± 2.27 | 124.78 ± 26.67 | 12.02 ± 2.33 | 14.69 ± 0.67 |
| Model control group | **53.26 ± 21.69$^{++}$** | **5.66 ± 0.99$^{++}$** | **105.60 ± 39.48$^{++}$** | **577.00 ± 218.58$^{++}$** | **18.42 ± 5.69$^{+}$** | 17.62 ± 3.57 |
| Cisplatin group | **11.09 ± 3.64*** | 6.01 ± 0.82 | 58.25 ± 10.63 | 393.25 ± 114.89 | 7.91 ± 2.36 | 20.45 ± 3.43 |
| Kanglaite soft capsule group | 38.41 = 12.59 | 4.60 ± 0.66 | 85.00 ± 47.35 | 313.50 ± 87.43 | **20.60 ± 5.07$^{\#}$** | 16.05 ± 1.51 |
| Cisplatin + Kanglaite soft capsule group | **2.61 ± 0.64*&** | **3.94 ± 0.83$^{\#}$** | 45.33 ± 10.40 | 296.00 ± 118.49 | 11.66 ± 4.94 | 18.07 ± 2.54 |
| Cisplatin + GLP-1 low-dose group | **12.37 ± 5.46**** | 4.65 ± 0.53 | **41.00 ± 4.42*** | **292.00 ± 74.93*** | 11.19 ± 0 73 | 14.25 ± 0.60 |
| Cisplatin + GLP-1 high-dose group | **4.22 ± 0.72*&** | **6.53 ± 1.25$^{\#}$** | 52.25 ± 5.20 | **236.50 ± 62.22*** | 10.34 ± 2.54 | 15.95 ± 1.18 |

Note: Compared to the normal control group, $^{+}P \leq 0.05$, $^{++}P \leq 0.01$; compared to the model control group, $^{*}P \leq 0.05$, $^{**}P \leq 0.01$; compared to the cisplatin group, $^{\#}P \leq 0.05$, $^{\#\#}P \leq 0.01$; compared to the Kanglaite soft capsule group, $^{\&}P \leq 0.05$; compared to the cisplatin + Kanglaite soft capsule group, $^{\star}P \leq 0.05$.

**Effect of Ganoderma Lucidum Polysaccharide GLP-1 Combined with Chemotherapy on $CD3^+/CD4^+$ and $CD3^+/CD8^+$ Ratios in LLC-Bearing Mice**

As shown in Table 9, compared to the normal control group, the blood $CD3^+/CD8^+$ ratio in the mice of the model control group significantly increased ($P \leq 0.05$). There was no statistically significant difference in the treatment groups when compared to the model control group. Compared to the cisplatin group, the blood $CD3^+/CD8^+$ ratio in the mice of the Kanglaite soft capsule group significantly decreased ($P \leq 0.05$). Compared to the Kanglaite soft capsule group, the blood $CD3^+/CD8^+$ ratio in the mice of the cisplatin + Kanglaite soft capsule group significantly increased ($P \leq 0.05$). There was no significant statistical difference between treatment groups compared to the cisplatin + Kanglaite soft capsule group.

Table 9: Effect of Ganoderma lucidum polysaccharide GLP-1 combined with chemotherapy on $CD3^+/CD4^+$ and $CD3^+/CD8^+$ Ratios in LLC-Bearing Mice ($\bar{x} \pm s$)

| Group | $CD3^+/CD4^+$ | $CD3^+/CD8^+$ |
|---|---|---|
| Normal control group | 0.062 ± 0.008 | 0.283 ± 0.066 |
| Model control group | 0.124 ± 0.042 | **1.460 ± 0.706$^{+}$** |
| Cisplatin group | 0.093 ± 0.025 | 1.571 ± 0.406 |

(continued)

| Group | CD3+/CD4+ | CD3+/CD8+ |
|---|---|---|
| Kanglaite soft capsule group | 0.091 ± 0.005 | **0.561 ± 0.095#** |
| Cisplatin + Kanglaite soft capsule group | 0.174 ± 0.094 | **1.050 ± 0.172&** |
| Cisplatin + GLP-1 low-dose group | 0.131 ± 0.071 | 2.285 ± 1.077 |
| Cisplatin + GLP-1 high-dose group | 0.148 ± 0.050 | 3.195 ± 1.273 |

Note: Compared to the normal control group, $^+P \leq 0.05$; compared to the model control group, $^*P \leq 0.05$; compared to the cisplatin group, $^\#P \leq 0.05$; compared to the Kanglaite soft capsule group, $^\&P \leq 0.05$, $^{\&\&}P \leq 0.01$; compared to the cisplatin + Kanglaite soft capsule group, $^\star P \leq 0.05$, $^{\star\star}P \leq 0.01$.

**Effect of Ganoderma Lucidum Polysaccharide GLP-1 Combined with Chemotherapy on Bone Marrow Cells in LLC-Bearing Mice**

As shown in Table 10, compared to the normal control group, the model control group showed a significant increase in the mononuclear system in the bone marrow ($P \leq 0.05$), a trend toward an increase in the myeloid and erythroid system, the myeloid/erythroid ratio, and other cells, and a decreasing trend in the red cell and lymphatic systems, though these changes were not statistically significant. Compared to the model control group, the cisplatin + GLP-1 low-dose group and Kanglaite soft capsule group showed a significant increase in the red cell system ($P \leq 0.05$) and a significant decrease in the myeloid/erythroid ratio and mononuclear cell system in the bone marrow of mice ($P \leq 0.05$). The Kanglaite soft capsule group and the cisplatin + GLP-1 high-dose group showed a significant decrease in the mononuclear cell system in the bone marrow of mice ($P \leq 0.01$). There was no statistically significant difference across the treatment groups when compared to the cisplatin group. Compared to the Kanglaite soft capsule group, the cisplatin + GLP-1 low-dose and high-dose groups showed a significant increase in lymphocytes in the bone marrow of mice ($P \leq 0.05$). Compared to the cisplatin + Kanglaite soft capsule group, the cisplatin + GLP-1 high-dose group showed a significant increase in the myeloid and erythroid system in the bone marrow of mice ($P \leq 0.05$) and a significant decrease in the red cell system ($P \leq 0.05$), with no significant differences in the other groups.

Table 10: Effect of Ganoderma lucidum polysaccharide GLP-1 combined with chemotherapy on bone marrow cells in LLC-bearing mice ($\bar{x} \pm s$)

| Group | Myeloid and erythroid system | Red cell system | Myeloid/erythroid ratio | Lymphatic system | Mononuclear cell system | Other cells |
|---|---|---|---|---|---|---|
| Normal control group | 52.00 ± 4.91 | 20.50 ± 5.63 | 2.90 ± 0.69 | 25.50 ± 9.00 | 1 50 ± 0.29 | 0.50 ± 0. 00 |
| Model control group | 62.17 = 12.71 | 15.00 ± 5.27 | 7.93 ± 5.44 | 17.50=9.45 | **3.50 ± 0.76+** | 1.83 ± 0.33 |
| Cisplatin group | 56.13 ± 4.95 | 22.75 ± 5.67 | 3.68 ± 1.68 | 15.75 ± 3.44 | 2.88 ± 0.55 | 2.50 ± 2.01 |
| Kanglaite soft capsule group | 56.00 ± 7.54 | 30.63 ± 7.77 | 2.38 ± 0.76 | **8.13 ± 1.11#** | **2.00 ± 0.46*** | 3.25 ± 0.97 |
| Cisplatin + Kanglaite soft capsule group | 41.00 ± 8.58 | **38.50 ± 10.11*** | 1.33 ± 0.50 | 15.83 ± 3.88 | **2.00 ± 0.76*** | 2.67 ± 0.33 |
| Cisplatin + GLP-1 low-dose group | 47.83 ± 5.07 | **28.33 ± 2.35*** | **1.67 ± 0.09*** | **1983 ± 8.09&** | **1.83 ± 0.44*** | 217 ± 1.01 |
| Cisplatin + GLP-1 high-dose group | **63.50 ± 5.30*** | **15.67 ± 3.17*** | 4.70 ± 1.56 | **18.33 ± 1.96&** | **1.33 ± 0.60**** | 1.17 ± 0.17 |

Note: Compared to the normal control group, $^+P \leq 0.05$; compared to the model control group, $^*P \leq 0.05$, $^{**}P \leq 0.01$; compared to the cisplatin group, $^\#P \leq 0.05$, $^{\#\#}P \leq 0.01$; compared to the Kanglaite soft capsule group, $^\&P \leq 0.05$, $^{\&\&}P \leq 0.01$; compared to the cisplatin + Kanglaite soft capsule group, $^\star P \leq 0.05$, $^{\star\star}P \leq 0.01$.

**Effect of Ganoderma Lucidum Polysaccharide GLP-1 Combined with Chemotherapy on Tumor, Spleen, Kidney, and Stomach Histopathology in LLC-Bearing Mice**

As shown in Figure 21, the model control group displayed dense tumor cells around the tumor, closely arranged with large cell volume, high nucleus-to-cytoplasm ratio, and frequent mitotic figures. In the cisplatin group, large areas of necrosis and hemorrhage were visible at the tumor center, with abundant tumor cells; in the cisplatin + Kanglaite soft capsule group and cisplatin + GLP-1 high-dose group, necrosis was visible at the tumor center with reduced peripheral tumor cell numbers.

As shown in Figures 22 and 23, kidney and stomach pathology in all groups showed no significant abnormalities.

As shown in Figure 24, in the spleen, significant hematopoiesis and diverse cell types were noted in the red marrow of the model control group. The cisplatin + Kanglaite soft capsule group showed no significant abnormalities in the spleen, while varying degrees of hematopoiesis and diverse cell types were observed in the spleen of the other groups.

In Figure 21: A: Normal control group; B: Model control group; C: Cisplatin group; D: Kanglaite soft capsule group; E: Cisplatin + Kanglaite soft capsule group; F: Cisplatin + GLP-1 low-dose group, G: Cisplatin + GLP14 high-dose group

In Figures 22 and 23: A: Normal control group; B: Model control group; C: Cisplatin group; D: Kanglaite soft capsule group; E: Cisplatin + Kanglaite soft capsule group; F: Cisplatin + GLP-1 low-dose group, G: Cisplatin + GLP14 high-dose group

In Figure 24: A: Normal control group; B: Model control group; C: Cisplatin group; D: Kanglaite soft capsule group; E: Cisplatin + Kanglaite soft capsule group; F: Cisplatin + GLP-1 low-dose group, G: Cisplatin + GLP14 high-dose group

Conclusion

Ganoderma Lucidum Polysaccharide GLP-1 combined with cisplatin significantly inhibits tumor growth in LLC-bearing mice and exhibits a significant synergistic effect.

Discussion and Conclusion

In recent years, with the gradual deterioration of the environment and factors such as smoking, the incidence of lung cancer has been increasing annually, with a high mortality rate and a trend towards younger age groups. Clinically, the main treatment methods involve surgery and radiotherapy/chemotherapy to eradicate tumors early and reduce tumor burden. However, due to the high invasiveness, metastasis, and resistance to radiotherapy/chemotherapy of lung cancer cells, postoperative immune suppression occurs, which accelerates tumor metastasis and development, resulting in poor clinical prognosis.

The experimental results show that in the model control group of mice, the tumor volume significantly increased, the thymus index significantly decreased, and the spleen index significantly increased. There was a significant increase in red blood cell counts and a significant decrease in white blood cell counts in the blood, with significant abnormalities in liver and kidney functions. Additionally, there was a significant increase in $CD3^+$ level, an increase in $CD4^+$ level, and a decrease in $CD8^+$ level, indicating a decline in lymphatic system function during tumor development in the model control group.

Ganoderma lucidum polysaccharide GLP-1 combined with cisplatin significantly inhibited the growth of tumors in mice, significantly reduced the thymus and spleen indexes, and significantly decreased the number of red blood cells in mouse blood while significantly increasing white blood cell counts. Liver and kidney function indicators gradually returned to normal levels. The $CD4^+$ level in the blood of mice decreased, while the $CD8^+$ level increased, indicating that the combined application of GLP-1 and cisplatin can protect immune organs and enhance the body's own immune function, thereby playing a role in reducing toxicity and enhancing antitumor effects. Compared to the Kanglaite soft capsule group and the cisplatin + Kanglaite soft capsule group, Ganoderma lucidum polysaccharide GLP-1 + cisplatin significantly inhibited tumor growth, and the tumor inhibition effect of Ganoderma lucidum polysaccharide GLP-1 was significantly stronger than that of Kanglaite soft capsules.

In conclusion, Ganoderma Lucidum Polysaccharide GLP-1 combined with cisplatin significantly inhibits tumor growth in LLC-bearing mice and exhibits a significant synergistic effect.

**Experimental Study on the Antitumor Effect of Ganoderma Lucidum Polysaccharide GLP-1 Combined with Radiotherapy on LLC-Bearing Mice and Study Data**

**Study Objective**

To investigate the mechanism by which Ganoderma lucidum polysaccharide GLP-1 combined with radiotherapy enhances chemotherapy tolerance by utilizing C57 mice, into which LLC cells are inoculated in the right axilla to prepare

tumor-bearing models, providing experimental evidence for its clinical research.

**Experimental Materials**

**Test Sample**

Ganoderma lucidum polysaccharide GLP-1, batch number: ALM20200518A1, provided by Shenzhen Aolimei Oncology Medical Technology Co., Ltd.

**Positive Control**

Kanglaite soft capsules, batch number: 20210904, Zhejiang Kanglaite Pharmaceutical Co., Ltd.; cisplatin injection, batch number: 601211204, Jiangsu Hansoh Pharmaceutical Co., Ltd.

**Laboratory Animals**

80 SPF-grade male C57 mice, weighing 12-15 g, provided by Hunan SJA Laboratory Animal Co., Ltd. Laboratory animal production license number: SCXK (Xiang) 2019-0004; laboratory animal quality certification number: 430727211103153225. The animals were housed in Barrier Environment Laboratory D of Hunan Puruima Pharmaceutical Research Center Co., Ltd., under the laboratory animal use license number: SYXK (Xiang) 2020-0015.

**Main Reagents**

0.9% sodium chloride injection, batch number: 21071401C, Hunan Kangyuan Pharmaceutical Co., Ltd.; ALT assay kit, batch number: 201751; AST assay kit, batch number: 110620; CRE assay kit, batch number: 111644; BUN assay kit, batch number: 201749, all manufactured by Wako Pure Chemical Industries, Ltd. of Japan.

**Main Instruments**

S10 Portable High-Speed Disperser, Scientz; AR223CN Electronic Scale, Ohaus Instruments (Changzhou) Co., Ltd.; LABOSPECT003 Automatic Biochemical Analyzer, Hitachi, Japan; TDZ5-WS Benchtop Multi-Tube Automatic Balance Centrifuge, Hunan Kaida Industrial Development Co., Ltd.; ME2002E Electronic Scale, Mettler-Toledo Instruments (Shanghai) Co., Ltd.; Digital Caliper, Yongkang Zhengfeng Hardware Co., Ltd.; Flow Cytometer, BD Biosciences; ASP200S Fully Automatic Tissue Dehydrator, ASP300S Fully Automatic Tissue Dehydrator, TP1020 Fully Automatic Dehydrator, HI1210 Slide Spreader, HI1220 Slide Dryer, RM2235 Paraffin Microtome, EG1150H+C Tissue Embedding Station, AutoStainer XL Automatic Slide Stainer + CV5030 Automatic Cover Slipper, Leica, Germany; BX43 Biological Microscope + MD50 Digital Imaging System, CX31 Biological Microscope, Olympus, Japan.

**Study Content**

**Experimental Methods**

LLC cells in logarithmic growth phase at $1 \times 10^7$/mL were inoculated at 0.2 mL into the right axilla of 10 normal male C57 mice. Tumor growth was monitored, and tumor-bearing mice were prepared. Once the tumor volume in tumor-bearing mice exceeded 200 $mm^3$, the tumor tissue was aseptically harvested to prepare a tissue cell suspension. This was mixed at a 1:1 (V:V) ratio with culture medium to create a tumor tissue homogenate. This homogenate was injected into the right axilla of 72 male C57 mice at 0.2 mL per mouse. When the tumor volume in these tumor-bearing mice exceeded 100 $mm^3$, mice with well-grown tumors without ulceration were selected and randomized into groups based on tumor volume as follows: model control group, radiotherapy group (2 Gy/d), Kanglaite soft capsule group (1,404 mg/kg), radiotherapy + Kanglaite soft capsule group (2 Gy + 1,404 mg/kg), radiotherapy + GLP-1 low-dose group (2 Gy + 130 mg/kg), and radiotherapy + GLP-1 high-dose group (2 Gy + 1,170 mg/kg), with 10 mice per group. Additionally, 10 mice were selected as a normal control group. Except for the normal control group, all other animals underwent radiotherapy using an animal radiotherapy instrument. The animals were anesthetized, placed in homemade lead clothing, and the tumor tissue was exposed for radiation treatment at an intensity of 2 Gy/day for 5 consecutive days. The normal control group, model control group, and radiotherapy group were administered pure water by oral gavage. The chemotherapy group received intraperitoneal injections of cisplatin, and the other groups were administered their respective drug solutions by oral gavage (20 mL/kg) or intraperitoneal injection (10 mL/kg), once daily for 14 consecutive days. After the last dose, blood was collected from the orbital plexus to test for blood WBC, RBC, liver and kidney function indicators (ALT, AST, CRE,

BUN), and the levels of $CD3^+$, $CD4^+$, and $CD8^+$ in the blood were measured using flow cytometry; the spleen, thymus, and tumor were weighed to calculate the organ-to-body weight ratio, and histopathological examinations were performed on the spleen, thymus, stomach, kidneys, and liver.

**Dosage Design**

Based on preliminary study findings, Ganoderma lucidum polysaccharide GLP-1 was administered at a low dose of 130 mg/kg and a high dose of 1,170 mg/kg. The doses for each respective drug administered in this experiment are shown in Table 11, and the clinical intended dose was used in equivalent proportions.
The proposed clinical dose for Kanglaite soft capsules is 0.45 g per capsule, 6 capsules per dose, 4 doses per day, which totals 10.8 g per day. When converted to an equivalent mouse dose based on body surface area, it is calculated as 10.8 g/day $\times$ 0.0026 / 0.02 kg = 1,404 mg/kg. This study used the proposed clinical dose as a basis for the experiment.
The radiotherapy intensity for this study was designed to be 2 Gy per day.

Table 11: Experimental groups and dosage design

| Group | Dose (mg/kg) | Route of administration | Frequency of administration |
|---|---|---|---|
| Normal control group | - | Oral gavage | Once daily |
| Model control group | - | Oral gavage | Once daily |
| Radiotherapy group | 2Gy | Oral gavage | Radiotherapy: 2 Gy/d. once daily |
| Kanglaite soft capsule group | 1404 | Oral gavage | Once daily |
| Radiotherapy + Kanglaite soft capsule group | 2Gy+1404 | Oral gavage | Radiotherapy: 2 Gy/d; Kanglaite soft capsule: once daily |
| Radiotherapy + GLP-1 low-dose group | 2Gy+130 | Oral gavage | Radiotherapy: 2 Gy/d; GLP-1: once daily |
| Radiotherapy + GLP-1 high-dose group | 2Gy+1170 | Oral gavage | Radiotherapy: 2 Gy/d; GLP-1: once daily |

**Test Indicators**

**Efficacy Indicators**

**Evaluation of quality of life and survival time in animals:** Throughout the experimental process, detailed records were kept for each group of animals regarding weight, mortality, mental state, fur color, smoothness of fur, and fecal form, including incidences of diarrhea (if any).
**Tumor volume measurement:** Tumor dimensions (length and width) were measured with a caliper every three days post-dose, and the tumor volume was calculated as follows: tumor volume (TV) = 1 / 2 $\times$ a (length) $\times$ $b^2$ (width). Relative tumor volume (RTV) = $TV_t$ / $TV_0$, where $TV_0$ is the tumor volume at the time of administration, and $TV_t$ is the tumor volume at each measurement. Relative tumor growth rate T/C (%) = $T_{RTV}$ / $C_{RTV}$ $\times$ 100%, where $T_{RTV}$ is the RTV for the treatment group and $C_{RTV}$ is the RTV for the control group.
**Immune organs:** After the last dose, spleen and thymus weights are measured, and organ coefficients are calculated. All indicators of the animals were photographed and recorded. organ coefficient (%) = organ weight / fasting body weight $\times$ 100%
**Hematological** tests: After the last dose, routine blood tests (WBC and RBC) and biochemical tests (liver and kidney functions) were performed.
**Histopathology examination:** Thymus, spleen, nasal tissue, stomach, and kidneys were taken for HE staining, sectioning, and embedding pathology after the last dose.
**$CD4^+$ and $CD8^+$ content measurement:** After the last dose, lymphocyte subtypes $CD3^+$/$CD4^+$ and $CD3^+$/$CD8^+$ in the blood were measured using flow cytometry.

**Data Processing and Statistical Analysis**

Significant figures of the study data were rounded according to the nearest whole number. Statistical analysis was conducted in accordance with the center's *SOP,* using SPSS software. Measurement data are expressed as mean $\pm$ standard deviation ( $\bar{x} \pm s$), and normality and homogeneity of variance were tested using Leven's test. If not statistically significant ($P > 0.05$), one-way ANOVA was used for statistical analysis. If ANOVA was statistically significant ($P \leq 0.05$), the LSD test (parametric method) was used for comparison analysis. If the variance was not homogeneous ($P \leq 0.05$), the

Kruskal-Wallis test was employed. If the Kruskal-Wallis test was statistically significant ($P \leq 0.05$), the Dunnett's Test (non-parametric method) was used for comparison analysis. Statistical significance was determined at $\alpha = 0.05$, where $P \leq 0.05$ indicates statistical significance and $P \leq 0.01$ indicates highly significant differences.

## Experimental Results

### General Clinical Observation and Mortality in Animals

Prior to administration, the mice exhibited normal activity, movement, and gait. After treatment, mice in the radiotherapy group experienced a decrease in body weight. Mice 3M01, 3M10, 3M03, and 3M02 in the radiotherapy group died on D10, D11, and D12, respectively; mice 6M01 and 6M10 in the radiotherapy + Kanglaite soft capsule group died on D10; mouse 7M05 in the radiotherapy + GLP-1 low-dose group died on D7; and mice 8M08 and 8M05 in the radiotherapy + GLP-1 high-dose group died on D7 and D10, respectively.

The mortality rates for each group were as follows: 0%, 0%, 40%, 0%, 20%, 10%, and 20%.

Table 12: Statistics on the number of surviving animals and mortality rate in each group

| Group | Dose (mg/g) | Number of animals | Number of deaths | Number of surviving animals | Mortality rate (%) |
|---|---|---|---|---|---|
| Normal control group | - | 10 | 0 | 10 | 0 |
| Model control group | - | 10 | 0 | 10 | 0 |
| Radiotherapy group | 2Gy | 10 | 4 | 6 | 40 |
| Kanglaite soft capsule group | 1404 | 10 | 0 | 10 | 0 |
| Radiotherapy + Kanglaite soft capsule group | 2Gy+1404 | 10 | 2 | 8 | 20 |
| Radiotherapy + GLP-1 low-dose group | 2Gy+130 | 10 | 1 | 9 | 10 |
| Radiotherapy + GLP-1 high-dose group | 2Gy+1170 | 10 | 2 | 8 | 20 |

### Effect of Ganoderma Lucidum Polysaccharide GLP-1 Combined with Radiotherapy on Body Weight of LLC-Bearing Mice

As shown in Table 13, compared to the normal control group, the body weight of the mice in the model control group significantly decreased after administration on D1 ($P \leq 0.01$). Compared to the model control group, the body weight of mice in the radiotherapy + GLP-1 low-dose and high-dose groups, and the Kanglaite soft capsule group significantly decreased on D4 post-administration; the body weight of mice in the radiotherapy + GLP-1 low-dose and high-dose groups, the Kanglaite soft capsule group, and the radiotherapy group significantly decreased on D7 post-administration ($P \leq 0.01$); the body weight of mice in the radiotherapy + GLP-1 low-dose and high-dose groups, the Kanglaite soft capsule group, and the radiotherapy group significantly decreased on D10 post-administration ($P \leq 0.05$ or $P \leq 0.01$); the body weight of mice in the radiotherapy + GLP-1 low-dose and high-dose groups, the Kanglaite soft capsule group, and the radiotherapy group significantly decreased on D13 post-administration ($P \leq 0.05$ or $P \leq 0.01$). Compared to the radiotherapy group, the body weight of mice in the radiotherapy + GLP-1 low-dose and high-dose groups, and the Kanglaite soft capsule group significantly decreased on D4 post-administration ($P \leq 0.05$ or $P \leq 0.01$); the body weight of mice in the Kanglaite soft capsule group significantly increased from D7 to D10 post-administration ($P \leq 0.05$ or $P \leq 0.01$); the body weight of mice in the radiotherapy + Kanglaite soft capsule group significantly decreased from D10 to D13 post-administration ($P \leq 0.05$); the body weight of mice in the radiotherapy + GLP-1 low-dose group significantly decreased on D13 post-administration ($P \leq 0.05$ or $P \leq 0.01$). Compared to the Kanglaite soft capsule group, the body weight of mice in the radiotherapy + GLP-1 low-dose group, and the Kanglaite soft capsule group significantly decreased on D4 post-administration ($P \leq 0.05$ or $P \leq 0.01$); the body weight of mice in the radiotherapy + GLP-1 low-dose and high-dose groups and the Kanglaite soft capsule group significantly decreased from D7 to D13 post-administration ($P \leq 0.01$). There was no statistical difference between groups compared to the radiotherapy + Kanglaite soft capsule group.

Table 13: Effect of Ganoderma lucidum polysaccharide GLP-1 combined with radiotherapy on body weight of LLC-bearing mice (Mean ± SEM)

| Group | Weight (g) | | | | |
|---|---|---|---|---|---|
| | D1 | D4 | D7 | D10 | D13 |
| Normal control group | 19.4 ± 0.2 | 20.0 ± 0.3 | 20.2 ± 0.3 | 21.0 ± 0.4 | 21.3 ± 0 5 |

(continued)

| Group | Weight (g) | | | | |
|---|---|---|---|---|---|
| | D1 | D4 | D7 | D10 | D13 |
| Model control group | **173 ± 0.5**++ | 19.3 ± 0.3 | 20.0 ± 0.4 | 20.3 ± 0.3 | 21.2 ± 0.4 |
| Radiotherapy group | 17.3 ± 0.3 | 18.7 ± 0.2 | **15.3 ± 0.2**** | **15.9 ± 0.4**** | **17.7 ± 0.2**** |
| Kanglaite soft capsule group | 17.2 ± 0.4 | 18.5 ± 0.3 | **19.4 ± 0.3*#** | **203 ± 0.4*#** | **20.8 ± 0.4##** |
| Radiotherapy + Kanglaite soft capsule group | 17.2 ± 0.2 | **17.5 ± 0.4******** | **14.4 ± 0.4******** | **14.0 ± 0.7**#****** | **15.0 ± 0.8**##****** |
| Radiotherapy + GLP-1 low-dose group | 17.4 ± 0.4 | **17.1 ± 0.3**##****** | **14.4 ± 0.3******** | **14.8 ± 0.3******** | **153 ± 0.4**#****** |
| Radiotherapy + GLP-1 high-dose group | 17.2 ± 0.4 | **17.6 ± 0.4****** | **15.3 ± 0.4******** | **169 ± 1.0******** | **18.0 ± 1.0******** |

Note: Compared to the normal control group, ++*P* ≤ 0.01; compared to the model control group, **P* ≤ 0.05, ***P* ≤ 0.01; compared to the radiotherapy group, #*P* ≤ 0.05, ##*P* ≤ 0.01; compared to the Kanglaite soft capsule group, **P* ≤ 0.05, ***P* ≤ 0.01; compared to the radiotherapy + Kanglaite soft capsule group, ■*P* ≤ 0.05, ■■*P* ≤ 0.01

**Effect of Ganoderma Lucidum Polysaccharide GLP-1 Combined with Radiotherapy on Tumor Volume and Relative Tumor Volume in LLC-Bearing Mice**

As shown in Table 14, compared to the model control group, the tumor volume significantly decreased in the radiotherapy + GLP-1 low-dose group and the Kanglaite soft capsule group from D7 to D13 post-administration (*P* ≤ 0.05 or *P* ≤ 0.01); the tumor volume significantly decreased in the radiotherapy + GLP-1 high-dose group on D13 post-administration (*P* ≤ 0.05 or *P* ≤ 0.01); the tumor volume significantly decreased in the radiotherapy group on D7 and D13 post-administration (*P* ≤ 0.01). Compared to the radiotherapy group, the tumor volume significantly increased in the Kanglaite soft capsule group on D10 post-administration (*P* ≤ 0.01). Compared to the Kanglaite soft capsule group, the tumor volume significantly decreased in the radiotherapy + GLP-1 low-dose group and the Kanglaite soft capsule group from D7 to D13 post-administration (*P* ≤ 0.05 or *P* ≤ 0.01); the tumor volume significantly increased in the radiotherapy + GLP-1 high-dose group on D7 post-administration (*P* ≤ 0.05).

As shown in Table 15, compared to the model control group, the relative tumor volume significantly decreased in the radiotherapy + Kanglaite soft capsule group and GLP-1 low-dose group from D7 to D13 post-administration (*P* ≤ 0.05 or *P* ≤ 0.01); the relative tumor volume significantly increased in the radiotherapy group on D7 post-administration (*P* ≤ 0.05). Compared to the Kanglaite soft capsule group, the relative tumor volume significantly decreased in the radiotherapy + GLP-1 low-dose group and Kanglaite soft capsule group from D7 to D13 post-administration (*P* ≤ 0.05 or *P* ≤ 0.01).

Table 14: Effect of Ganoderma lucidum polysaccharide GLP-1 combined with radiotherapy on tumor volume of LLC-bearing mice (Mean ± SEM)

| Group | Tumor volume ($mm^3$) | | | | |
|---|---|---|---|---|---|
| | D1 | D4 | D7 | D10 | D13 |
| Model control group | 158.62 ± 27.50 | 219.97 ± 38.60 | 328.90 ± 78.75 | 684.88 ± 200.57 | 2429.66 ± 481.24 |
| Radiotherapy group | 158.51 = 28.00 | 196.80 ± 28.13 | **103.52 ± 24.02**** | 238.00 ± 83.11 | **963.85 ± 376.94**** |
| Kanglaite soft capsule group | 157.63 ± 25.68 | 254.11 ± 41.41 | 450.98 ± 132.95 | **1163.38 ± 340.63*** | 2157.52 ± 545.80 |
| Radiotherapy + Kanglaite soft capsule group | 158.21 ± 24.05 | 321.36 ± 62.07 | **125.33 ± 62.07**** | **76.63 ± 45.01****** | 258.21 ± 120.86**** |
| Radiotherapy + GLP-1 low-dose group | 157.88 ± 24.25 | 209.40 ± 42.21 | **67.24 ± 31.37******** | **121.38 ± 38.72****** | **266.09 ± 80.80****** |

(continued)

| Group | Tumor volume ($mm^3$) | | | | |
|---|---|---|---|---|---|
| | D1 | D4 | D7 | D10 | D13 |
| Radiotherapy + GLP-1 high-dose group | 158.73 = 24.08 | 249.73 ± 29.44 | **197.14 ± 70.99*** | 579.36 = 184.04 | **1065.63 ± 334.90*** |

Note: Compared to the model control group, *$P \leq 0.05$, **$P \leq 0.01$; compared to the radiotherapy group, #$P \leq 0.05$, ##$P \leq 0.01$; compared to the Kanglaite soft capsule group, ★$P \leq 0.05$, ★★$P \leq 0.01$.

Table 15: Effect of Ganoderma lucidum polysaccharide GLP-1 combined with radiotherapy on relative tumor volume of LLC-bearing mice (Mean ± SEM)

| Group | Relative tumor volume | | | |
|---|---|---|---|---|
| | D4 | D7 | D10 | D13 |
| Model control group | 1.53 ± 0.22 | 2.17 ± 0.45 | 4.87 ± 1.35 | 13.57 ± 4.16 |
| Radiotherapy group | 1.81 ± 0.46 | **1.04 ± 0.37*** | 2.36 ± 0.93 | 8.68 ± 3.3 |
| Kanglaite soft capsule group | 2.05 ± 0.43 | **3.58 ± 1.18#** | 11.14=4.27 | 19.76 ± 6.94 |
| Radiotherapy + Kanglaite soft capsule group | 2.69 ± 0.77 | **1.02 ± 0.57**** | **0.44 ± 0.24****** | **1.79 ± 0.85******* |
| Radiotherapy + GLP-1 low-dose group | 1.93 ± 0.59 | **0.48 ± 0.14****** | **1.23 ± 0.59***** | **2.49 ± 1.00****** |
| Radiotherapy + GLP-1 high-dose group | 2.25 ± 0.71 | 2.56 ± 1.75 | 5.98 ± 2.60 | 13.02 ± 5.86 |

Note: Compared to the model control group, *$P \leq 0.05$, **$P \leq 0.01$; compared to the radiotherapy group, #$P \leq 0.05$; compared to the radiotherapy + cisplatin group, &$P \leq 0.05$, &&$P \leq 0.01$; compared to the Kanglaite soft capsule group, *$P \leq 0.05$, **$P \leq 0.01$.

**Effect of Ganoderma Lucidum Polysaccharide GLP-1 Combined with Radiotherapy on Relative Tumor Growth Rate (T/C) in LLC-Bearing Mice**

As shown in Table 16, for mice in the radiotherapy + Kanglaite soft capsule group, the T/C values from D7 to D13 post-administration were 47.0%, 9.0%, and 13.2%, respectively, all ≤ 60.0%. In the radiotherapy + GLP-1 low-dose group, the T/C values from D7 to D13 post-administration were 22.1%, 25.3%, and 18.3%, respectively, all ≤ 60.0%.

Table 16: Effect of Ganoderma lucidum polysaccharide GLP-1 combined with radiotherapy on relative tumor growth rate in mice (Mean ± SEM)

| Group | Relative tumor growth rate (%) | | | |
|---|---|---|---|---|
| | D4 | D7 | D10 | D13 |
| Model control group | -- | -- | -- | -- |
| Radiotherapy group | 118.3 | **47.9** | **48.5** | 64.0 |
| Kanglaite soft capsule group | 134.0 | 165.0 | 228.7 | 145.6 |
| Radiotherapy + Kanglaite soft capsule group | 175.8 | **47.0** | **9.0** | **13.2** |
| Radiotherapy + GLP-1 low-dose group | 126.1 | **22.1** | **25.3** | **18.3** |
| Radiotherapy + GLP-1 high-dose group | 147.1 | 118 | 122.8 | 95.9 |

Note: Relative tumor growth rate: T/C (%) > 60 is ineffective; T/C (%) ≤ 60 is effective.

**Effect of Ganoderma Lucidum Polysaccharide GLP-1 Combined with Radiotherapy on Organ Coefficients and Tumor Growth Inhibition Rates in LLC-Bearing Mice**

As shown in Table 17, compared to the normal control group, the spleen and tumor organ-to-body weight ratios in the model control group significantly increased ($P \leq 0.01$), while the thymus organ-to-body weight ratio significantly decreased ($P \leq 0.05$). Compared to the model control group, the spleen coefficient significantly decreased in the radiotherapy group ($P \leq 0.01$); the spleen coefficient significantly decreased in the radiotherapy + GLP-1 high-dose group ($P \leq 0.05$); the spleen and tumor coefficients both significantly decreased in the radiotherapy + GLP-1 low-dose group ($P \leq 0.05$ or $P \leq 0.01$); the thymus and spleen coefficients significantly decreased in the radiotherapy + Kanglaite soft

capsule group ($P \leq 0.01$). Compared to the radiotherapy group, the thymus index significantly increased in the Kanglaite soft capsule group ($P \leq 0.01$). Compared to the Kanglaite soft capsule group, the spleen and tumor coefficients in the radiotherapy + GLP-1 low-dose group significantly decreased ($P \leq 0.01$). No significant differences were observed in other groups. The tumor growth inhibition rates for each group were 25.0%, -35.9%, 67.6%, 75.5%, and -8.6%, respectively.

Table 17: Effect of Ganoderma lucidum polysaccharide GLP-1 combined with radiotherapy on organ coefficients and tumor growth inhibition rates in LLC-bearing mice (Mean ± SEM)

| Group | Thymus coefficient (%) | Spleen coefficient (%) | Tumor coefficient (%) | Tumor growth inhibition rate (%) |
|---|---|---|---|---|
| Normal control group | 0.251 ± 0.017 | 0.395 ± 0.013 | 0 ± 0 | - |
| Model control group | **0.177 ± 0.019⁺** | **0.923 ± 0.144⁺⁺** | **9.332 ± 2.218⁺⁺** | - |
| Radiotherapy group | 0.130 ± 0.026 | **0.284 ± 0.024**** | 6.999 ± 2.170 | 25.0 |
| Kanglaite soft capsule group | 0.182 ± 0.026 | **0.843 ± 0.063##** | 12.680 ± 3.508 | -35.9 |
| Radiotherapy + Kanglaite soft capsule group | **0.095 ± 0.025****** | **0.258 ± 0.044****** | **3.023 ± 1.261★★** | **67.6** |
| Radiotherapy + GLP-1 low-dose group | 0.125 ± 0.015 | **0.318 ± 0.023****★★** | **2.290 ± 0.810*★★** | **75.5** |
| Radiotherapy + GLP-1 high-dose group | 0.152 ± 0.023 | **0.545 ± 0.140*** | 10.132 ± 2.654 | -8.6 |

Note: Compared to normal control group, $^{+}P \leq 0.05$, $^{++}P \leq 0.01$; compared to model control group, $^{*}P \leq 0.05$, $^{**}P \leq 0.01$; compared to radiotherapy group, $^{\#}P \leq 0.05$, $^{\#\#}P \leq 0.01$; compared to Kanglaite soft capsule group, ★$P \leq 0.05$, $^{**}P \leq 0.01$; compared to the radiotherapy + Kanglaite soft capsule group, ■$P \leq 0.05$, ■■$P \leq 0.01$.

As shown in Figure 25, the corresponding groups are as follows: 2: model control group, 3: radiotherapy group, 5: Kanglaite soft capsule group, 6: radiotherapy + Kanglaite soft capsule group, 7: radiotherapy + GLP-1 low-dose group, 8: radiotherapy + GLP-1 high-dose group

**Effect of Ganoderma Lucidum Polysaccharide GLP-1 Combined with Radiotherapy on Blood Biochemical Indicators in LLC-Bearing Mice**

As shown in Table 18, compared to the normal control group, the blood WBC, ALT, AST, and BUN levels in the model control group significantly increased ($P \leq 0.05$ or $P \leq 0.01$), and the RBC level significantly decreased ($P \leq 0.05$). Compared to the model control group, the blood WBC level in the radiotherapy + GLP-1 low-dose, high-dose, and Kanglaite soft capsule groups, and the Kanglaite soft capsule group significantly decreased post-administration ($P \leq 0.05$ or $P \leq 0.01$); the blood RBC level in the radiotherapy + GLP-1 low-dose group significantly increased post-administration ($P \leq 0.05$ or $P \leq 0.01$); the blood ALT level in the Kanglaite soft capsule group significantly decreased post-administration ($P \leq 0.05$ or $P \leq 0.01$); the blood BUN level in the radiotherapy + GLP-1 low-dose, high-dose, and Kanglaite soft capsule groups significantly decreased post-administration ($P \leq 0.05$); the blood CRE level in the radiotherapy + Kanglaite soft capsule group significantly decreased post-administration ($P \leq 0.05$). Compared to the radiotherapy group, the blood WBC level in the radiotherapy + GLP-1 low-dose, high-dose, and Kanglaite soft capsule groups significantly decreased post-administration ($P \leq 0.05$ or $P \leq 0.01$); the blood RBC level in the radiotherapy + GLP-1 low-dose group significantly increased post-administration ($P \leq 0.05$ or $P \leq 0.01$); the blood CRE level in the radiotherapy + Kanglaite soft capsule group significantly decreased post-administration ($P \leq 0.05$). Compared to the Kanglaite soft capsule group, the blood WBC level in the radiotherapy + GLP-1 low-dose, high-dose, and Kanglaite soft capsule groups significantly decreased post-administration ($P \leq 0.05$ or $P \leq 0.01$); the blood AST level in the radiotherapy + GLP-1 low-dose and Kanglaite soft capsule groups significantly decreased post-administration ($P \leq 0.05$ or $P \leq 0.01$). There was no statistical difference between groups compared to the radiotherapy + Kanglaite soft capsule group.

Table 18: Effect of Ganoderma lucidum polysaccharide GLP-1 combined with radiotherapy on blood biochemical indicators in LLC-bearing mice (Mean ± SEM)

| Group | WBC (10°/L) | RBC ($10^{12}$/L) | ALT (U/L) | AST (U/L) | BUN (mmol/L) | CRE (umol/L) |
|---|---|---|---|---|---|---|
| Normal control group | 10.58 ± 0.34 | 8.57 ± 0.19 | 34 ± 1 | 96 ± 3 | 6.95 ± 0.47 | 11.20 ± 0.65 |
| Model control group | **26.71 ± 6.00++** | **4.92 ± 0.22++** | **45 ± 4+** | **200 ± 46++** | **10.77 ± 1.21+** | 10.88 ± 0.82 |
| Radiotherapy group | 17.57± 3.38 | 4.49 ± 0.46 | 40 ± 8 | 149 ± 11 | 7 44 ± 0.70 | 10.57 ± 0.65 |
| Kanglaite soft capsule group | 18.31 ± 6.35* | 5.24 ± 0.92 | **32 ± 1**** | 229 ± 34 | 8.58 ± 0.97 | 9.81 ± 0.46 |
| Radiotherapy + Kanglaite soft capsule group | **1.84 ± 0.24***#**** | 5.41 ± 1.04 | 41 ± 7 | **122 ± 12*** | **7.65 ± 0.57*** | **8.64 ± 0.36**** |
| Radiotherapy + GLP-1 low-dose group | **2.52 ± 0.32**##**** | **6.81 ± 0.45**#** | 46 ± 5 | **130 ± 15*** | **7.54 ± 0.28*** | 9.37 ± 0.60 |
| Radiotherapy + GLP-1 high-dose group | **5.72 ± 1.37**#**** | 623 ± 0.55 | 36 ± 3 | 223 ± 60 | **7.48 ± 0.82*** | 9.70 ± 0.32 |

Note: Compared to normal control group, $^{+}P \leq 0.05$, $^{++}P \leq 0.01$; compared to model control group, $^{*}P \leq 0.05$, $^{**}P \leq 0.01$; compared to radiotherapy group, $^{\#}P \leq 0.05$, $^{\#\#}P \leq 0.01$; compared to Kanglaite soft capsule group, $^{\star}P \leq 0.05$, $^{\star\star}P \leq 0.01$; compared to the radiotherapy + Kanglaite soft capsule group, $^{\blacksquare}P \leq 0.05$.

**Effect of Ganoderma Lucidum Polysaccharide GLP-1 Combined with Radiotherapy on $CD3^+/CD4^+$ and $CD3^+/CD8^+$ Ratios in LLC-Bearing Mice**

As shown in Table 19, compared to the normal control group, the blood $CD3^+/CD8^+$ ratio in the mice of the model control group significantly increased ($P \leq 0.05$). Compared to the normal control group, the blood $CD3^+/CD4^+$ ratio in the mice of the radiotherapy + Kanglaite soft capsule group and the Kanglaite soft capsule group significantly decreased ($P \leq 0.05$); the blood $CD3^+/CD8^+$ ratio in the mice of the radiotherapy + Kanglaite soft capsule group and the Kanglaite soft capsule group significantly decreased ($P \leq 0.05$). Compared to the radiotherapy group, the blood $CD3^+/CD4^+$ ratio in the mice of the radiotherapy + Kanglaite soft capsule group and the Kanglaite soft capsule group significantly decreased ($P \leq 0.05$); the blood $CD3^+/CD8^+$ ratio in the mice of the radiotherapy + Kanglaite soft capsule group and the Kanglaite soft capsule group significantly decreased ($P \leq 0.05$). No significant differences were observed in the other groups.

Table 19: Effect of Ganoderma lucidum polysaccharide GLP-1 combined with radiotherapy on $CD3^+/CD4^+$ and $CD3^+/CD8^+$ ratios in LLC-bearing mice (Mean ± SEM)

| Group | $CD3^+/CD4^+$ | $CD3^+/CD8^+$ |
|---|---|---|
| Normal control group | 8.96 ± 0.91 | 40.44 ± 3.61 |
| Model control group | 12.27 ± 2.67 | **117.06 ± 58.00+** |
| Radiotherapy group | 11.82 ± 0.55 | 59.35 ± 6.69 |
| Kanglaite soft capsule group | **4.36 ± 0.70*#** | **18.41 ± 5.64*#** |
| Radiotherapy + Kanglaite soft capsule group | **4.41 ± 0.76*#** | **14.69 ± 3.76*#** |
| Radiotherapy + GLP-1 low-dose group | 6.89 ± 1.08 | 104.82 ± 43.62 |
| Radiotherapy + GLP-1 high-dose group | 8.30 ± 0.57 | 147.85 ± 54.04 |

Note: Compared to the normal control group, $^{+}P \leq 0.05$; compared to the model control group, $^{*}P \leq 0.05$; compared to the radiotherapy group, $^{\#}P \leq 0.05$, $^{\#\#}P \leq 0.01$; compared to the Kanglaite soft capsule group, $^{\star}P \leq 0.05$, $^{**}P \leq 0.01$; compared to the radiotherapy + Kanglaite soft capsule group, $^{\blacksquare}P \leq 0.05$.

**Effect of Ganoderma Lucidum Polysaccharide GLP-1 Combined with Radiotherapy on the Pathology of Thymus, Spleen, Nasal, Gastric, and Renal Tissues in LLC-Bearing Mice**

As shown in the results of Figures 26, 27, 28, 29, and 30, the model control group mice exhibited diffuse red pulp in the spleen and increased extramedullary hematopoiesis. The radiotherapy group showed a reduction in thymic lymphocyte count, diffuse medulla; increased extramedullary hematopoiesis and reduced white pulp lymphocyte count in the spleen; and multiple hemorrhages in the nasal turbinates' own membrane. After treatment with Ganoderma lucidum polysaccharide GLP-1 combined with radiotherapy, there was a decrease in medullary lymphocyte count in the thymus, an increase in plasma cells in the spleen's white pulp, and no abnormal lesions were observed in the nasal turbinates. No abnormal lesions were observed in the stomach and kidneys in any treatment groups.

In Figures 26, 27, 28, 29 and 30: A: Normal control group, B: Model control group, C: Radiotherapy group; E: Kanglaite soft capsule group; F: Radiotherapy + Kanglaite soft capsule group; G: Radiotherapy + GLP-1 low-dose group, H: Radiotherapy + GLP-1 high-dose group.

**Conclusion**

Ganoderma Lucidum Polysaccharide GLP-1 combined with radiotherapy significantly inhibits tumor growth in LLC-bearing mice and exhibits a significant synergistic effect.

**Discussion and Conclusion**

In recent years, with the gradual deterioration of the environment and factors such as smoking, the incidence of lung cancer has been increasing annually, with a high mortality rate and a trend towards younger age groups. Clinically, the main treatment methods involve surgery and radiotherapy/chemotherapy to eradicate tumors early and reduce tumor burden. However, due to the high invasiveness, metastasis, and resistance to radiotherapy/chemotherapy of lung cancer cells, postoperative immune suppression occurs, which accelerates tumor metastasis and development, resulting in poor clinical prognosis.

The experimental results show that in the model control group of mice, the tumor volume significantly increased, the thymus index significantly decreased, and the spleen index significantly increased. There was a significant increase in red blood cell counts and a significant decrease in white blood cell counts in the blood, with significant abnormalities in liver and kidney functions. Additionally, there was an increase in $CD4^+$ level and a significant decrease in $CD8^+$ level, indicating a decline in lymphatic system function during tumor development in the model control group. The radiotherapy group exhibited significant reductions in tumor volume and body weight, with a mortality rate of 40%. The experimental results indicate that Ganoderma lucidum polysaccharide GLP-1 combined with radiotherapy significantly inhibited the growth of mouse tumors, significantly reduced the thymus and spleen indices, significantly lowered the number of erythrocytes in the blood, and increased the number of leukocytes. Liver and kidney function indicators also showed improvements. Histopathological results indicated that Ganoderma lucidum polysaccharide GLP-1 increased the number of plasma cells in the spleen's white pulp. The combined use of GLP-1 and radiotherapy can protect immune organs and enhance the body's own immune function, thereby reducing toxicity and enhancing antitumor effects. Compared to the Kanglaite soft capsule group and the radiotherapy + Kanglaite soft capsule group, Ganoderma lucidum polysaccharide GLP-1 + radiotherapy significantly inhibited tumor growth, and the tumor inhibition effect of Ganoderma lucidum polysaccharide GLP-1 was significantly stronger than that of Kanglaite soft capsules.

In conclusion, ganoderma Lucidum Polysaccharide GLP-1 combined with radiotherapy significantly inhibits tumor growth in LLC-bearing mice and exhibits a significant synergistic effect.

**Experimental Study on the Antitumor Effect of Ganoderma Lucidum Polysaccharide GLP-1 Combined with Chemotherapy on H22-Bearing Mice and Study Data**

**Experimental Objective**

To study the antitumor effect of Ganoderma lucidum polysaccharide GLP-1 on hepatoma-bearing mice which are prepared by implanting H22 hepatoma tumor homogenate in the right axilla of C57 mice. This study aims to provide experimental evidence for clinical studies of GLP-1.

**Experimental Materials**

**Test Sample**

Ganoderma lucidum polysaccharide GLP-1, batch number: H2202208151A1, provided by Shenzhen Aolimei Oncology Medical Technology Co., Ltd.

**Positive Control**

Cisplatin, batch number: E21268081, product of Shanghai Aladdin Biochemical Technology Co., Ltd.

**Laboratory Animals**

80 SPF male C57 mice, weighing 16-18 g, supplied by Guangdong Medical Laboratory Animal Center. Laboratory animal production license number: SCXK (Yue) 2022-0002; laboratory animal quality certification number: 44007200107158.

**Main Reagents**

PBS buffer, prepared by Shenzhen Aolimei Oncology Medical Technology Co., Ltd.; fetal bovine serum, product of Zhejiang Tianhang Biotechnology Co., Ltd.; 1640 culture medium, product of Gibco; 0.25% trypsin, product of Gibco.

**Main Instruments**

Vernier caliper, product of Shanghai Tool Works Co., Ltd.; I-2000 scale, Dongguan Nancheng Changxie Electronic Products Factory; ophthalmic scissors and tweezers, products of Shanghai Jinzhong Medical Instrument Co., Ltd.; CCL-170B-8 $CO_2$ incubator, product of ESCO, Singapore; Luna-II cell counter, product of Nanjing Hengqiao Instrument Co., Ltd.

**Experimental Methods**

A homogenate suspension of H22 hepatoma solid tumors was injected subcutaneously under the axilla of 65 healthy male C57 mice to create a solid tumor model. Once all mouse tumors averaged a volume of about 230 mm3, mice were randomized into groups based on tumor volume and were administered the respective drugs or drug solvents via oral gavage or intraperitoneal injection for 25 consecutive days. Longest and shortest diameters of tumors were measured every three days to calculate tumor volume, and mouse weights were recorded every three days. At the end of the experiment, tumors, spleens, and thymuses were harvested and weighed to calculate tumor, spleen, and thymus indices.

**Dosage Design**

Based on previous experimental results, Ganoderma lucidum polysaccharide GLP-1 was administered at a low dose of 50 mg/kg and a high dose of 150 mg/kg as shown in Table 20.

Rationale for cisplatin dosage design: Based on the clinical dosage of cisplatin, which should not exceed 100 $mg/m^2$ per person per day, and considering the tolerance of mice to cisplatin, a dose of 3 mg/kg has been selected as the administration dosage.

Table 20: Experimental groups and dosage design

| Group | Dose (mg/kg) | Method of administration | Administration volume (mL/10 g) | Frequency of administration |
|---|---|---|---|---|
| Model control group | - | Oral gavage | 0.2 | Once daily |
| Cisplatin | 3 | Intraperitoneal injection | 0.2 | Once every 3 days |
| Cisplatin + GLP-1 low-dose group | 3+50 | Intraperitoneal injection + oral gavage | 0.2+0.2 | Cisplatin, once every 3 days; GLP-1, once daily |
| Cisplatin + GLP-1 high-dose group | 3+150 | Intraperitoneal injection + oral gavage | 0.2+0.2 | Cisplatin, once every 3 days; GLP-1, once daily |
| Normal group | - | Oral gavage | 0.2 | Once daily |

**Test Indicators**

**Efficacy Indicators**

**Relative tumor growth inhibition rate**

Relative tumor growth inhibition rate (%) = (1 - $T_{RTV}$ / $C_{RTV}$) × 100%. Where $T_{RTV}$ is the relative tumor volume in the experimental group, and $C_{RTV}$ is the relative tumor volume in the model control group. Relative tumor volume (RTV) = $V_t$ / $V_0$, where $V_t$ is the tumor volume on day t of dosing, and $V_0$ is the tumor volume at the time of grouping. Evaluation criteria: A relative tumor growth inhibition rate of ≥ 40% and a statistical analysis with P < 0.05 indicate effective inhibition.

**Tumor growth inhibition rate**

Tumor growth inhibition rate (%) = (1 - T / C) × 100%. Where T represents the average tumor weight in the treatment group, and C represents the average tumor weight in the model control group. Evaluation criteria: A tumor growth inhibition rate of ≥ 40% and a statistical analysis with P < 0.05 indicate effective inhibition.

**Spleen and thymus organ coefficients:** After the last dose, spleen, thymus, and tumor weights are measured, and organ coefficients are calculated.

$$\text{Tumor index (\%)} = (\text{tumor weight / body weight}) \times 100\%.$$

$$\text{Immune organ index (mg/g)} = (\text{organ mass / body weight}) \times 1000.$$

**Data Processing and Statistical Analysis**

Statistical analyses were performed using SPSS 17.0, with the significance level set at P ≤ 0.05. Measurement data were expressed as mean ± standard deviation ($\bar{x} \pm s$). Normality and homogeneity of variance were tested using Leven's test. If data met normality and homogeneity of variance (P > 0.05), one-way ANOVA and LSD test were used for statistical analysis. If data did not meet normality and homogeneity of variance (P < 0.05), the Kruskal-Wallis test was used. If the Kruskal-Wallis test was statistically significant (P < 0.05), comparison analysis was performed using Dunnett's Test (a non-parametric method). Evaluation considered statistical differences and biological significance.

**Experimental Results**

**Animal Mortality**

As shown in Table 21, the mortality rate for all groups of mice was 0.

Table 21: Statistics on the number of surviving animals and mortality rate in each group

| Group | Total number of animals | Number of deaths | Mortality rate (%) | Survival time (days) |
|---|---|---|---|---|
| Model control group | 8 | 0 | 0 | 25±0 |
| Cisplatin | 8 | 0 | 0 | 25±0 |
| Cisplatin + GLP-1 low-dose group | 8 | 0 | 0 | 25±0 |
| Cisplatin + GLP-1 high-dose group | 8 | 0 | 0 | 25±0 |
| Normal group | 8 | 0 | 0 | 25±0 |

**Effect of Ganoderma Lucidum Polysaccharide GLP-1 Combined with Chemotherapy on Body Weight of H22-Bearing Mice**

As indicated in Table 22, compared to the normal group, the body weight of mice in the model control group significantly increased from D21 to D25, the body weight of mice in the cisplatin group significantly decreased from D18 to D25, the body weight of mice in the cisplatin + GLP-1 low-dose group significantly decreased from D15 to D25, and the body weight of mice in the cisplatin + GLP-1 high-dose group significantly decreased from D12 to D25. Compared to the

model control group, the body weight of mice in the cisplatin group and the cisplatin + GLP-1 low-dose group significantly decreased from D9 to D25, and the body weight of mice in the cisplatin + GLP-1 high-dose group significantly decreased from D6 to D25. Compared to the cisplatin group, the body weight of mice in the cisplatin + GLP-1 high-dose group significantly decreased on D25.

Table 22: Effect of Ganoderma lucidum polysaccharide GLP-1 combined with chemotherapy on body weight of H22-bearing mice ($\bar{x} \pm s$)

| **Group** | Weight (g) | | | | | | | | |
|---|---|---|---|---|---|---|---|---|---|
| | D0 | D3 | D6 | D9 | D12 | D15 | D18 | D21 | D25 |
| Model control group | 20.4±1.8 | 21.3±1.7 | 21.7±1.8 | 22.1±1.9 | 22.6±2.3 | 23.2±2.4 | 24.6±2.9 | **25.4±3.2$^{+}$** | **26.8±3$^{+}$** |
| Cisplatin | 21.4±0.6 | 21.1±0.9 | 20.6±0.8 | **20.1±1*** | **19.5±0.9*** | **19.2±1.2*** | **18.4±1.3$^{+*}$** | **17.8±1.5$^{+*}$** | **16.9±1.6$^{+*}$** |
| Cisplatin + GLP-1 low-dose group | 21.4±1.5 | 21.3±1.6 | 20.8±1.8 | **20.5±1.8*** | **20.1±1.8*** | **19.3±1.8$^{+*}$** | **18.4±1.8$^{+*}$** | **17.6±1.7$^{+*}$** | **16.5±1.6$^{+*}$** |
| Cisplatin + GLP-1 high-dose group | 21±1 | 20.8±1.2 | 20.1±1.2* | **19.6±1.2*** | **18.8±1.2$^{+*}$** | **18.1±1.2$^{+*}$** | **17.3±1.2$^{+*}$** | **16.3±1.1$^{+*}$** | **14.9±1$^{+*\#}$** |
| Normal group | 19.1±1.6 | 20.2±1.6 | 20.5±1.1 | 20.8±1.4 | 21.1±1.4 | 21.4±1.3 | 22±1.5 | 22.1±1.7 | 22.5±1.8 |

Note: Compared to the normal control group, $^{+}P \le 0.05$; compared to the model control group, $^{*}P < 0.05$; compared to the cisplatin group, $^{\#}P < 0.05$.

**Effect of Ganoderma Lucidum Polysaccharide GLP-1 Combined with Chemotherapy on Tumor Volume in H22-Bearing Mice**

As shown in Table 23, compared to the model control group, the tumor volume in the cisplatin group, the cisplatin + GLP-1 low-dose group, and the cisplatin + GLP-1 high-dose group was significantly reduced from D3 to D25.

Compared to the cisplatin group, the tumor volume in the cisplatin + GLP-1 low-dose group and cisplatin + GLP-1 high-dose group was significantly reduced from D15 to D25.

Table 23: Effect of Ganoderma lucidum polysaccharide GLP-1 combined with chemotherapy on tumor volume in H22-bearing mice ($\bar{x} \pm s$)

| Group | Tumor volume ($mm^3$) | | | | | | | | |
|---|---|---|---|---|---|---|---|---|---|
| | D0 | D3 | D6 | D9 | D12 | D15 | D18 | D21 | D25 |
| Model control group | 240.7±74.4 | 376.6±135 | 453.4±121.6 | 728.8±234.1 | 1368.2±1469.6 | 1426.9±1309.2 | 1734.7±1579.4 | 2169±1896.1 | 3486.7±2196.2 |
| Cisplatin | 241.8±75 | **283.3±79.5*** | **326.7±96.1*** | **456.8±149.9*** | **437.8±146.1*** | **480.6±239.3*** | **425.3±208.5*** | **649.9±311.2*** | **462.4±199.5*** |
| Cisplatin + GLP-1 low-dose group | 219.9±65.7 | **236.7±86.5*** | **308.5±99.3*** | **366±109.8*** | **390±105*** | **242.5±92.8*#** | **227.9±95.9*#** | **256.3±114.9*#** | **269.3±152.8*#** |
| Cisplatin + GLP-1 high-dose group | 231.5±72.1 | **241.3±65.9*** | **314.7±38.2*** | **349.2±47.8*** | **335.4±45.5*** | **202.7±33.8*#** | **164.8±34.2*#** | **196.6±80.2*#** | **182.5±41.3*#** |

Note: Compared to the model control group, *$P$ < 0.05; Compared to the cisplatin group, #$P$ < 0.05.

**Effect of Ganoderma Lucidum Polysaccharide GLP-1 Combined with Chemotherapy on Relative Tumor Growth Inhibition Rate in H22-Bearing Mice**

As shown in Table 24, compared with the model control group, the relative tumor growth inhibition rate in the cisplatin group was above 60% from D12 and up to 86.6%, and the relative tumor growth inhibition rate in the cisplatin + GLP-1 low-dose group and high-dose group was above 40% from D9 and up to 94.3%. Compared to the cisplatin group, the relative tumor growth inhibition rate for the cisplatin + GLP-1 low-dose group ranged from 30.4% to 54.8% ($P < 0.05$) from D15 to D25, and the relative tumor growth inhibition rate for the cisplatin + GLP-1 high-dose group ranged from 52.4% to 69.0% ($P < 0.05$) from D15 to D25.

Table 24: Effect of Ganoderma lucidum polysaccharide GLP-1 combined with chemotherapy on relative tumor growth inhibition rate in H22-bearing mice (vs. the model control group)

| Group | Relative tumor growth inhibition rate (compared to model control group) (%) | | | | | | | |
|---|---|---|---|---|---|---|---|---|
| | D3 | D6 | D9 | D12 | D15 | D18 | D21 | D25 |
| Model control group | - | - | - | - | - | - | - | - |
| Cisplatin | **22.2*** | **28.5*** | **37.9*** | **64.1*** | **64.5*** | **73.9*** | **68.4*** | **86.6*** |
| Cisplatin + GLP-1 low-dose group | **30.7*** | **26.6*** | **43.1*** | **63.5*** | **78.4*** | **83.2*** | **85.7*** | **90.7*** |
| Cisplatin + GLP-1 high-dose group | **29.6*** | **25.1*** | **46.0*** | **69.5*** | **83.1*** | **88.9*** | **90.$^{2*}$** | **94.3*** |

Note: Compared to the model control group, $^{*}P < 0.05$.

Table 25: Effect of Ganoderma lucidum polysaccharide GLP-1 combined with chemotherapy on relative tumor growth inhibition rate in H22-bearing mice (vs. the cisplatin group)

| Group | Relative tumor growth inhibition rate (compared to cisplatin group) (%) | | | | | | | |
|---|---|---|---|---|---|---|---|---|
| | D3 | D6 | D9 | D12 | D15 | D18 | D21 | D25 |
| Cisplatin | - | - | - | - | - | - | - | - |
| Cisplatin + GLP-1 low-dose group | 10.84 | -2.60 | 8.4 | -1.6 | **39.1#** | **35.6#** | **54.8#** | **30.4#** |
| Cisplatin + GLP-1 high-dose group | 9.40 | -4.69 | 13.1 | 14.9 | **52.4#** | **57.4#** | **69.0#** | **57.8#** |

Note: Compared to the cisplatin group, $^{\#}P < 0.05$.

**Effect of Ganoderma Lucidum Polysaccharide GLP-1 Combined with Chemotherapy on Organ Coefficients and Tumor Growth Inhibition Rates in H22-Bearing Mice**

As shown in Table 26, compared to the model control group, the tumor index, spleen index, and thymus index was significantly reduced in the cisplatin group and the cisplatin + GLP-1 group. Compared to the cisplatin group, the tumor index was significantly reduced in the cisplatin + GLP-1 high-dose group, and there was no significant difference in the spleen index and thymus index in the cisplatin + GLP-1 group. The thymus index was significantly reduced in the cisplatin + GLP-1 group compared to the normal group.

Compared to the model control group, the tumor growth inhibition rate was 84.2% in the cisplatin group, and 88.2% and 92.8% in the cisplatin + GLP-1 low-dose and high-dose groups, respectively. Compared to the cisplatin group, the tumor growth inhibition rate was 25.3% and 54.4% in the cisplatin + GLP-1 low-dose and high-dose groups, respectively.

Table 26: Effect of Ganoderma lucidum polysaccharide GLP-1 combined with chemotherapy on organ indices and tumor growth inhibition rates in H22-bearing mice

| Group | Tumor index (%) | Spleen index (mg/g) | Thymus index (mg/g) | Tumor growth inhibition rate (%) vs. the model control group | Tumor growth inhibition rate (%) vs. the cisplatin group |
|---|---|---|---|---|---|
| Model control group | 15±9.8 | 5±1.6 | 1.3±0.3 | - | - |
| Cisplatin | **3.8±2.3*** | **2.2±0.2*** | **0.5±0$^{+*}$** | 84.2 | - |

(continued)

| Group | Tumor index (%) | Spleen index (mg/g) | Thymus index (mg/g) | Tumor growth inhibition rate (%) vs. the model control group | Tumor growth inhibition rate (%) vs. the cisplatin group |
|---|---|---|---|---|---|
| Cisplatin + GLP-1 low-dose group | **3±2.2*** | **2.3±0.4*** | **0.5±0.2+*** | 88.2 | 25.3 |
| Cisplatin + GLP-1 high-dose group | **2±1*#** | **2.2±0.1*** | **0.4±0.1+*** | 92.8 | 54.4 |
| Normal group | - | 3.2±0.5 | 1.8±0.2 | - | - |

Note: Compared to the normal control group, $^{+}P \leq 0.05$; compared to the model control group, $^{*}P < 0.05$; compared to the cisplatin group, $^{\#}P < 0.05$.

Figures 31, 32, 33, and 34 show images of the tumor-bearing mice, corresponding to the groups as follows: Figure 31: model control group, Figure 32: cisplatin group, Figure 33: cisplatin + GLP-1 low-dose group, Figure 34: cisplatin + GLP-1 high-dose group.

Figures 35, 36, 37, and 38 show images of the tumors in the tumor-bearing mice, corresponding to the groups as follows: Figure 35: model control group, Figure 36: cisplatin group, Figure 37: cisplatin + GLP-3 low-dose group, Figure 38: cisplatin + GLP-3 high-dose group.

Conclusion

Ganoderma Lucidum Polysaccharide GLP-1 combined with cisplatin significantly inhibits tumor growth in H22-bearing mice and exhibits a significant synergistic effect.

**Experimental Study on the Antitumor Effect of Ganoderma Lucidum Polysaccharide GLP-1 Combined with Chemotherapy on 4T1 Breast Tumor-Bearing Mice and Study Data**

**Experimental Objective**

To study the antitumor effects of Ganoderma lucidum polysaccharide on breast cancer-bearing mice using BALB/c mice subcutaneously inoculated with 4T1 breast cancer cells on their left shoulder back, providing experimental evidence for its clinical research.

**Experimental Materials**

**Test Sample**

Ganoderma lucidum polysaccharide GLP-1, batch number: H20220706A, provided by Shenzhen Aolimei Oncology Medical Technology Co., Ltd.

**Positive Control**

Cisplatin, batch number: E21268081, product of Shanghai Aladdin Biochemical Technology Co., Ltd.

**Laboratory Animals**

70 SPF female BALB/c mice, weighing 16-18 g, supplied by Guangdong Medical Laboratory Animal Center. Laboratory animal production license number: SCXK (Yue) 2022-0002; laboratory animal quality certification number: 44007200113250.

**Main Reagents**

PBS buffer, prepared by Shenzhen Aolimei Oncology Medical Technology Co., Ltd.; fetal bovine serum, product of Zhejiang Tianhang Biotechnology Co., Ltd.; 1640 culture medium, product of Gibco; 0.25% trypsin, product of Gibco.

### Main Instruments

Vernier caliper, product of Shanghai Tool Works Co., Ltd.; I-2000 scale, Dongguan Nancheng Changxie Electronic Products Factory; ophthalmic scissors and tweezers, products of Shanghai Jinzhong Medical Instrument Co., Ltd.; CCL-170B-8 $CO_2$ incubator, product of ESCO, Singapore; Luna-II cell counter, product of Nanjing Hengqiao Instrument Co., Ltd.

### Experimental Methods

A suspension of 4T1 breast cancer cells was injected subcutaneously into the left shoulder back of 60 healthy female BALB/c mice to create solid tumor models. When the mean tumor volume reached approximately 190 $mm^3$, the mice were randomized into groups based on tumor volume, and were administered the respective drugs or drug solvents via oral gavage or intraperitoneal injection over 18 consecutive days. Longest and shortest diameters of tumors were measured every three days to calculate tumor volume, and mouse weights were recorded every three days. At the end of the experiment, tumors, spleens, and thymuses were harvested and weighed to calculate tumor, spleen, and thymus indices.

### Dosage Design

Based on previous experimental results, Ganoderma lucidum polysaccharide GLP-1 was administered at a low dose of 50 mg/kg and a high dose of 150 mg/kg as shown in Table 27.

Rationale for cisplatin dosage design: Based on the clinical dosage of cisplatin, which should not exceed 100 $mg/m^2$ per person per day, and considering the tolerance of mice to cisplatin, a dose of 4 mg/kg has been selected as the administration dosage.

**Table 27: Experimental groups and dosage design**

| Group | Dose (mg/kg) | Method of administration | Administration volume (mL/10 g) | Frequency of administration |
|---|---|---|---|---|
| Model control group | - | Oral gavage | 0.2 | Once daily |
| Cisplatin | 4 | Intraperitoneal injection | 0.2 | Once every 3 days |
| Cisplatin + GLP-1 low-dose group | 4+50 | Intraperitoneal injection + oral gavage | 0.2+0.2 | Cisplatin, once every 3 days; GLP-1, once daily |
| Cisplatin + GLP-1 high-dose group | 4+150 | Intraperitoneal injection + oral gavage | 0.2+0.2 | Cisplatin, once every 3 days; GLP-1. once daily |
| Normal group | - | Oral gavage | 0.2 | Once daily |

### Test Indicators

### Efficacy Indicators

#### Relative tumor growth inhibition rate

Relative tumor growth inhibition rate (%) = $(1 - T_{RTV} / C_{RTV}) \times 100\%$. Where $T_{RTV}$ is the relative tumor volume in the experimental group, and $C_{RTV}$ is the relative tumor volume in the model control group. Relative tumor volume (RTV) = $V_t / V_0$, where $V_t$ is the tumor volume on day t of dosing, and $V_0$ is the tumor volume at the time of grouping. Evaluation criteria: A relative tumor growth inhibition rate of ≥ 40% and a statistical analysis with $P < 0.05$ indicate effective inhibition.

#### Tumor growth inhibition rate

Tumor growth inhibition rate (%) = $(1 - T / C) \times 100\%$. Where T represents the average tumor weight in the treatment group, and C represents the average tumor weight in the model control group. Evaluation criteria: A tumor growth inhibition rate of ≥ 40% and a statistical analysis with $P < 0.05$ indicate effective inhibition.

**Spleen and thymus organ coefficients:** After the last dose, spleen, thymus, and tumor weights are measured, and organ coefficients are calculated.

$$\text{Tumor index (\%)} = (\text{tumor weight / body weight}) \times 100\%.$$

$$\text{Immune organ index (mg/g)} = (\text{organ mass / body weight}) \times 1000.$$

**Data Processing and Statistical Analysis**

Statistical analyses were performed using SPSS 17.0, with the significance level set at $P \leq 0.05$. Measurement data were expressed as mean ± standard deviation ($\bar{x} \pm s$). Normality and homogeneity of variance were tested using Leven's test. If data met normality and homogeneity of variance ($P > 0.05$), one-way ANOVA and LSD test were used for statistical analysis. If data did not meet normality and homogeneity of variance ($P < 0.05$), the Kruskal-Wallis test was used. If the Kruskal-Wallis test was statistically significant ($P < 0.05$), comparison analysis was performed using Dunnett's Test (a non-parametric method). Evaluation considered statistical differences and biological significance.

**Experimental Results**

**Animal Mortality**

As shown in Table 28, the mortality rate of mice in the model control group, the cisplatin group, and the cisplatin + GLP-1 low-dose group was 12.5%, and the mortality rate of mice in the rest of the groups was 0.

Table 28: Statistics on the number of surviving animals and mortality rate in each group

| Group | Total number of animals | Number of deaths | Mortality rate (%) | Survival time (days) |
|---|---|---|---|---|
| Model control group | 8 | 1 | 12.5 | 18±0 |
| Cisplatin | 8 | 1 | 12.5 | 17.1±2.3 |
| Cisplatin + GLP-1 low-dose group | 8 | 1 | 12.5 | 17.5±1.3 |
| Cisplatin + GLP-1 high-dose group | 8 | 0 | 0 | 18±0 |
| Normal group | 8 | 0 | 0 | 18±0 |

**Effect of Ganoderma Lucidum Polysaccharide GLP-1 Combined with Chemotherapy on Body Weight of 4T1 Breast Tumor-Bearing Mice**

As indicated in Table 29, compared to the normal group, the body weight of mice in the model control group significantly increased from D6 to D18, the body weight of mice in the cisplatin + GLP-1 low-dose group significantly decreased from D6 to D18, and the body weight of mice in the cisplatin group and the cisplatin + GLP-1 high-dose group significantly decreased from D3 to D18. Compared to the model control group, the body weight of mice in the cisplatin group, cisplatin + GLP-1 low-dose group, and cisplatin + GLP-1 high-dose group significantly decreased from D3 to D18. Compared to the cisplatin group, the body weight of mice in the cisplatin + GLP-1 high-dose group significantly decreased on D6.

Table 29: Effect of Ganoderma lucidum polysaccharide GLP-1 combined with chemotherapy on body weight of 4T1 breast tumor-bearing mice ($\bar{x} \pm s$)

| Group | Weight (g) | | | | | | |
|---|---|---|---|---|---|---|---|
| | D0 | D3 | D6 | D9 | D12 | D15 | D18 |
| Model control group | 19.7±1.2 | 20.8±1.2 | **22±0.9$^{+}$** | **22.3±1.1$^{+}$** | **23.1±1$^{+}$** | **22.8±0.8$^{+}$** | **22.7±0.8$^{+}$** |
| Cisplatin | 19.3±0.7 | **18.7±1.1$^{+*}$** | **17.8±1.3$^{+*}$** | **14.8±1.7$^{+*}$** | **14±0.9$^{+*}$** | **14.6±0.8$^{+*}$** | **14.7±0.6$^{+*}$** |
| Cisplatin + GLP-1 low-dose group | 19.7±0.8 | **19.4±0.8$^{*}$** | **16.7±1.7$^{+*}$** | **14.8±1.3$^{+*}$** | **13.8±1.4$^{+*}$** | **15.3±0.8$^{+*}$** | **14.7±0.8$^{+*}$** |

(continued)

| Group | Weight (g) | | | | | | |
|---|---|---|---|---|---|---|---|
| | D0 | D3 | D6 | D9 | D12 | D15 | D18 |
| Cisplatin + GLP-1 high-dose group | 19.5±0.4 | **19±0.4**$^{+*}$ | **16.3±0.8**$^{+*\#}$ | **14.5±1.6**$^{+*}$ | **14±1.1**$^{+*}$ | **14.1±1**$^{+*}$ | **13.9±1**$^{+*}$ |
| Normal group | 19.3±1.3 | 20.2±1 | **20.7+1.1**$^{*}$ | **20.6±1.3**$^{*}$ | **20.7±1.5**$^{*}$ | **21±1.5**$^{*}$ | **21.3±1.8**$^{*}$ |
| Note: Compared to the normal control group, $^{+}P \leq 0.05$; compared to the model control group, $^{*}P < 0.05$; compared to the cisplatin group, $^{\#}P < 0.05$. | | | | | | | |

**Effect of Ganoderma Lucidum Polysaccharide GLP-1 Combined with Chemotherapy on Tumor Volume in 4T1 Breast Tumor-Bearing Mice**

As shown in Table 30, compared to the model control group, the tumor volume in the cisplatin group, the cisplatin + GLP-1 low-dose group, and the cisplatin + GLP-1 high-dose group was significantly reduced from D3 to D18.

Compared to the cisplatin group, the tumor volume in the cisplatin + GLP-1 low-dose group was significantly reduced from D3 to D12, and the tumor volume in the cisplatin + GLP-1 high-dose group was significantly reduced from D6 to D18.

Table 30: Effect of Ganoderma lucidum polysaccharide GLP-1 combined with chemotherapy on tumor volume in 4T1 breast tumor-bearing mice ($\bar{x} \pm s$)

| Group | Tumor volume ($mm^3$) | | | | | | |
|---|---|---|---|---|---|---|---|
| | D0 | D3 | D6 | D9 | D12 | D15 | D18 |
| Model control group | 201.4±43.3 | 891.5±238.7 | 1625.9±259.1 | 2127.6±254 | 3013.6±684.3 | 3607.4±491.8 | 4450.8±464 |
| Cisplatin | 190.5±34.8 | **601.9±146.6*** | **1120.5±346.5*** | **1216.8±402.8*** | **1417.6±392.2*** | **1506.8±324.2*** | **1638.5±208*** |
| Cisplatin + GLP-1 low-dose group | 187±35.4 | **473.8±127.4*#** | **708.7±257.3*#** | **796.8±311.2*#** | **930.2±383*#** | **1249.9±304.2*** | **1363.1±354.7*** |
| Cisplatin + GLP-1 high-dose group | 200.4±42.8 | **558±169.3*** | **675.1±193.4*#** | **634.5±230.9*#** | **718.1+236.8*#** | **985.5±334.2*#** | **911.1±305.3*#** |

Note: Compared to the model control group, $^*P < 0.05$; Compared to the cisplatin group, $^{\#}P < 0.05$.

**Effect of Ganoderma Lucidum Polysaccharide GLP-1 Combined with Chemotherapy on Relative Tumor Growth Inhibition Rate in 4T1 Breast Tumor-Bearing Mice**

As shown in Table 31, compared to the model control group, the cisplatin group showed a relative tumor growth inhibition rate of over 40% from D9, peaking at 62.8% on D18. The cisplatin + GLP-4 low-dose group maintained a relative tumor growth inhibition rate of over 40% from D3 to D18, and the cisplatin + GLP-4 high-dose group maintained a relative tumor growth inhibition rate of over 55% from D6 to D18, peaking at 80.0% on D18.

Compared to the cisplatin group, the relative tumor growth inhibition rate for the cisplatin + GLP-1 low-dose group ranged from 23% to 35% ($P < 0.05$) from D3 to D12, and the relative tumor growth inhibition rate for the cisplatin + GLP-1 high-dose group ranged from 37% to 53% ($P < 0.05$) from D6 to D18.

Table 31: Effect of Ganoderma lucidum polysaccharide GLP-1 combined with chemotherapy on relative tumor growth inhibition rate in 4T1 breast tumor-bearing mice (vs. the model control group)

| Group | Relative tumor growth inhibition rate (compared to model control group) (%) | | | | | |
|---|---|---|---|---|---|---|
| | D3 | D6 | D9 | D12 | D15 | D18 |
| Model control group | - | - | - | - | - | - |
| Cisplatin | **29.1*** | **28.1*** | **40.5*** | **53.1*** | **58.5*** | **62.8*** |
| Cisplatin + GLP-1 low-dose group | **45.6*** | **53.5*** | **60.6*** | **68.1*** | **64.4*** | **67.6*** |
| Cisplatin + GLP-1 high-dose group | **39.8*** | **59.6*** | **71.8*** | **78.0*** | **74.1*** | **80.0*** |

Note: Compared to the model control group, *$P < 0.05$.

Table 32: Effect of Ganoderma lucidum polysaccharide GLP-1 combined with chemotherapy on relative tumor growth inhibition rate in 4T1 breast tumor-bearing mice (vs. the cisplatin group)

| Group | Relative tumor growth inhibition rate (compared to cisplatin group) (%) | | | | | |
|---|---|---|---|---|---|---|
| | D3 | D6 | D9 | D12 | D15 | D18 |
| Cisplatin | - | - | - | - | - | - |
| Cisplatin + GLP-1 low-dose group | **23.3#** | **35.4#** | **33.8#** | **31.9#** | 14.3 | 13.1 |
| Cisplatin + GLP-1 high-dose group | 15.1 | **43.8#** | **52.5#** | **53.1#** | **37.7#** | **46.4#** |

Note: Compared to the cisplatin group, #$P < 0.05$.

Effect of Ganoderma Lucidum Polysaccharide GLP-1 Combined with Chemotherapy on Organ Coefficients and Tumor Growth Inhibition Rates in 4T1 Breast Tumor-Bearing Mice

As shown in Table 33, compared to the model control group, the tumor index, spleen index, and thymus index was significantly reduced in the cisplatin group and the cisplatin + GLP-1 group. Compared to the cisplatin group, the tumor index was significantly reduced in the cisplatin + GLP-1 high-dose group, and there was no significant difference in the spleen index and thymus index in the cisplatin + GLP-1 group. Compared to the normal group, the thymus index was significantly reduced in the model control group, the cisplatin + GLP-1 group, and the spleen index was significantly increased in the model control group.

Compared to the model control group, the tumor growth inhibition rate was 71.6% in the cisplatin group, and 74.1% and 82.9% in the cisplatin + GLP-1 low-dose and high-dose groups, respectively. Compared to the cisplatin group, the tumor growth inhibition rate was 8.6% and 39.7% in the cisplatin + GLP-1 low-dose and high-dose groups, respectively.

Table 33: Effect of Ganoderma lucidum polysaccharide GLP-1 combined with chemotherapy on organ indices and tumor growth inhibition rates in 4T1 breast tumor-bearing mice

| Group | Tumor index (%) | Spleen index (mg/g) | Thymus index (mg/g) | Tumor growth inhibition rate (%) vs. the model control group | Tumor growth inhibition rate (%) vs. the cisplatin group |
|---|---|---|---|---|---|
| Model control group | 14.3±2.4 | 45.8±4+ | 1.4±0.5+ | - | |
| Cisplatin | **6.2±1.5*** | **3.8±0.9*** | **0.4±0.1+*** | 71.6 | |
| Cisplatin + GLP-1 low-dose group | **5.6±1.5*** | **4.1±1.3*** | **0.3±0.1+*** | 74.1 | 8.6 |

(continued)

| Group | Tumor index (%) | Spleen index (mg/g) | Thymus index (mg/g) | Tumor growth inhibition rate (%) vs. the model control group | Tumor growth inhibition rate (%) vs. the cisplatin group |
|---|---|---|---|---|---|
| Cisplatin + GLP-1 high-dose group | **3.9±1.4**$^{*\#}$ | **2.8±1.3**$^{*}$ | **0.2±0.1**$^{+*}$ | 82.9 | 39.7 |
| Normal group | **-** | **3.8±0.3**$^{*}$ | **2.6±0.5**$^{*}$ | - | |

Note: Compared to the normal control group, $^{+}P \leq 0.05$; compared to the model control group, $^{*}P < 0.05$; compared to the cisplatin group, $^{\#}P < 0.05$.

Figures 39, 40, 41, and 42 show images of the tumor-bearing mice, corresponding to the groups as follows: Figure 39: model control group, Figure 40: cisplatin group, Figure 41: cisplatin + GLP-1 low-dose group, Figure 42: cisplatin + GLP-1 high-dose group.

Figures 43, 44, 45, and 46 show images of the tumors in the tumor-bearing mice, corresponding to the groups as follows: Figure 43: model control group, Figure 44: cisplatin group, Figure 45: cisplatin + GLP-3 low-dose group, Figure 46: cisplatin + GLP-3 high-dose group.

Conclusion

Ganoderma Lucidum Polysaccharide GLP-1 combined with cisplatin significantly inhibits tumor growth in 4T1 breast tumor-bearing mice and exhibits a significant synergistic effect.

**Experimental Study on the Cytotoxic Effects of Ganoderma Lucidum Polysaccharide GLP-1 Combined with Cisplatin on LLC Cells and Study Data**

**Experimental Objective:**

To investigate the cytotoxic effects of Ganoderma lucidum polysaccharide GLP-1 on LLC cells, providing experimental basis for its clinical research.

**Experimental Materials**

**Test Sample**

Ganoderma lucidum polysaccharide GLP-1, batch number: H20220319A, provided by Shenzhen Aolimei Oncology Medical Technology Co., Ltd.

**Positive Control**

Cisplatin, batch number: E2128081, product of Shanghai Aladdin Biochemical Technology Co., Ltd.

**Cell Line**

LLC cell line, purchased from the Shanghai Institutes for Biological Sciences, Chinese Academy of Sciences.

**Main Reagents**

PBS buffer, prepared by Shenzhen Aolimei Oncology Medical Technology Co., Ltd.; 0.25% Trypsin, product of Gibco; MTT, product of Aladdin; DMSO, product of Guangdong Guanghua Sci-Tech Co., Ltd.; DMEM Culture Medium, product of Gibco.

**Main Instruments**

20 μL, 200 μL, and 1 mL pipettes, products of Mettler-Toledo Ltd.; Luna-II Cell Counter, product of Nanjing Hengqiao Instrument Co., Ltd.; ST-360 Microplate Reader, Shanghai Kehua Bio-Engineering Co., Ltd.

**Experimental Methods:**

LLC lung cancer cells were seeded in a 96-well cell culture plate at 3,000 cells per well and incubated at 37°C in a 5% $CO_2$ incubator for 24 h. After this, 2 mg/mL of GLP-1 solution was added to the test sample wells, and 2.5 μg/mL cisplatin solution was added to the positive control wells. The cultures were then incubated for another 48 h before measuring the absorbance at 570 nm.

**Experimental Results**

**Table 34**

| Group | Inhibition rate |
|---|---|
| Model control group | - |
| Cisplatin group | 35.2% |
| Cisplatin + GLP-1 group | 55.7% |

As shown in Figure 47, the distribution of LLC cells after 24 h of culture.
As shown in Figure 48, the distribution of LLC cells after 48 h of culture.
As shown in Figure 49, the distribution of LLC cells at 24 h after cisplatin treatment.
As shown in Figure 50, the distribution of LLC cells at 48 h after cisplatin treatment.
As shown in Figure 51, the distribution of LLC cells at 24 h after treatment with cisplatin + GLP-1.
As shown in Figure 52, the distribution of LLC cells at 48 h after treatment with cisplatin + GLP-1.

Conclusion

Ganoderma lucidum polysaccharide GLP-1 combined with cisplatin can inhibit the growth of LLC cells and enhances the antitumor efficacy of cisplatin.
The foregoing description concerns merely exemplary embodiments of the present invention and is not intended to limit the invention. Any modifications, equivalent substitutions, and improvements made within the spirit and principles of the invention should be included within the scope of the invention's protection.

## Claims

1. A type of Ganoderma lucidum polysaccharide GLP-1, **characterized in that** the molecular structural formula of the Ganoderma lucidum polysaccharide GLP-1 is

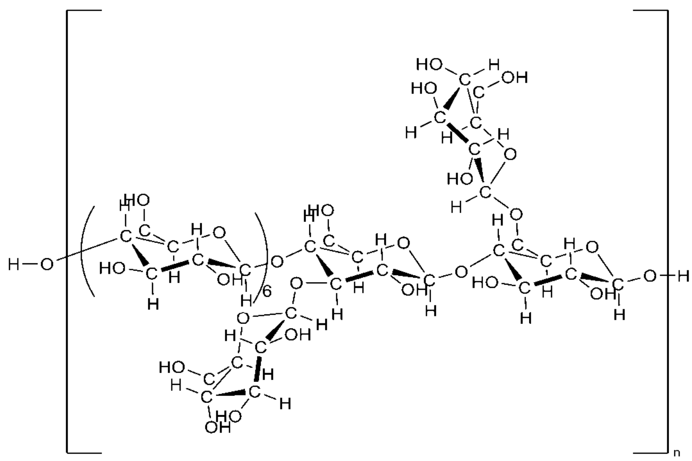

;

the molecular formula is: $(C_{60}H_{100}O_{50})n$; where n = 17-25.

2. The Ganoderma lucidum polysaccharide GLP-1 according to claim 1, wherein n is selected from 17, 18, 19, 20, 21, 22, 23, 24, or 25.

3. A method for extracting the Ganoderma lucidum polysaccharide GLP-1, comprising the steps of:

S1. dusting and drying Ganoderma lucidum, then crushing Ganoderma lucidum to make Ganoderma lucidum powder;
S2. placing the crushed Ganoderma lucidum powder in a sealed container mixed with water, heating under high temperature and pressure to fully dissolve the Ganoderma lucidum powder with the water into a medicinal juice solution;
S3. using membrane concentration technology to separate the medicinal juice solution to obtain a concentrated solution with an active ingredient and not fully dissolved medicinal residue; and
S4. mixing the concentrated solution containing the active ingredient with pure water to a water solution with a preset concentration, followed by multiple column chromatography separations to obtain the Ganoderma lucidum polysaccharide GLP-1 with the active ingredient.

4. The method for extracting Ganoderma lucidum polysaccharide GLP-1 according to claim 3, **characterized in that** in step S2, the mixture of the Ganoderma lucidum powder and the water in the sealed container is fully stirred and heated at a high temperature to 105-200°C, with boiling time lasting for 2-6 h, during which the internal pressure in the sealed container gradually increases as the heating temperature rises, forming a high-temperature and high-pressure environment within the sealed container.

5. The method for extracting Ganoderma lucidum polysaccharide GLP-1 according to claim 3, **characterized in that** in step S2, the mixture of the Ganoderma lucidum powder and the water in the sealed container is heated at a high temperature to 105-170°C, with boiling time lasting for 3-6 h, during which the internal pressure in the sealed container gradually increases as the heating temperature rises, forming the high-temperature and high-pressure environment within the sealed container.

6. The method for extracting the Ganoderma lucidum polysaccharide GLP-1 according to claim 4 or 5, **characterized in that** in step S4, the concentrated liquid containing the active ingredient is mixed with the pure water at a concentration ratio of 1:2 to 1:5.

7. The method for extracting the Ganoderma lucidum polysaccharide GLP-1 according to claim 6, **characterized in that** in step S3, Ganoderma lucidum residue is removed from the extracted water solution containing the active ingredient by using the membrane concentration technology, obtaining a concentrated liquid or paste containing the active ingredient.

8. The method for extracting the Ganoderma lucidum polysaccharide GLP-1 according to claim 7, **characterized in that** in step S1, the Ganoderma lucidum is rinsed with clean water to remove surface dust and dried at 105°C, and the dried Ganoderma lucidum is crushed, with the crushed Ganoderma lucidum powder being larger than 60 mesh.

9. The method for extracting the Ganoderma lucidum polysaccharide GLP-1 according to claim 5, **characterized in that** in step S2, the mixed liquid in the sealed container is heated at a high temperature to 105°C, 110°C, 115°C, 120°C,125°C, 130°C, 135°C, 140°C, 145°C, 150°C,155°C, 160°C, 165°C, 170°C,175°C, 180°C, 185°C, 190°C, 195°C, or 200°C, with boiling times of 3 h, 3.5 h, 4 h, 4.5 h, 5 h, 5.5 h, or 6 h, during which the internal pressure in the sealed container gradually increases as the heating temperature rises, forming the high-temperature and high-pressure environment within the sealed container.

10. The application of the Ganoderma lucidum polysaccharide GLP-1 and cisplatin in the preparation of anti-lung cancer, liver cancer, and breast cancer drug products according to claim 1 or 2, **characterized by** the combined use of the Ganoderma lucidum polysaccharide GLP-1 and the chemotherapy drug cisplatin, which can alleviate the toxic side effects caused by chemotherapy drugs on the human body, control and reduce tumor masses, and reduce and eliminate cancer cells.

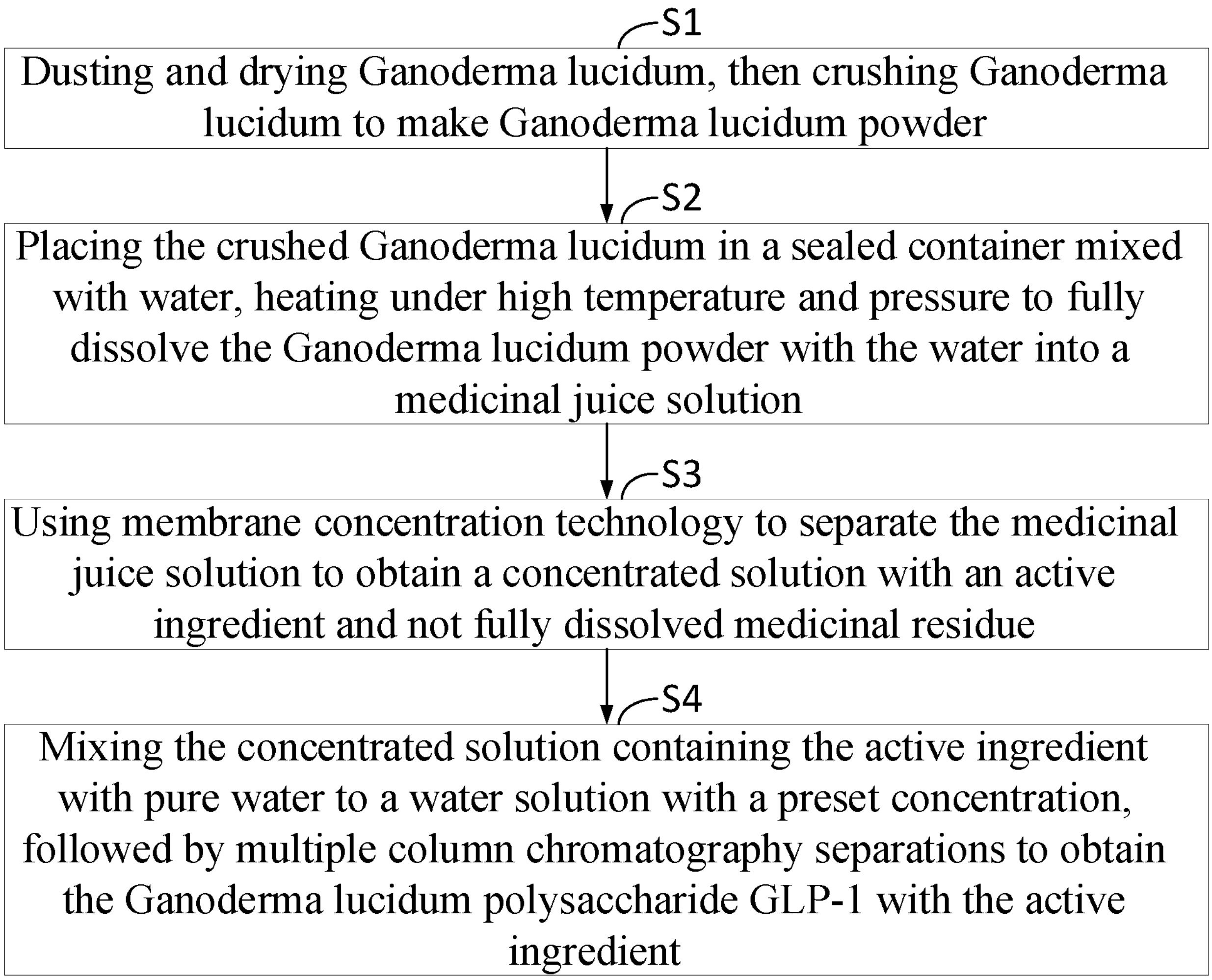
S1
Dusting and drying Ganoderma lucidum, then crushing Ganoderma lucidum to make Ganoderma lucidum powder
S2
Placing the crushed Ganoderma lucidum in a sealed container mixed with water, heating under high temperature and pressure to fully dissolve the Ganoderma lucidum powder with the water into a medicinal juice solution
S3
Using membrane concentration technology to separate the medicinal juice solution to obtain a concentrated solution with an active ingredient and not fully dissolved medicinal residue
S4
Mixing the concentrated solution containing the active ingredient with pure water to a water solution with a preset concentration, followed by multiple column chromatography separations to obtain the Ganoderma lucidum polysaccharide GLP-1 with the active ingredient

Fig. 1

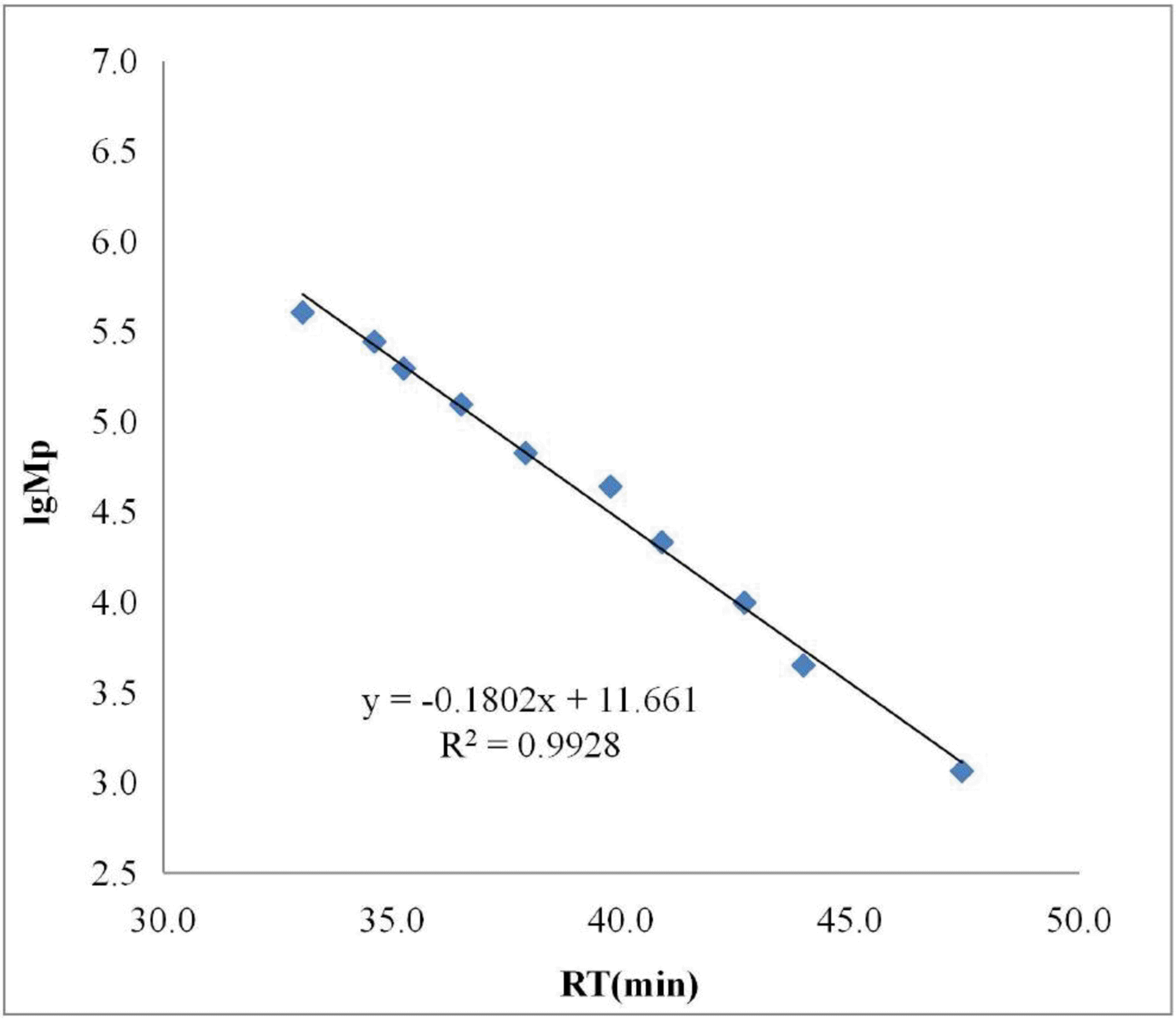
7.0
6.5
6.0
5.5
5.0
4.5
4.0
3.5
3.0
2.5
lgMp
y = -0.1802x + 11.661
R² = 0.9928
30.0
35.0
40.0
45.0
50.0
RT(min)

Fig. 2

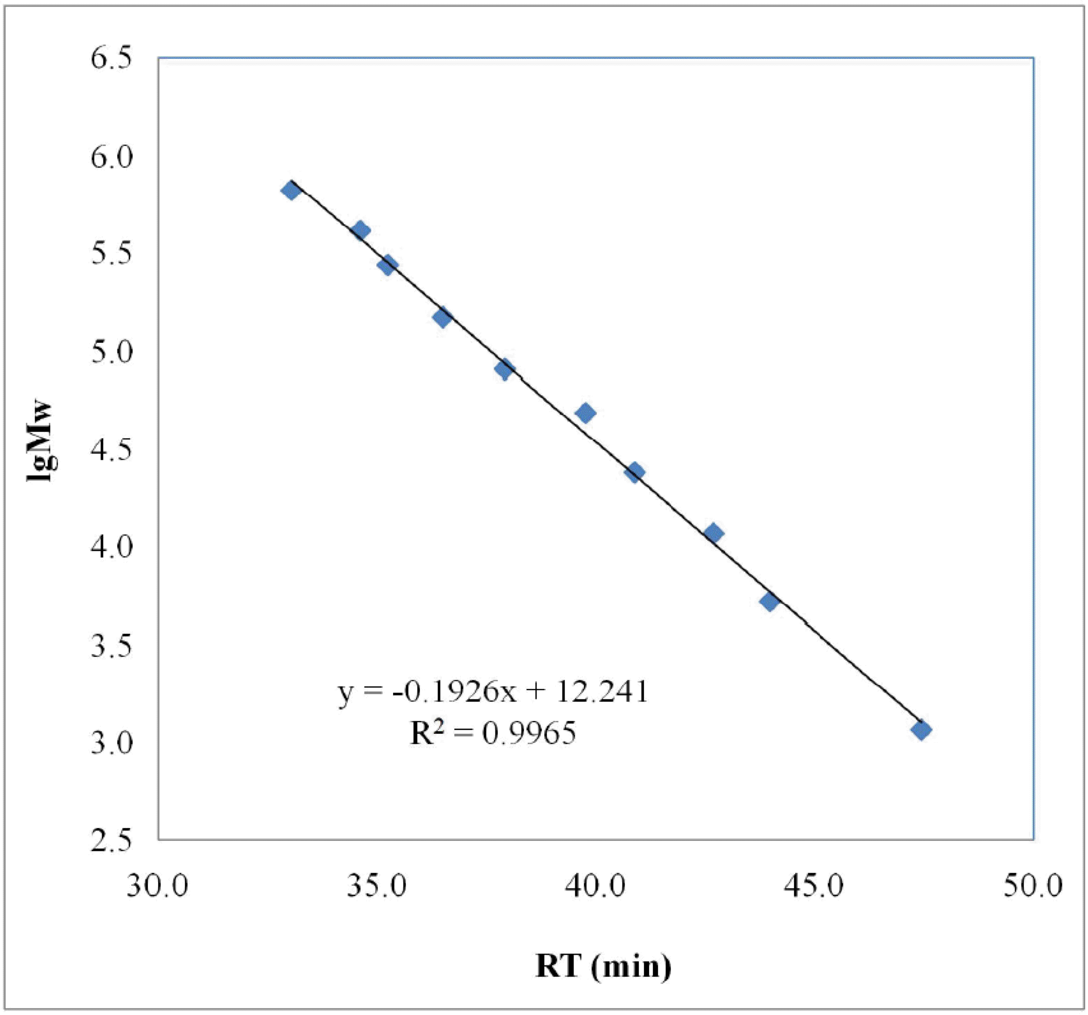
6.5
6.0
5.5
5.0
4.5
4.0
3.5
3.0
2.5
lgMw
y = -0.1926x + 12.241
R2 = 0.9965
30.0
35.0
40.0
45.0
50.0
RT (min)

Fig. 3

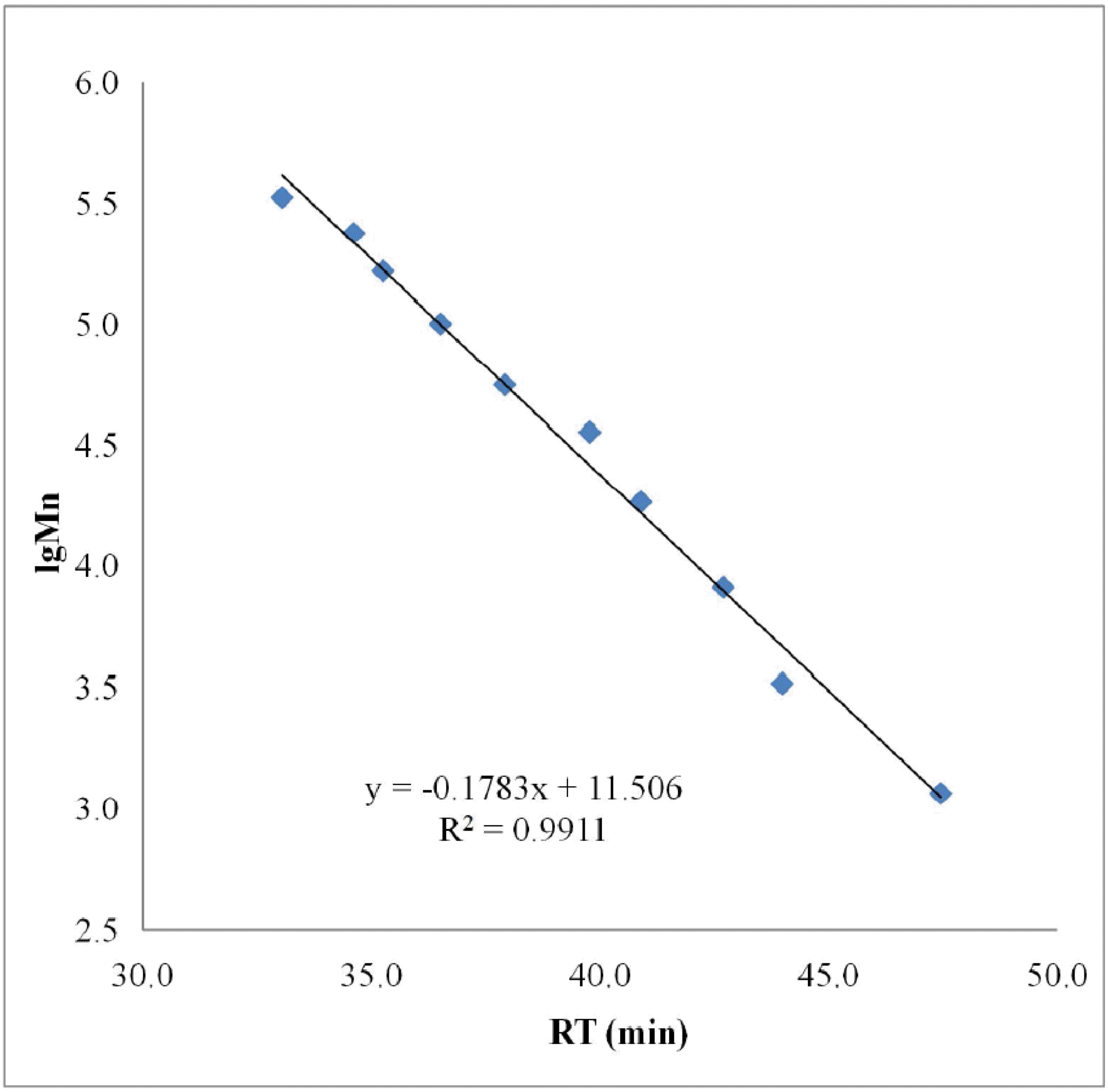
6.0
5.5
5.0
4.5
4.0
3.5
3.0
2.5
lgMn
y = -0.1783x + 11.506
R² = 0.9911
30.0
35.0
40.0
45.0
50.0
RT (min)

Fig. 4

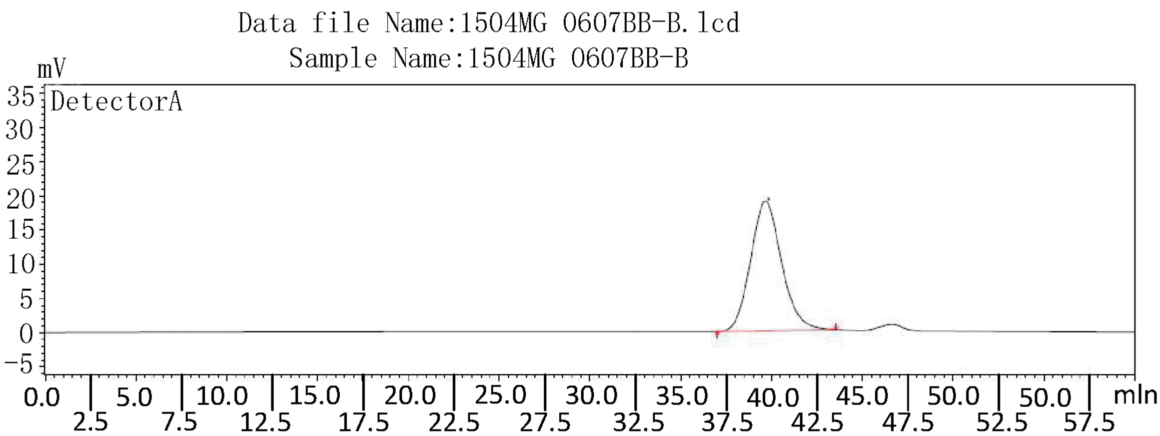
Data file Name:1504MG 0607BB-B.lcd
Sample Name:1504MG 0607BB-B
mV
DetectorA
35
30
25
20
15
10
5
0
-5
0.0
2.5
5.0
7.5
10.0
12.5
15.0
17.5
20.0
22.5
25.0
27.5
30.0
32.5
35.0
37.5
40.0
42.5
45.0
47.5
50.0
52.5
50.0
57.5
mln

Fig. 5

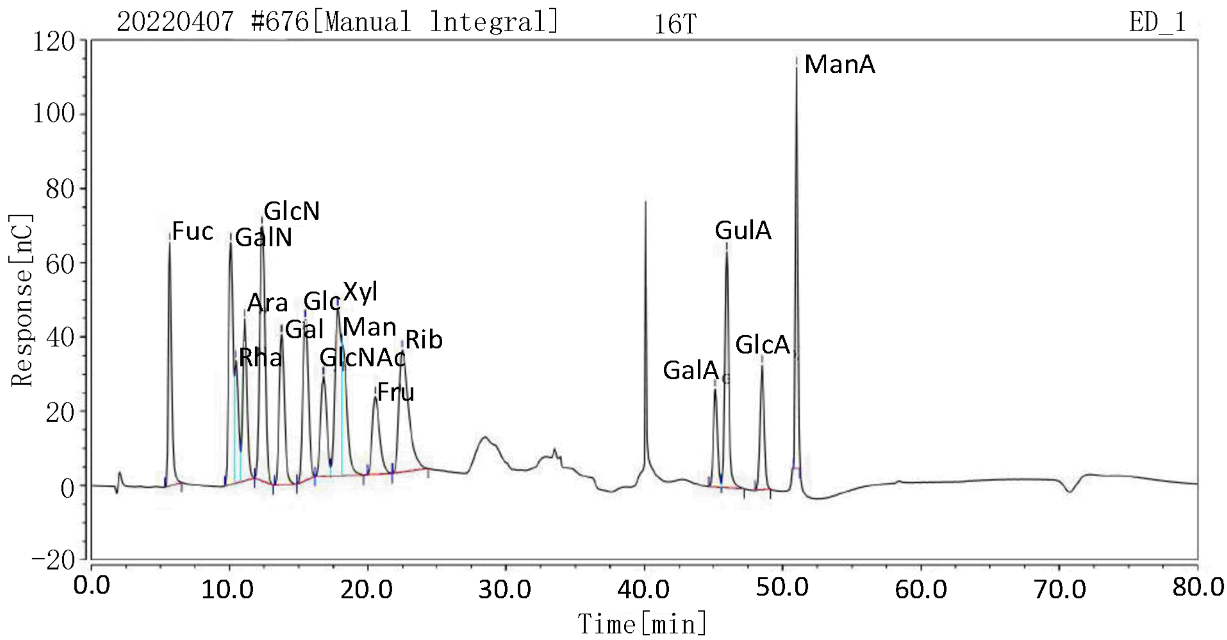
20220407 #676[Manual Integral]
16T
ED_1
Response[nC]
Time[min]
Fuc
GalN
GlcN
Rha
Ara
Gal
Glc
Xyl
GlcNAc
Man
Rib
Fru
GalA
GulA
GlcA
ManA

Fig. 6

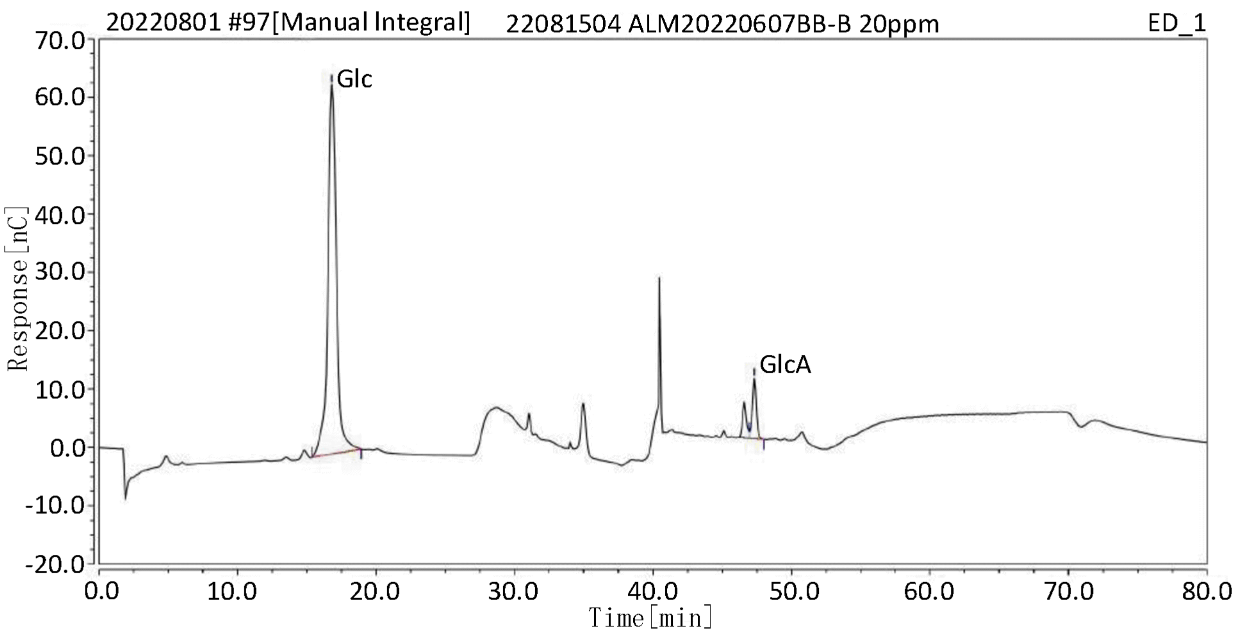
20220801 #97[Manual Integral]
22081504 ALM20220607BB-B 20ppm
ED_1
Response[nC]
Time[min]
Glc
GlcA

Fig. 7

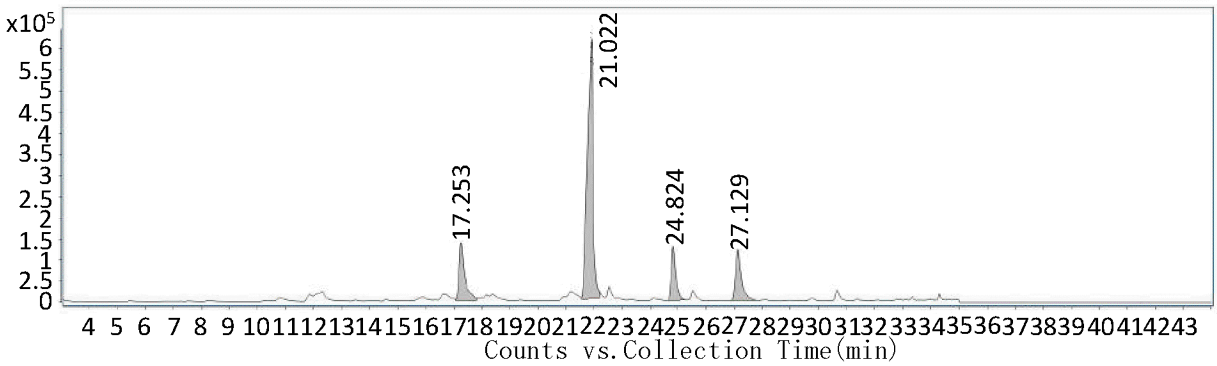
x10^5
6
5.5
5
4.5
4
3.5
3
2.5
2
1.5
1
2.5
0
17.253
21.022
24.824
27.129
4 5 6 7 8 9 10 11 12 13 14 15 16 17 18 19 20 21 22 23 24 25 26 27 28 29 30 31 32 33 34 35 36 37 38 39 40 41 42 43
Counts vs.Collection Time(min)

Fig. 8

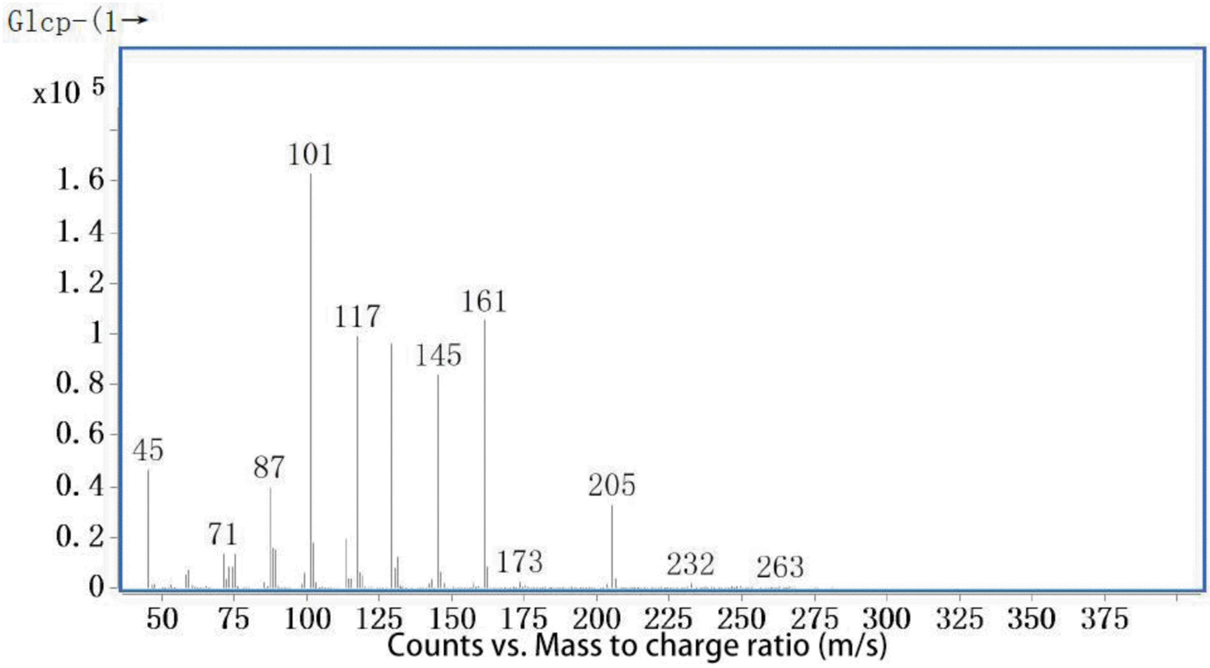
Glcp-(1→
x10 5
1.6
1.4
1.2
1
0.8
0.6
0.4
0.2
0
45
71
87
101
117
145
161
173
205
232
263
50 75 100 125 150 175 200 225 250 275 300 325 350 375
Counts vs. Mass to charge ratio (m/s)

Fig. 9

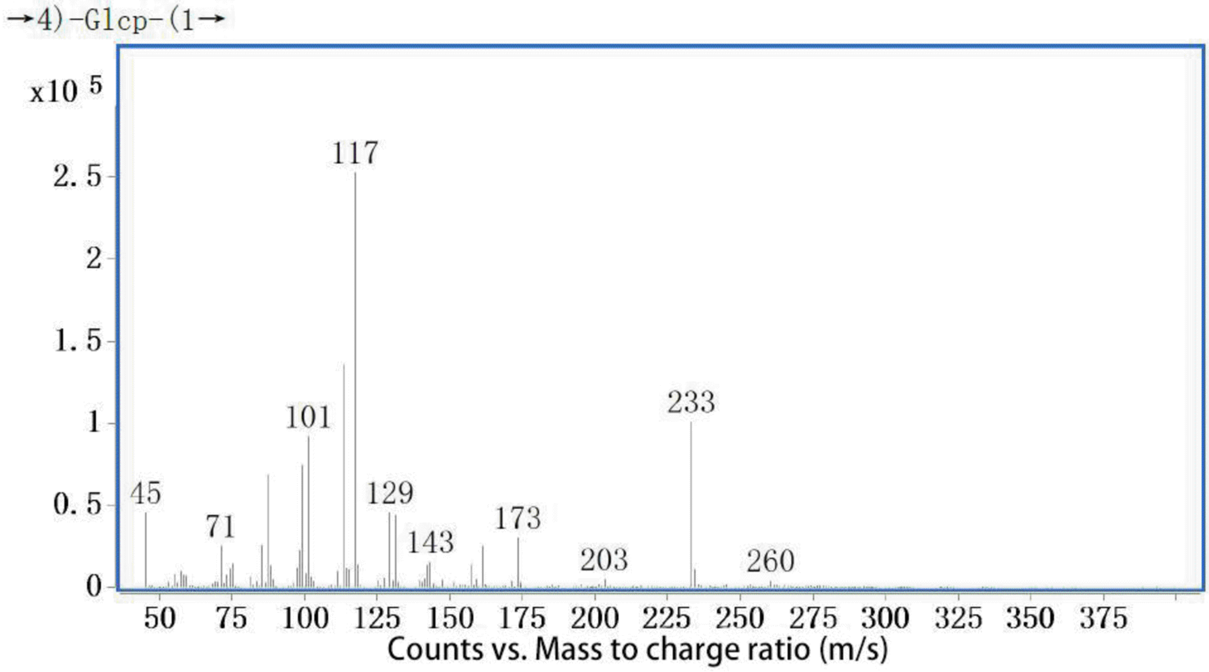
→4)-Glcp-(1→
x10 5
2. 5
2
1. 5
1
0. 5
0
117
101
233
45
129
71
173
143
203
260
50 75 100 125 150 175 200 225 250 275 300 325 350 375
Counts vs. Mass to charge ratio (m/s)

Fig. 10

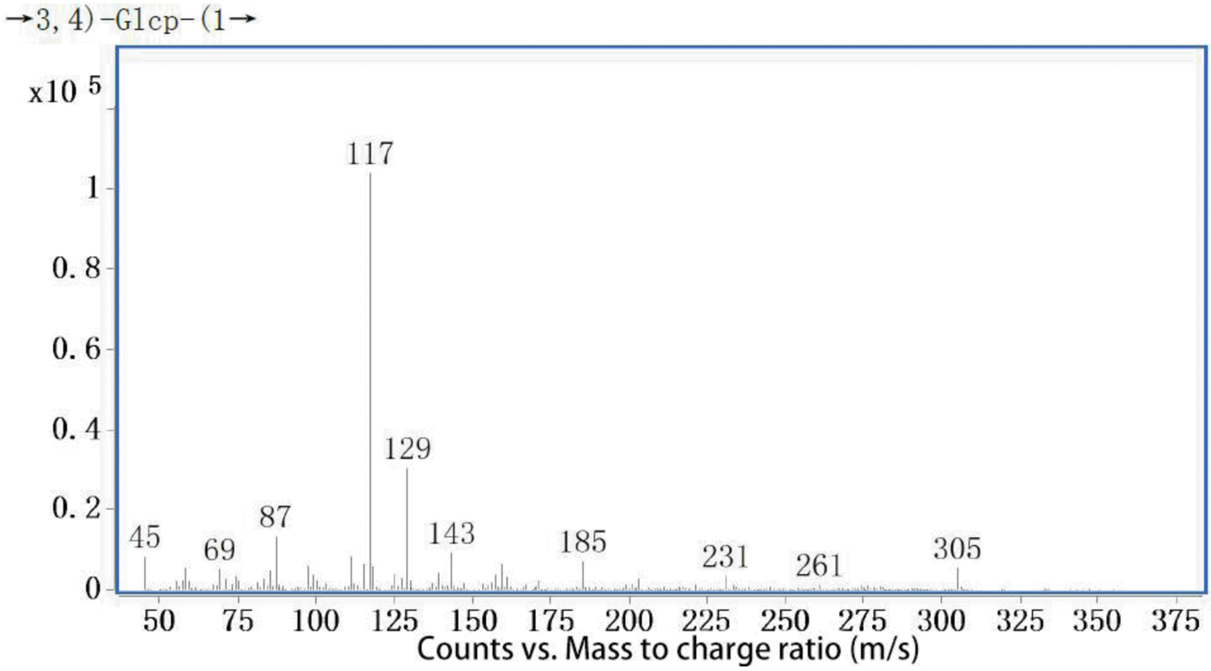
→3, 4)-Glcp-(1→
x10 5
1
0. 8
0. 6
0. 4
0. 2
0
117
129
87
143
45
69
185
231
261
305
50 75 100 125 150 175 200 225 250 275 300 325 350 375
Counts vs. Mass to charge ratio (m/s)

Fig. 11

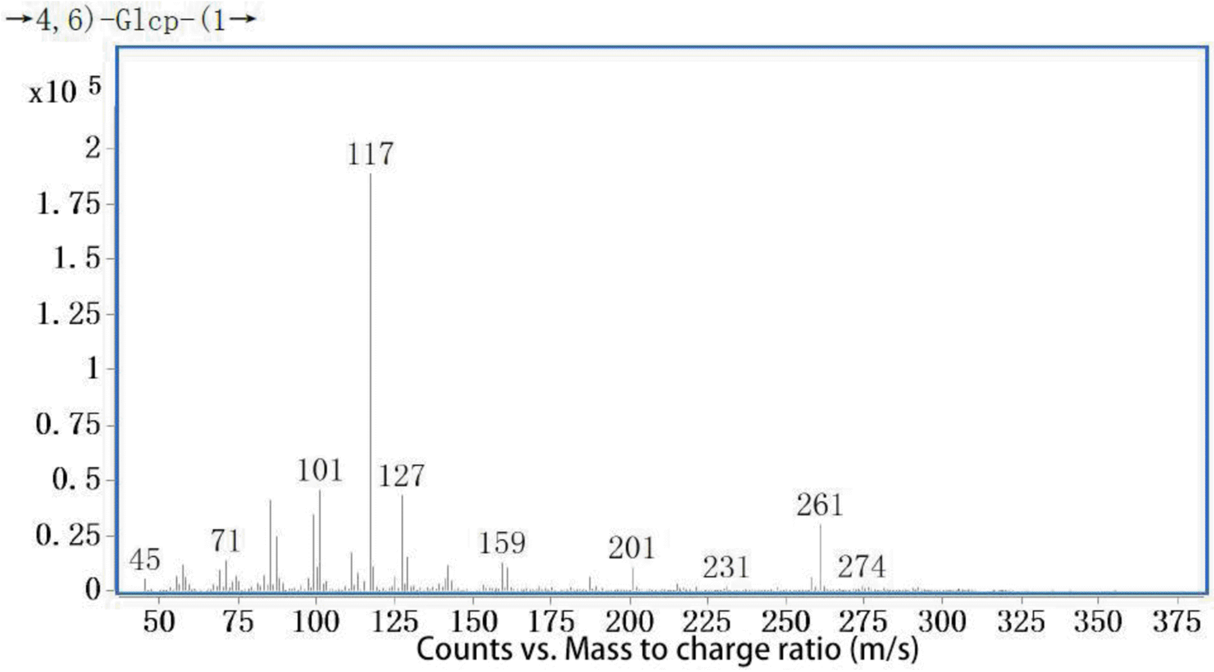
→4, 6)-Glcp-(1→
x10 5
2
1. 75
1. 5
1. 25
1
0. 75
0. 5
0. 25
0
117
101
127
261
45
71
159
201
231
274
50 75 100 125 150 175 200 225 250 275 300 325 350 375
Counts vs. Mass to charge ratio (m/s)

Fig. 12

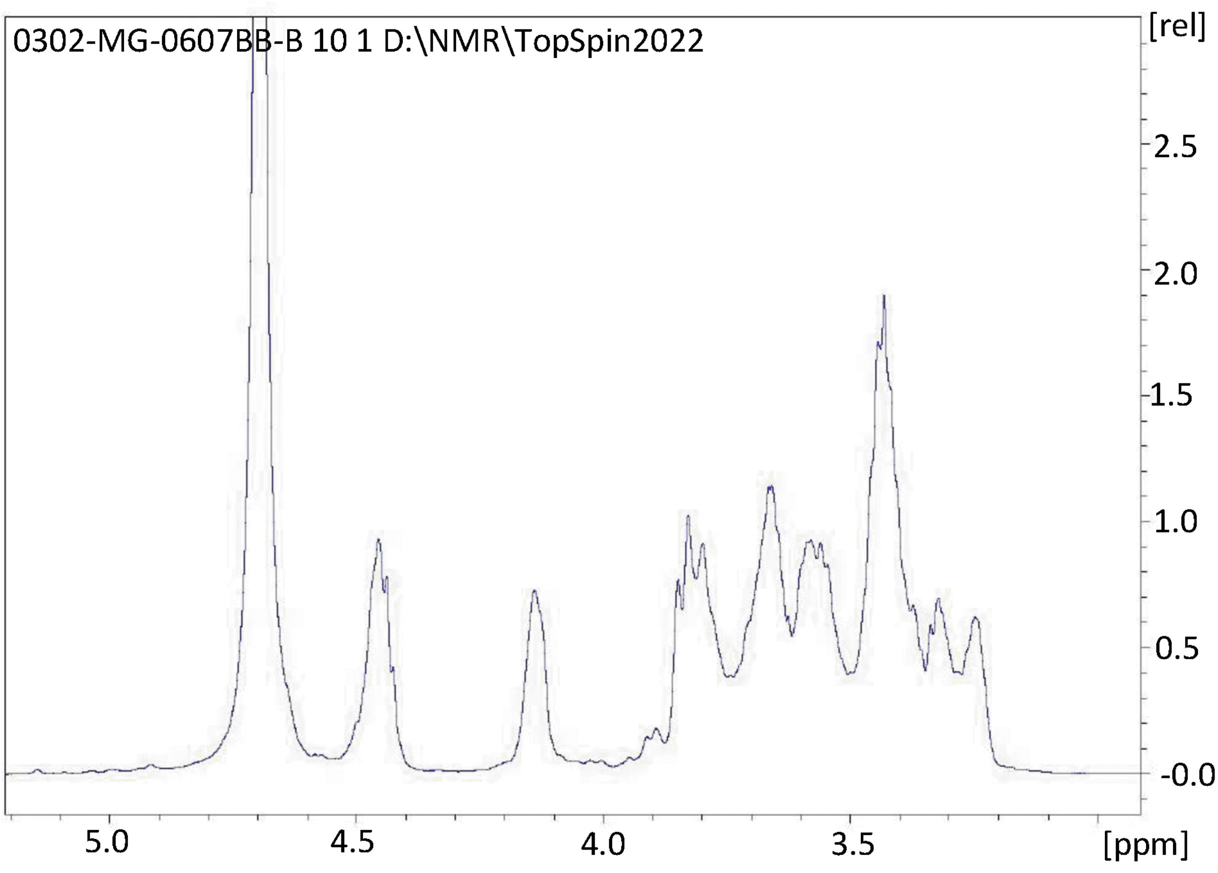
0302-MG-0607BB-B 10 1 D:\NMR\TopSpin2022
[rel]
2.5
2.0
1.5
1.0
0.5
-0.0
5.0
4.5
4.0
3.5
[ppm]

Fig. 13

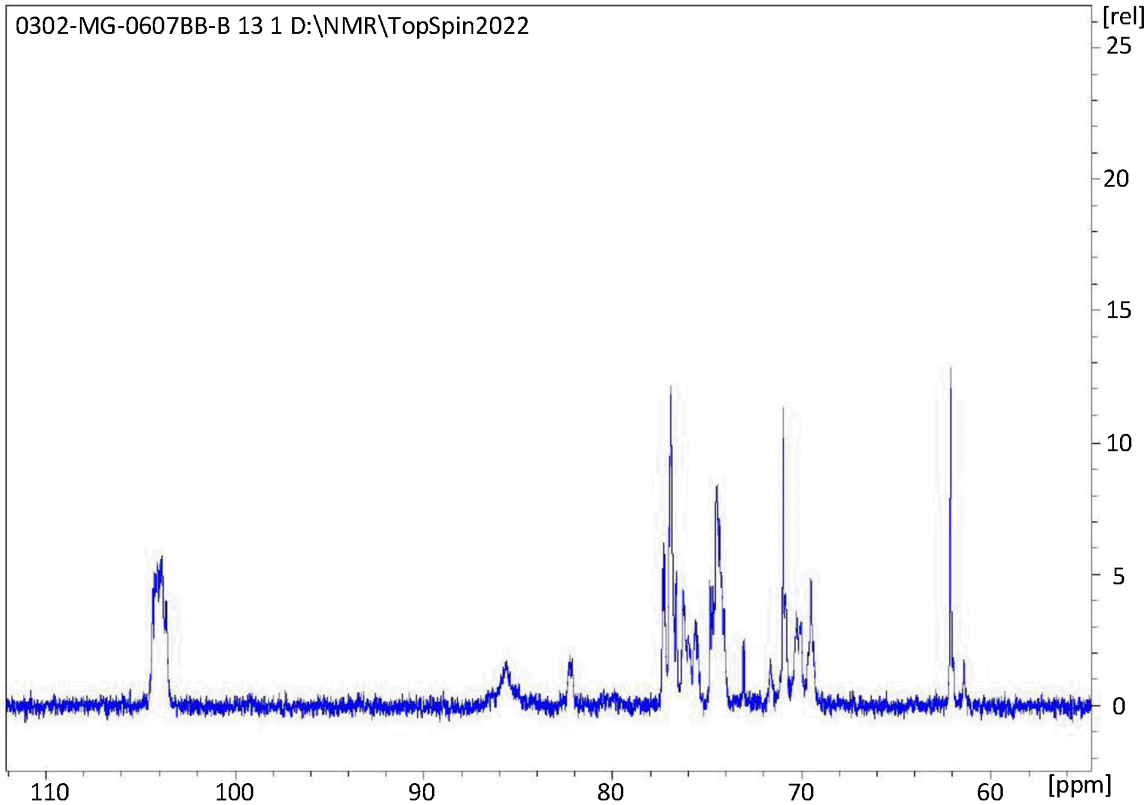
0302-MG-0607BB-B 13 1 D:\NMR\TopSpin2022
[rel]
25
20
15
10
5
0
110
100
90
80
70
60
[ppm]

Fig. 14

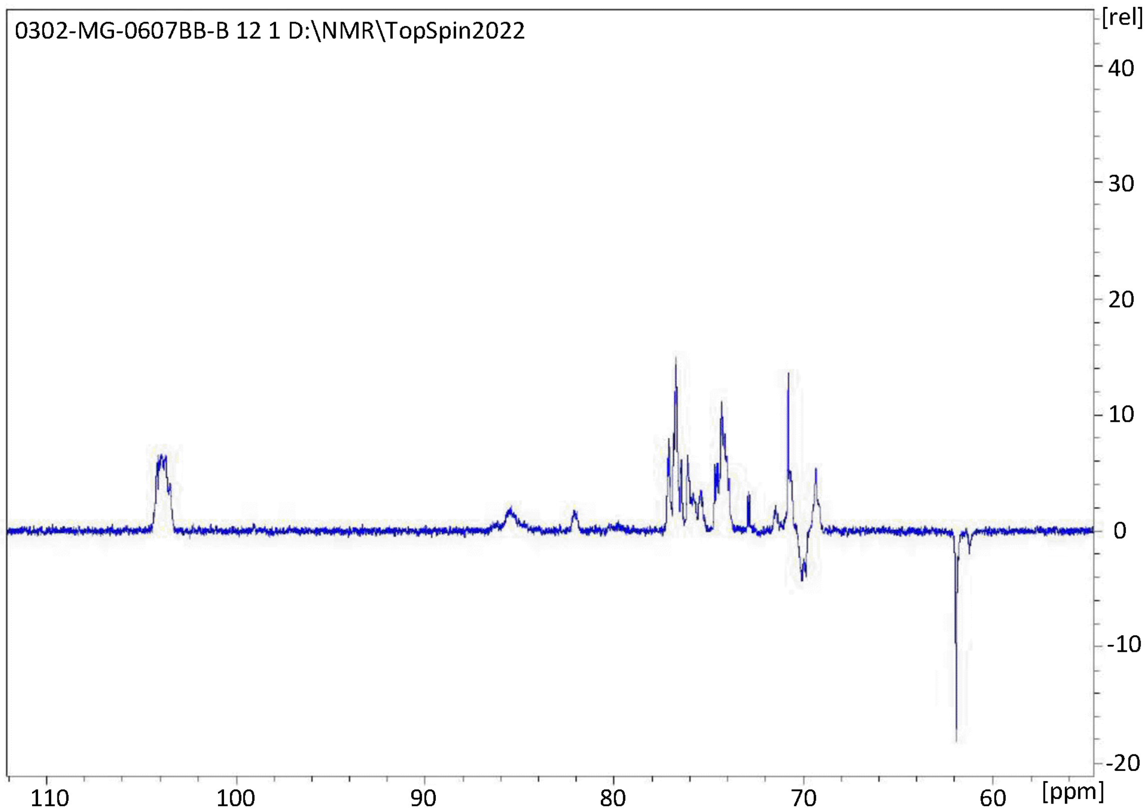
0302-MG-0607BB-B 12 1 D:\NMR\TopSpin2022
[rel]
40
30
20
10
0
-10
-20
110
100
90
80
70
60
[ppm]

Fig. 15

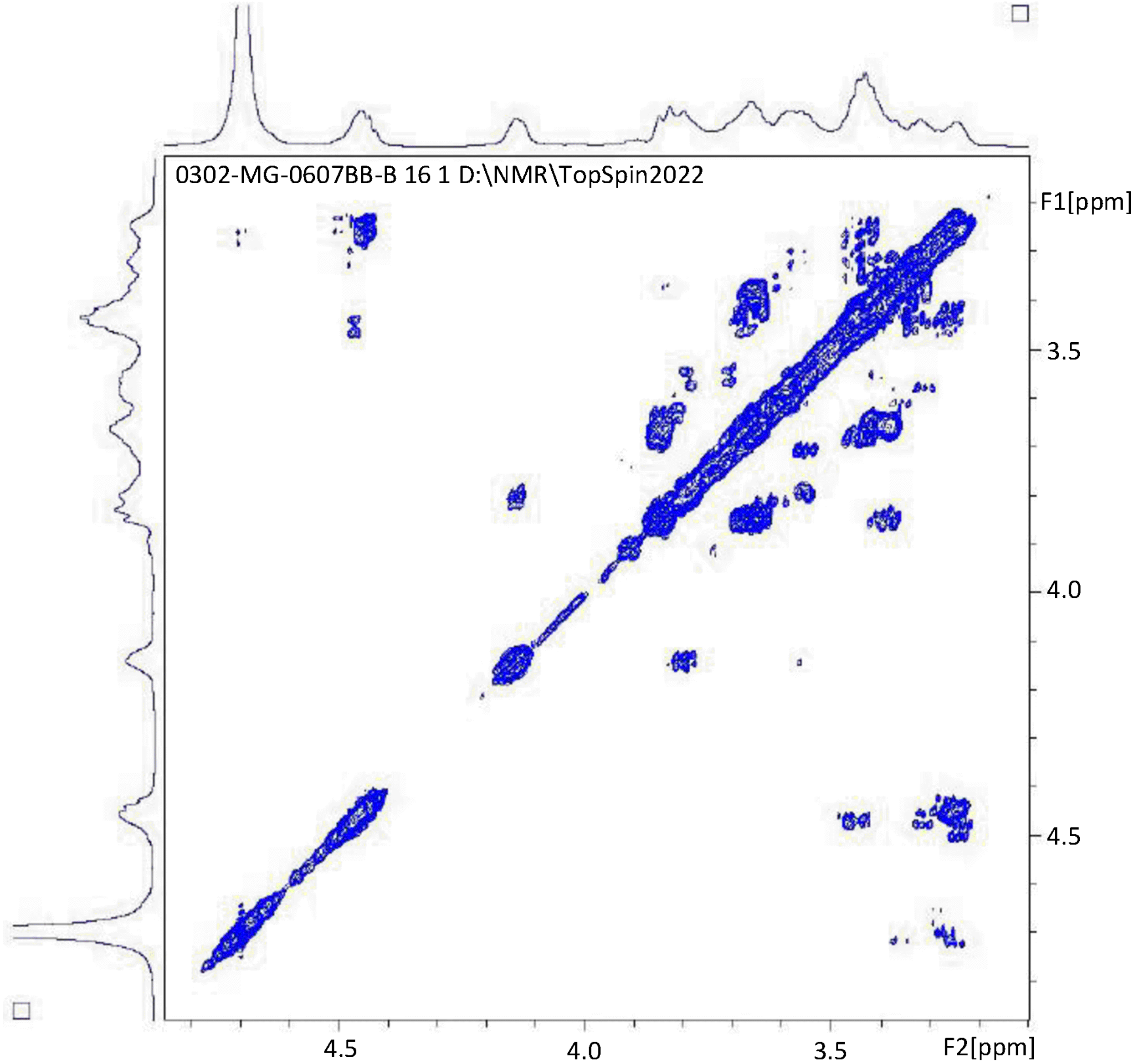
0302-MG-0607BB-B 16 1 D:\NMR\TopSpin2022
F1[ppm]
3.5
4.0
4.5
4.5
4.0
3.5
F2[ppm]

Fig. 16

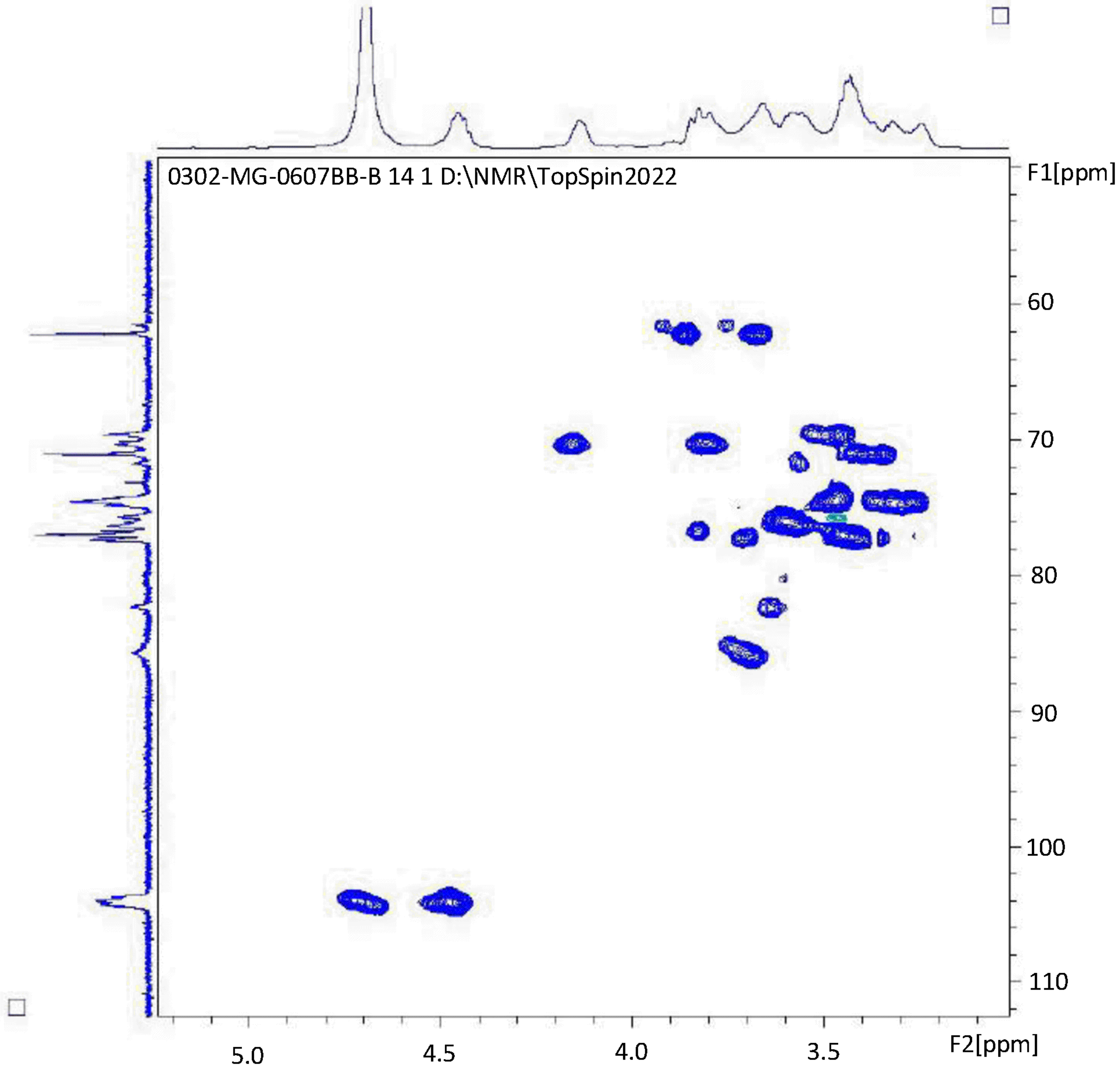
0302-MG-0607BB-B 14 1 D:\NMR\TopSpin2022
F1[ppm]
60
70
80
90
100
110
5.0
4.5
4.0
3.5
F2[ppm]

Fig. 17

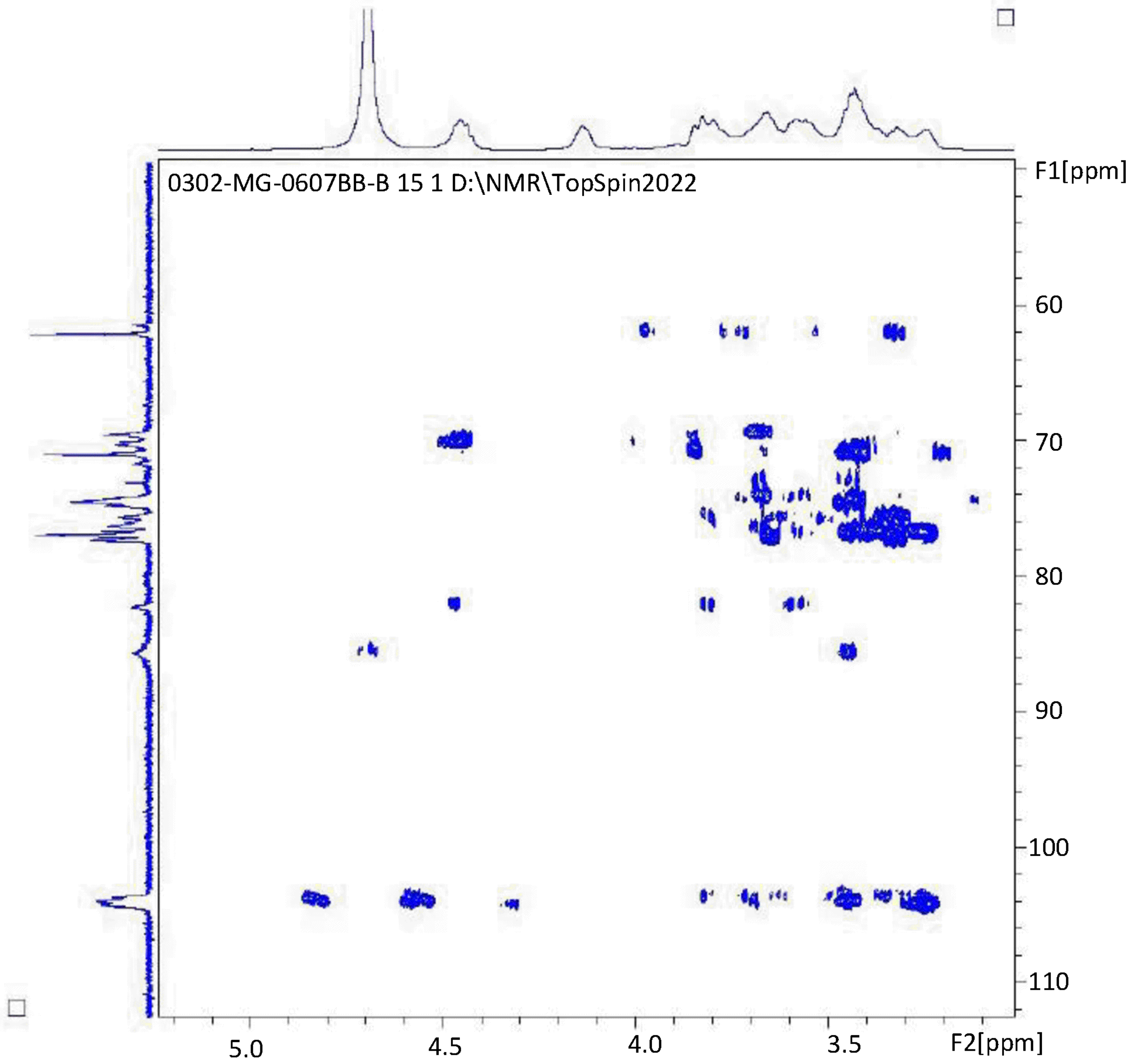
0302-MG-0607BB-B 15 1 D:\NMR\TopSpin2022
F1[ppm]
60
70
80
90
100
110
5.0
4.5
4.0
3.5
F2[ppm]

Fig. 18

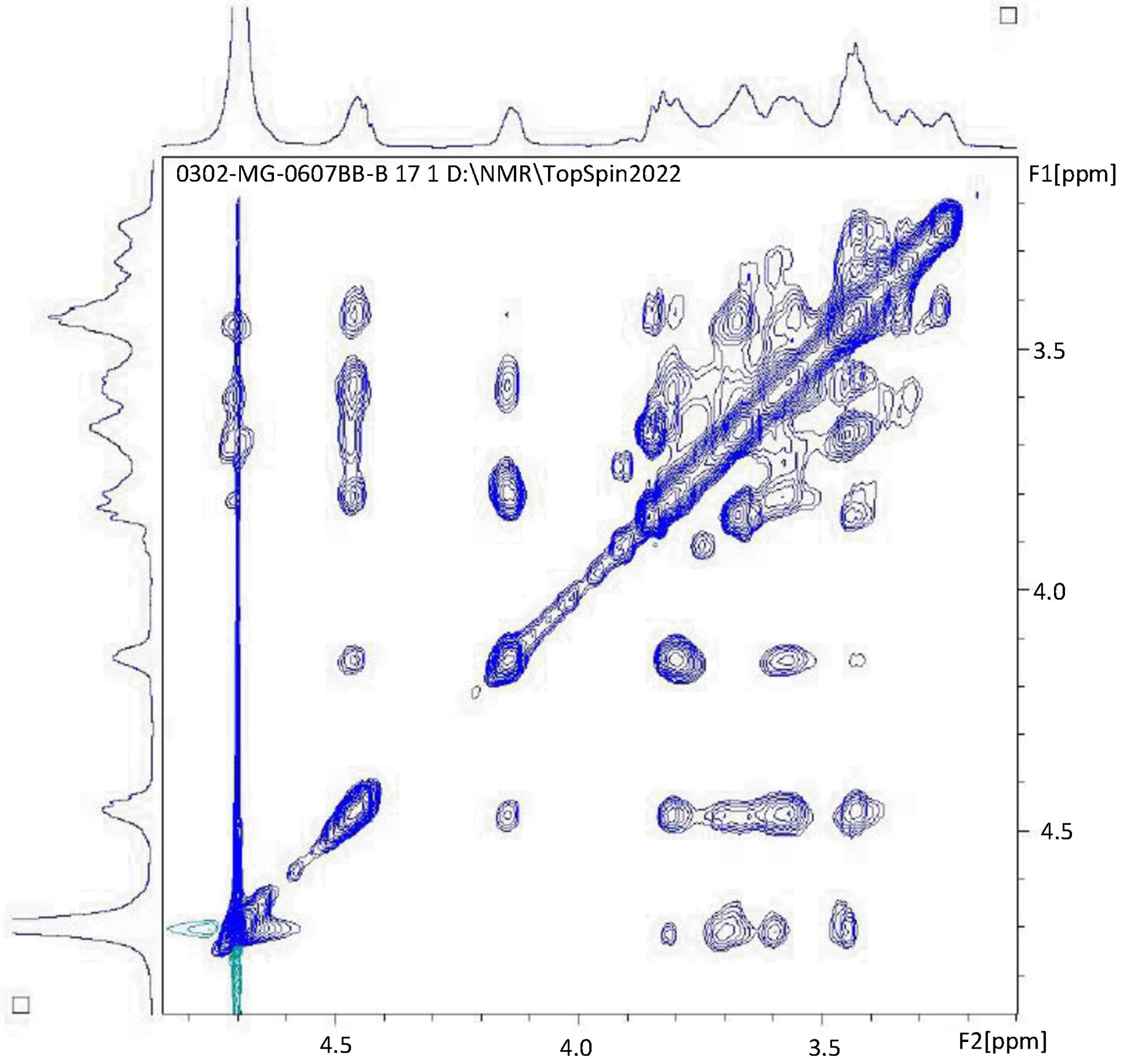
0302-MG-0607BB-B 17 1 D:\NMR\TopSpin2022
F1[ppm]
3.5
4.0
4.5
4.5
4.0
3.5
F2[ppm]

Fig. 19

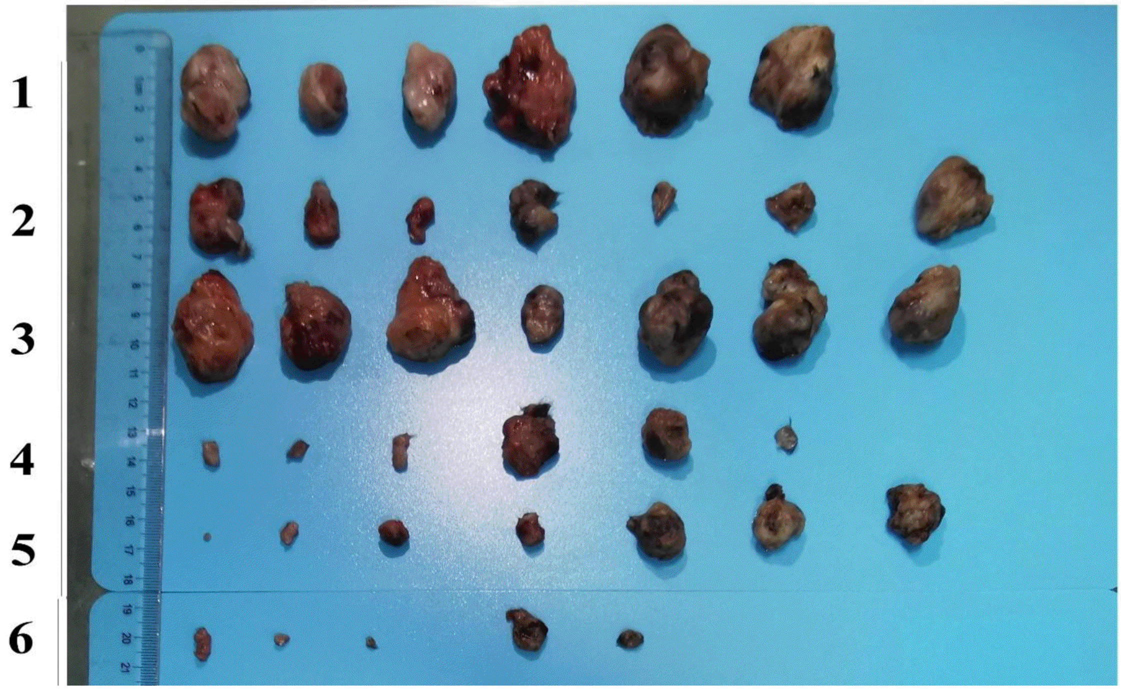


Fig. 20

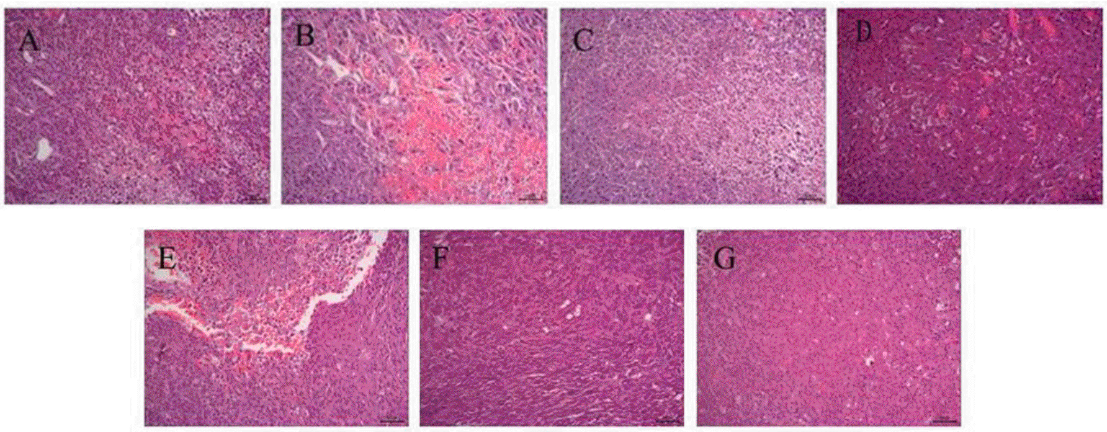


Fig. 21

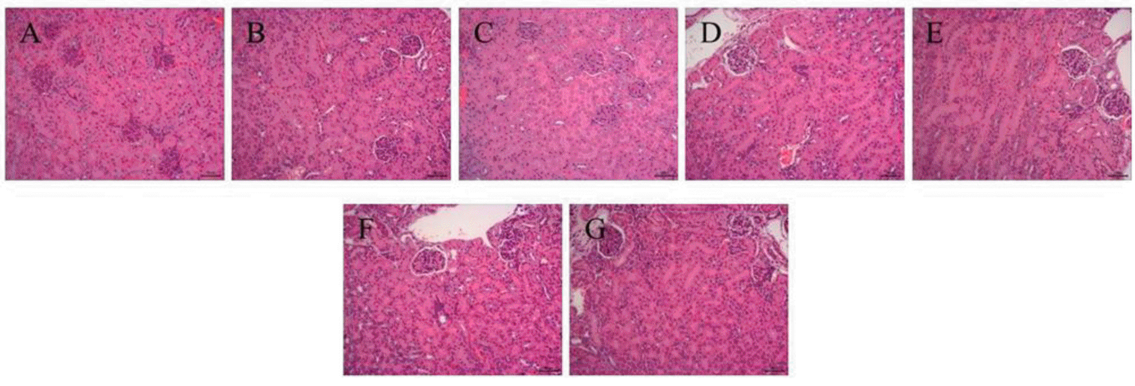


Fig. 22

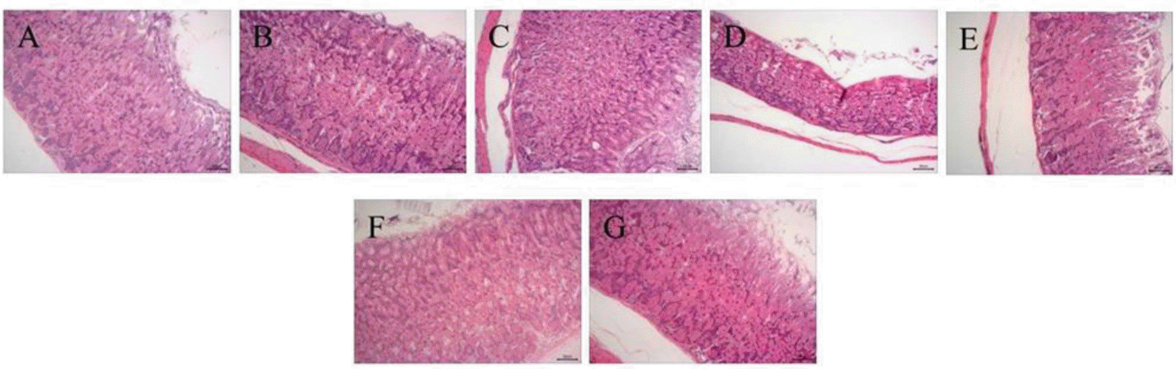


Fig. 23

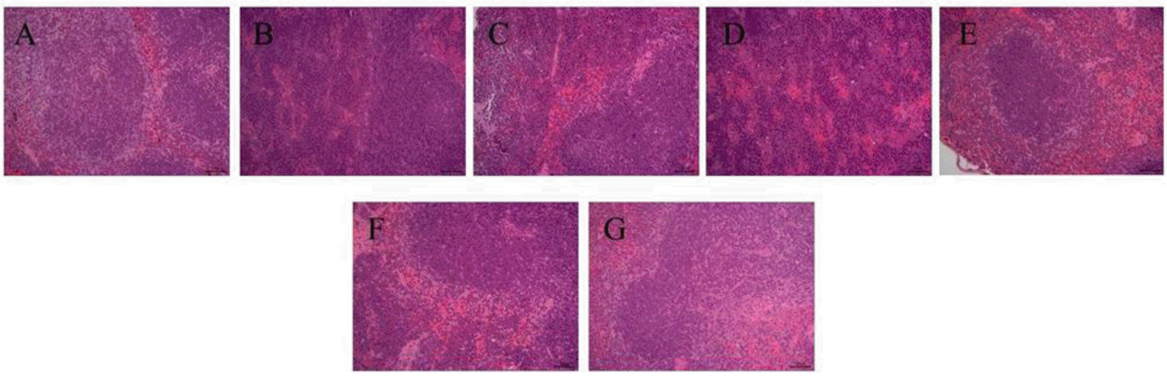


Fig. 24

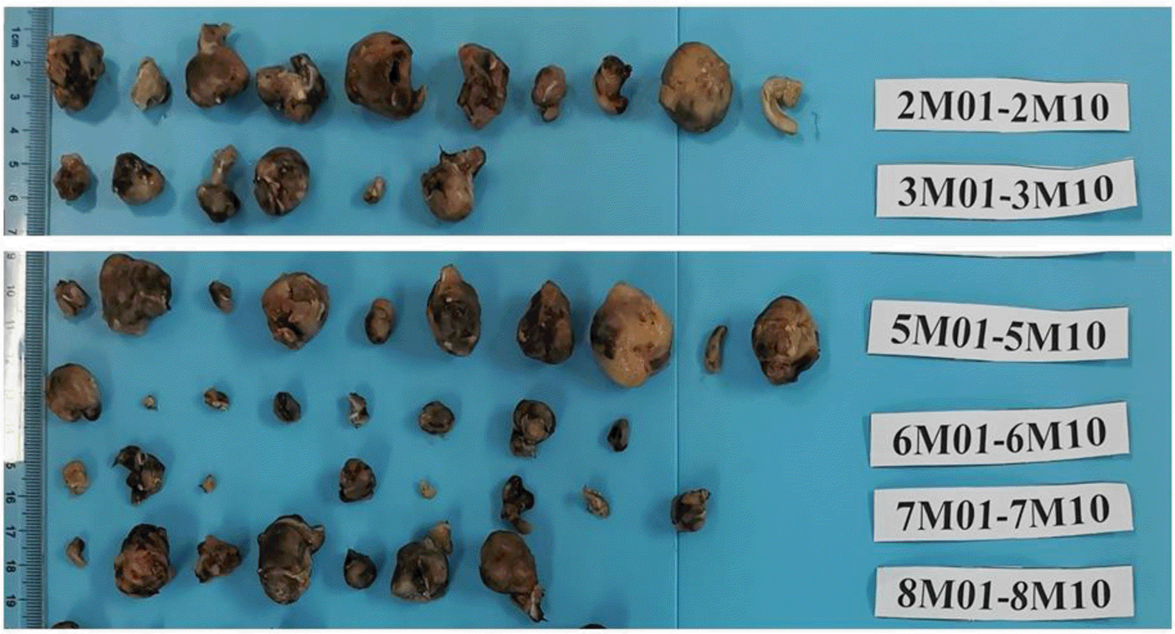


Fig. 25

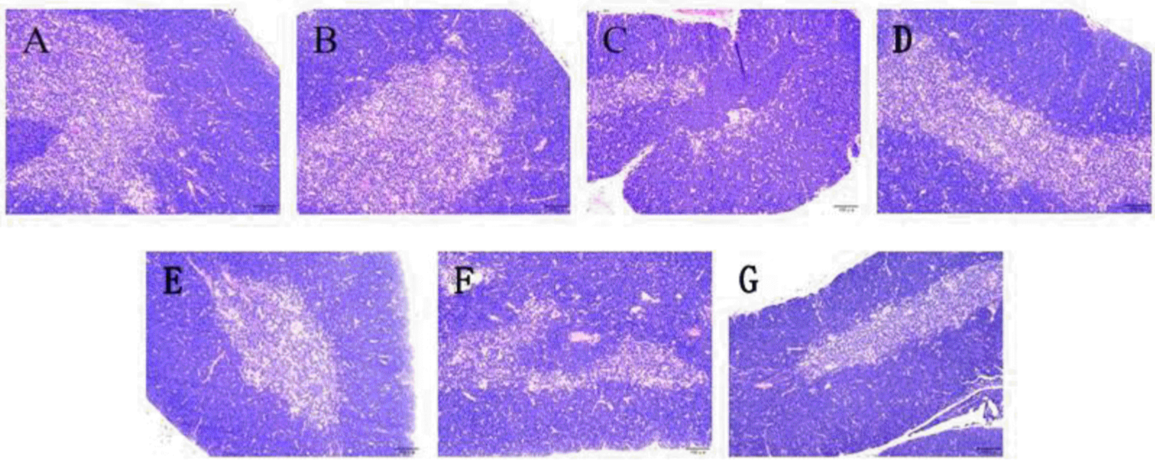


Fig. 26

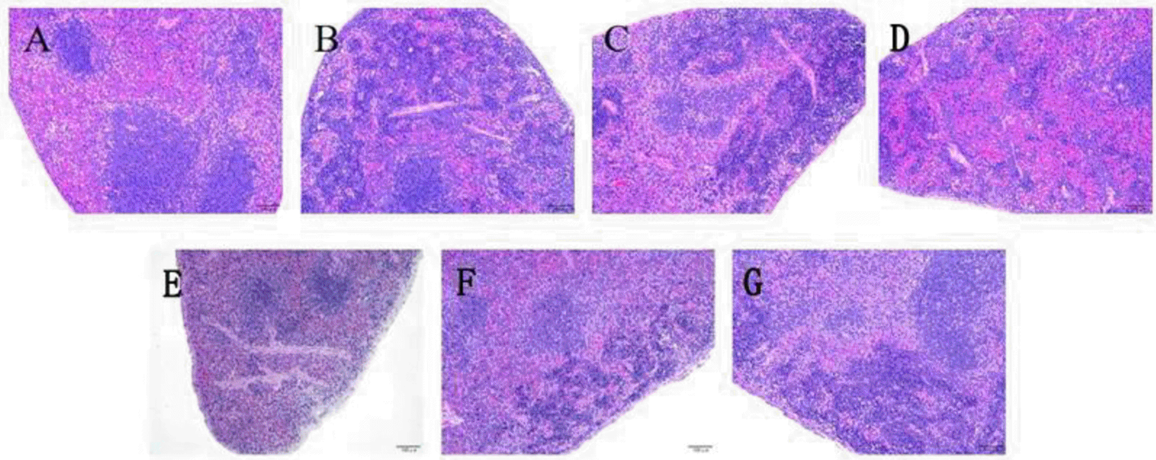


Fig. 27

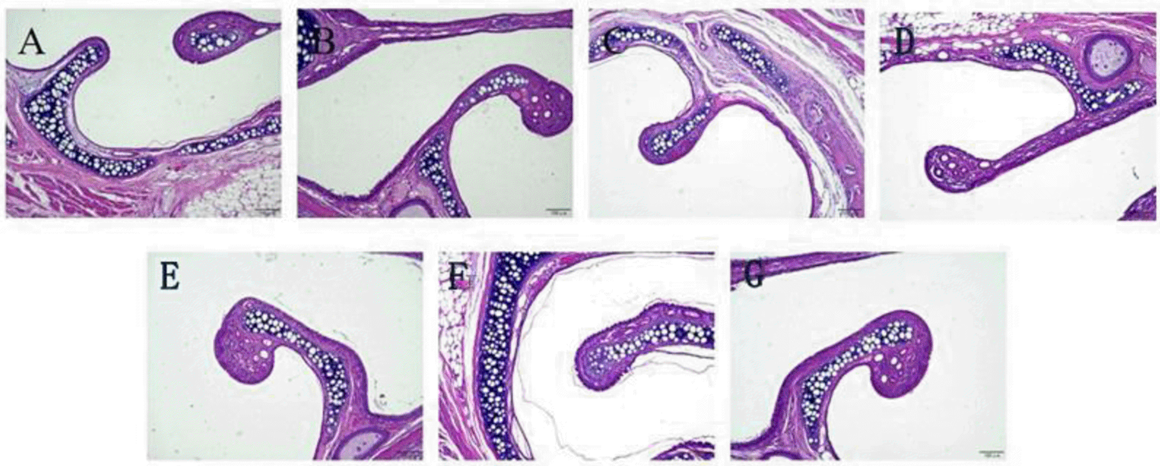


Fig. 28

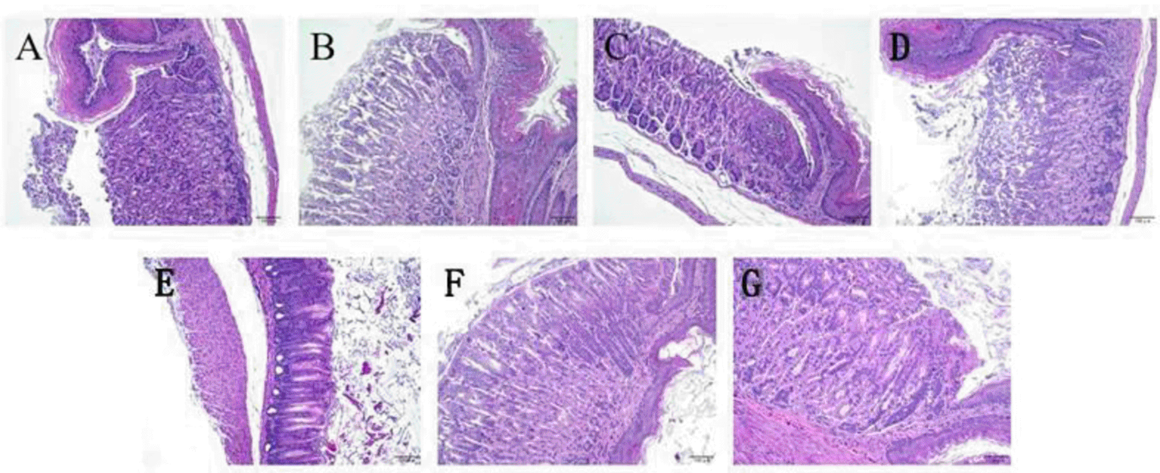


Fig. 29

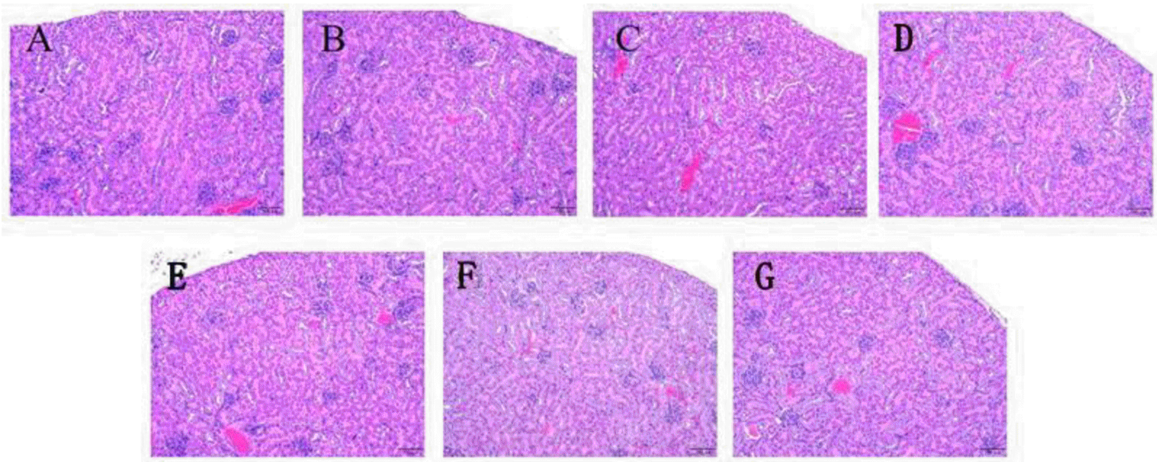


Fig. 30

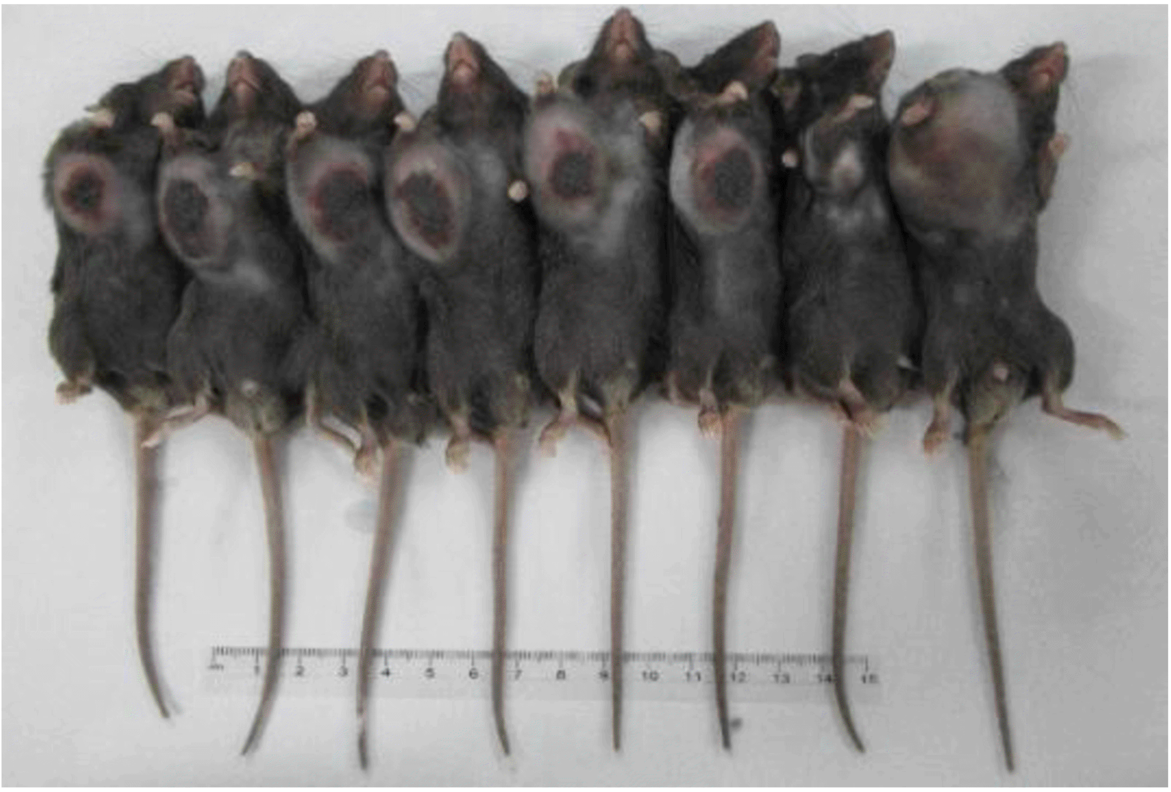

Fig. 31

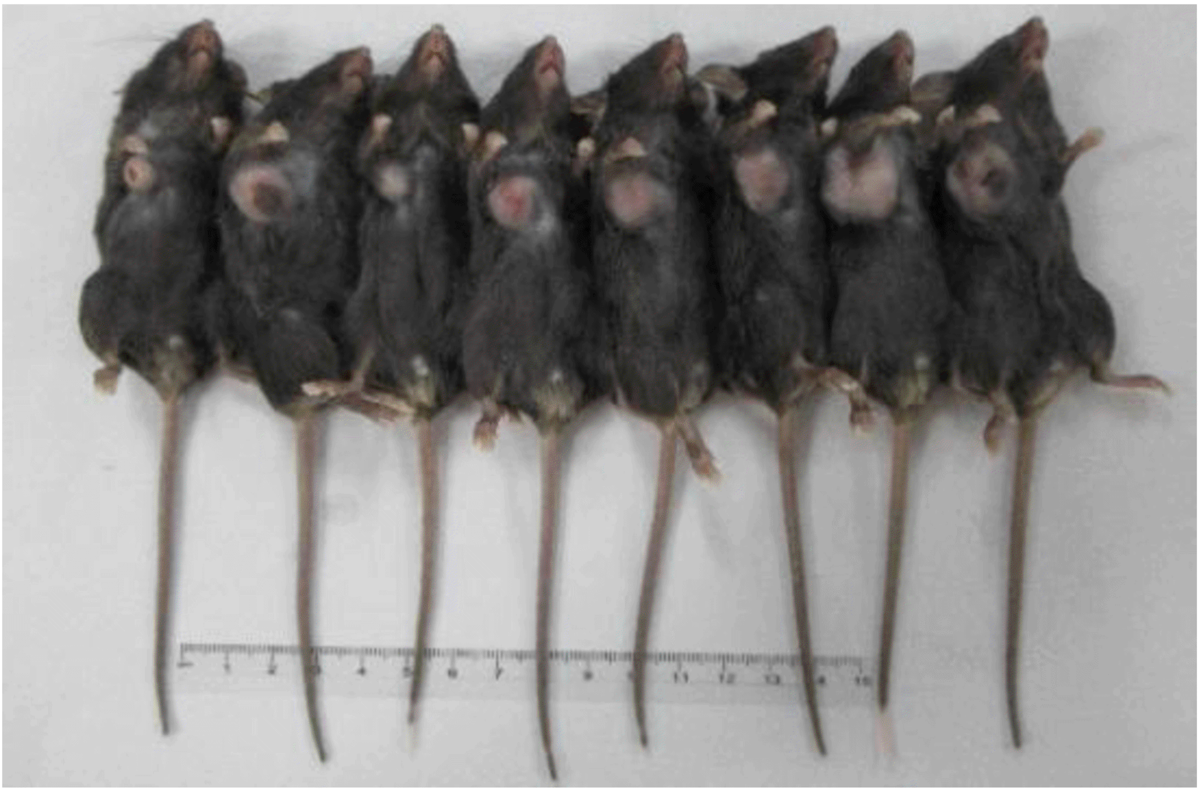

Fig. 30

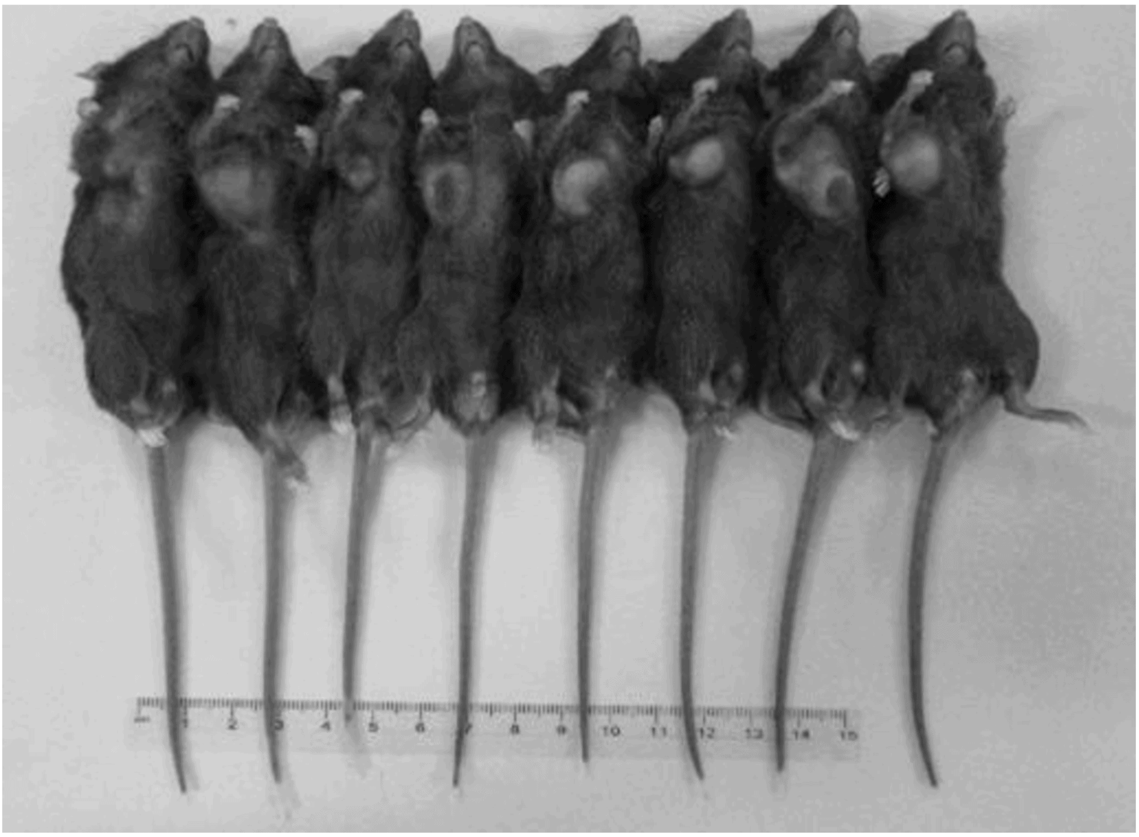

Fig. 31

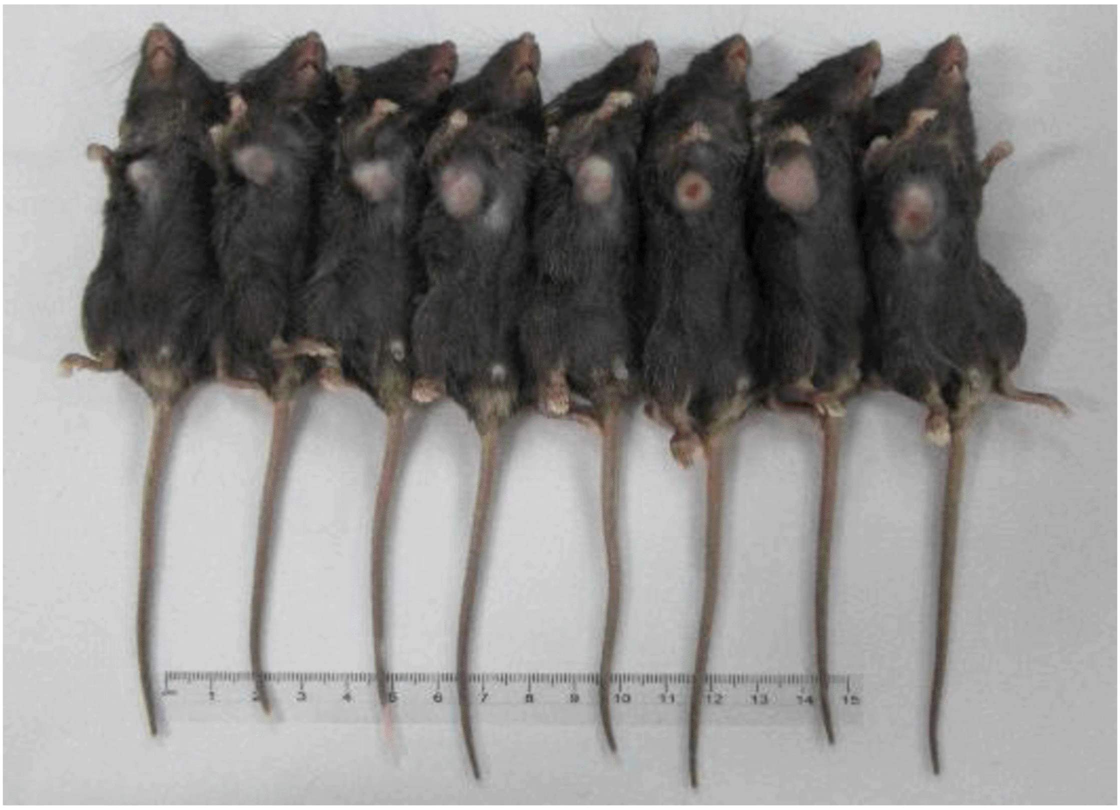

Fig. 32

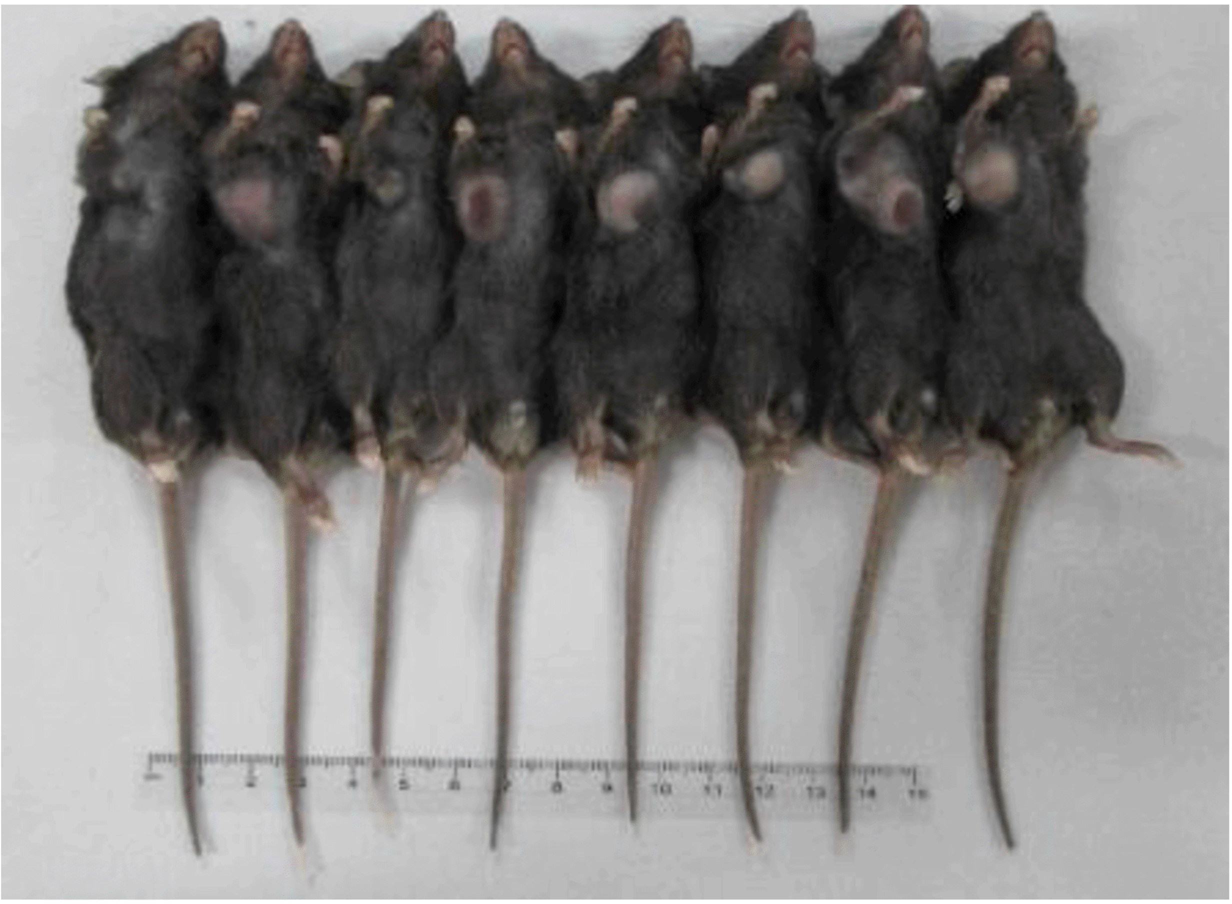

Fig. 33

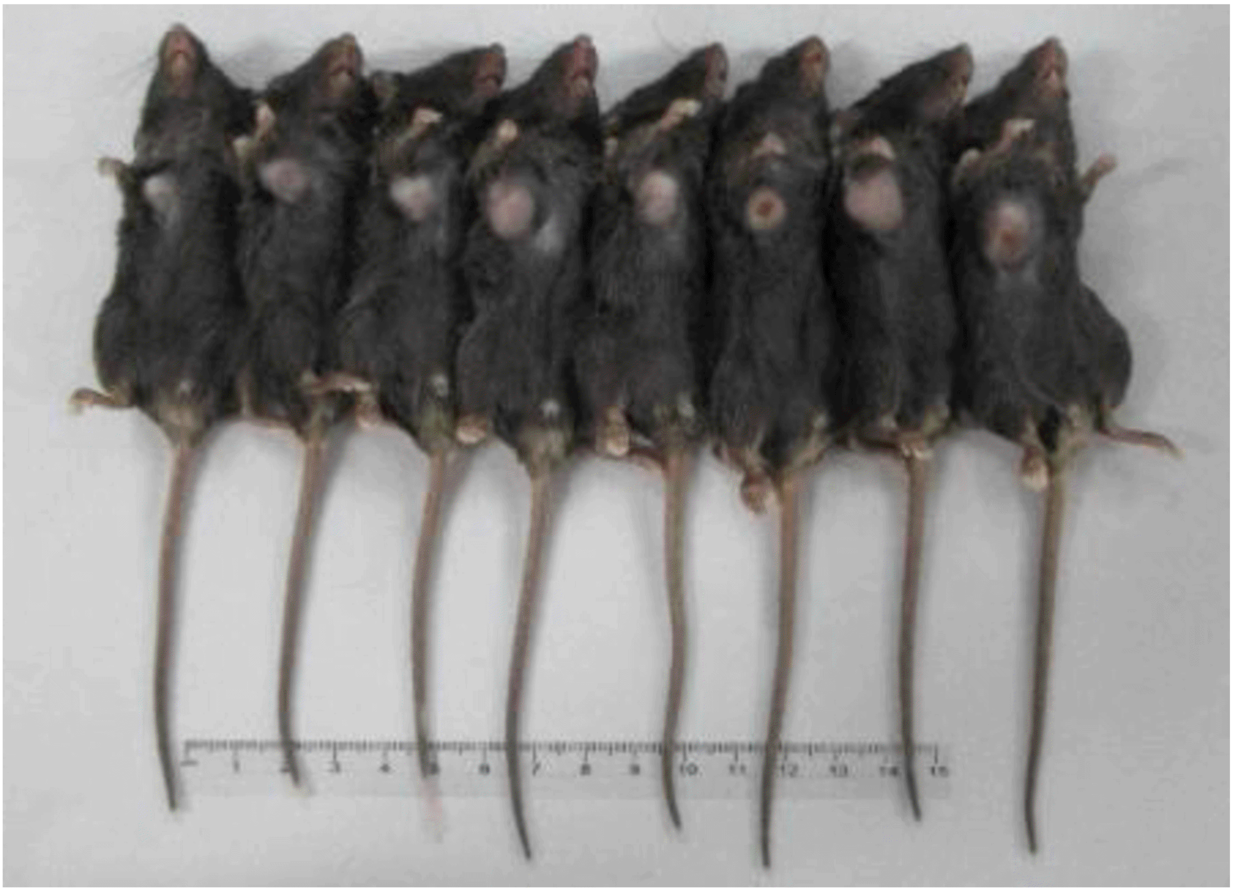

Fig. 34

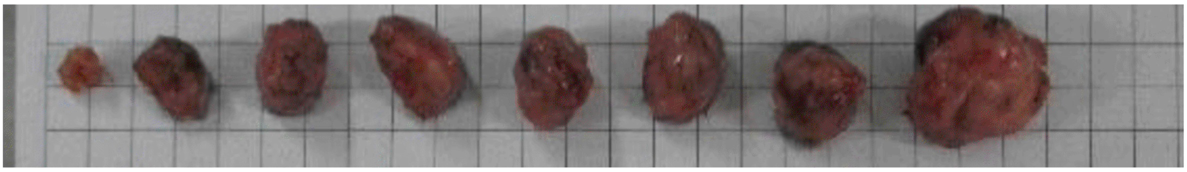

Fig. 35

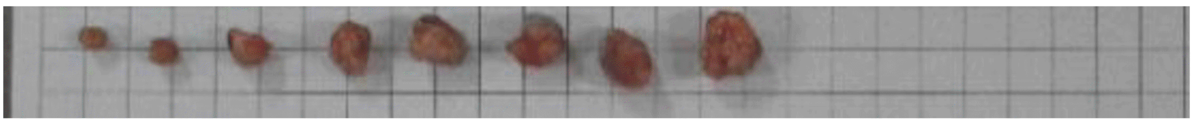

Fig. 36

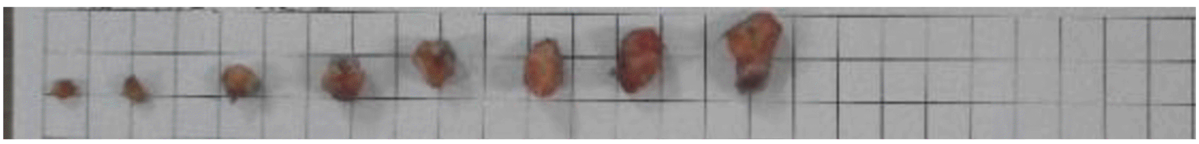

Fig. 37

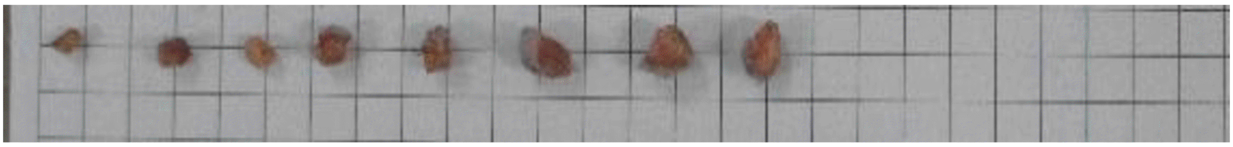

Fig. 38

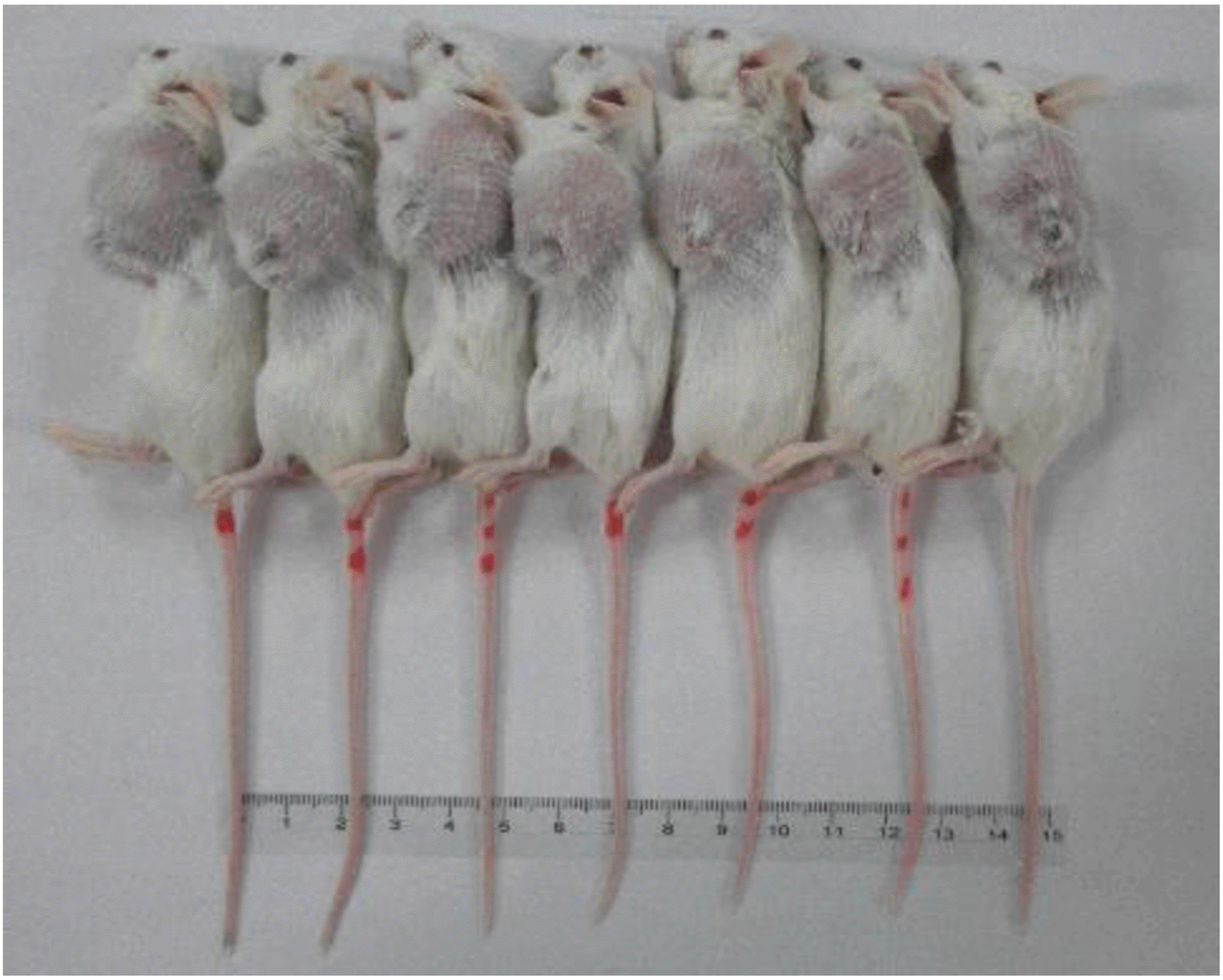

Fig. 39

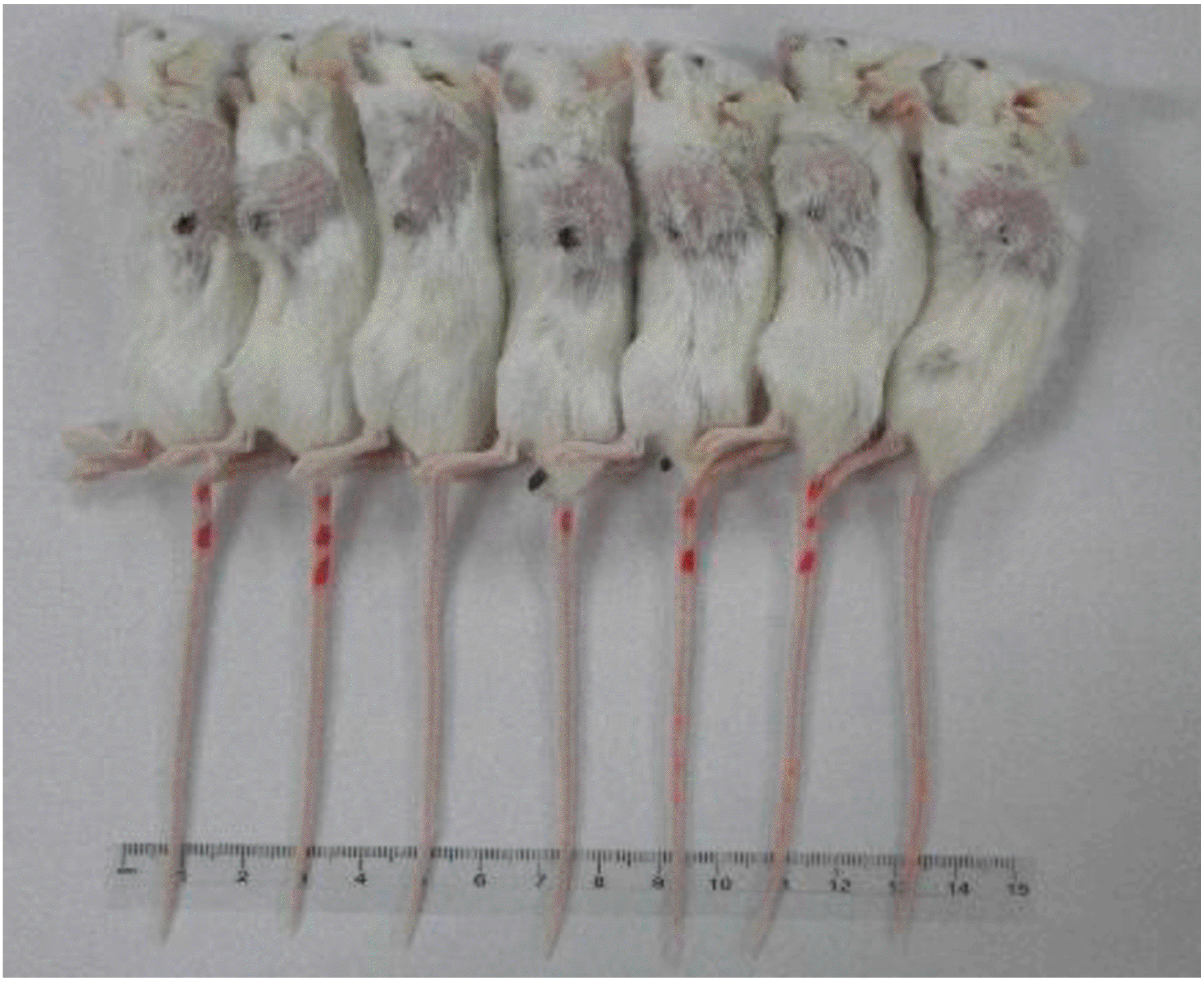

Fig. 40

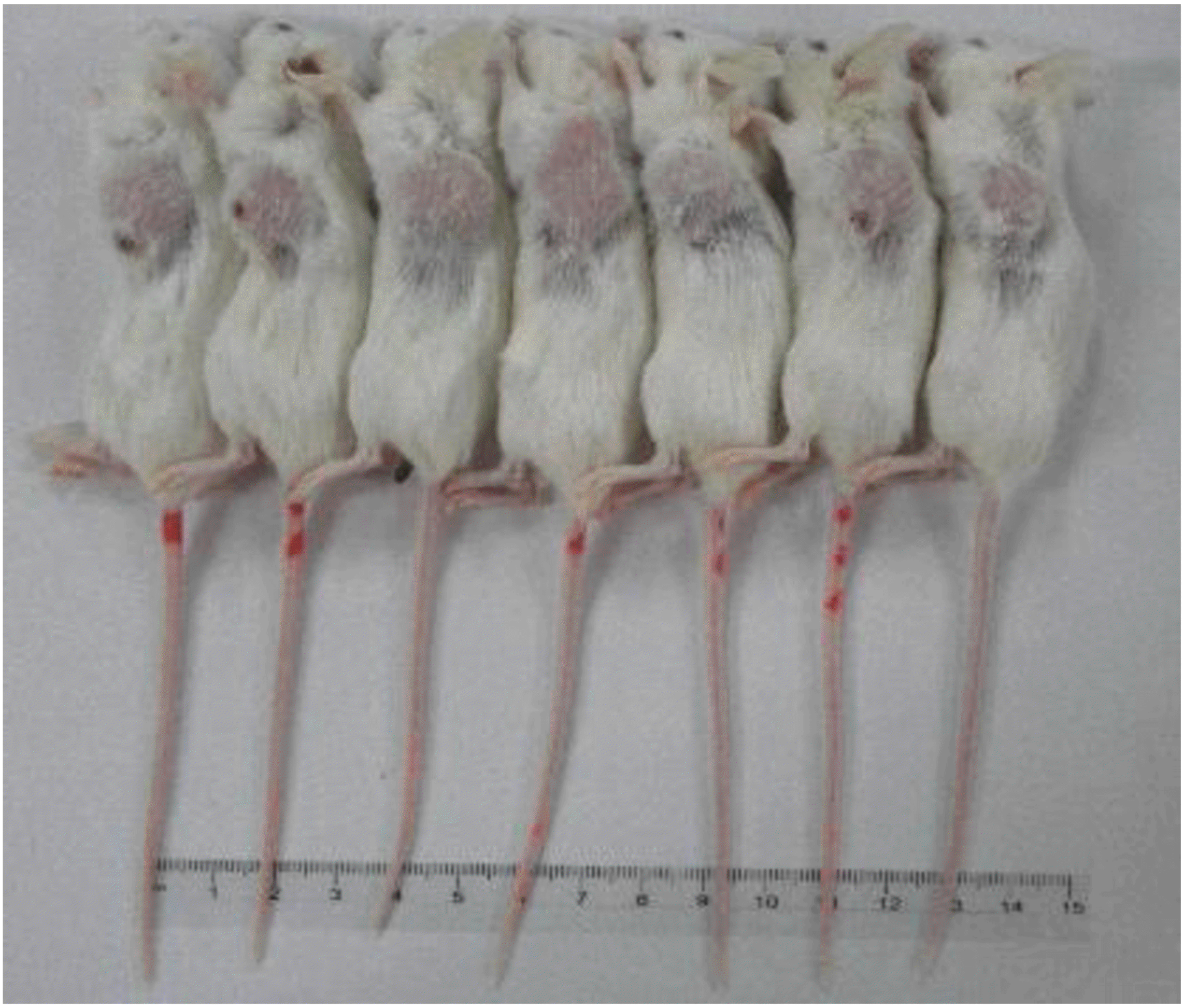


Fig. 41

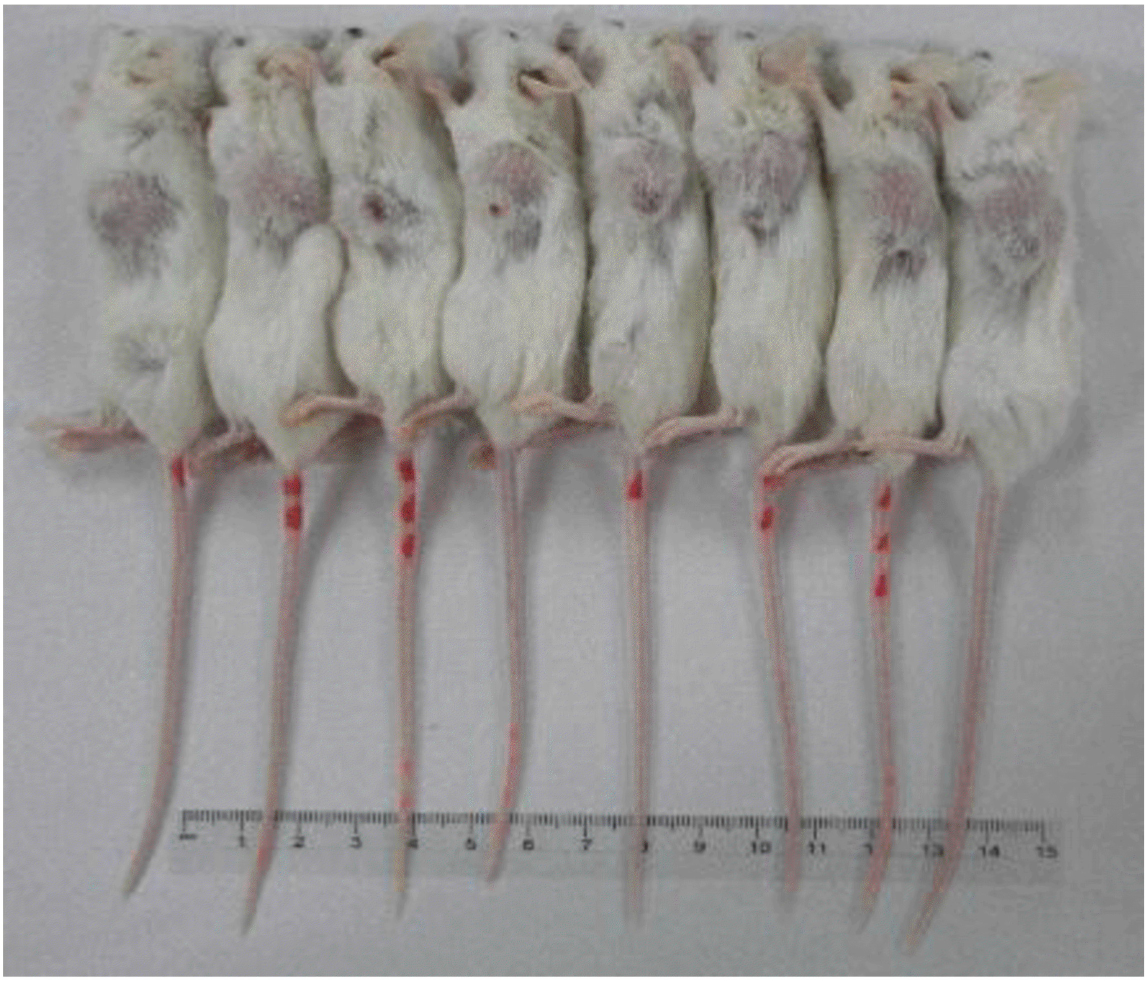

Fig. 42

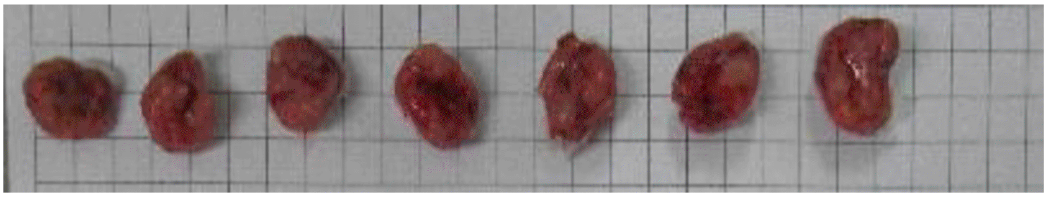

Fig. 43

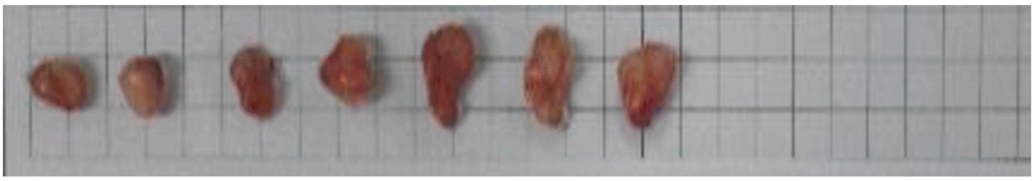

Fig. 44

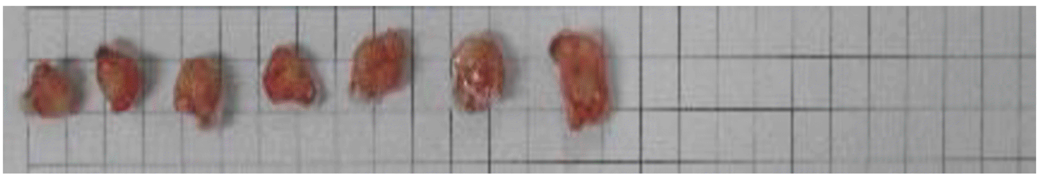

Fig. 45

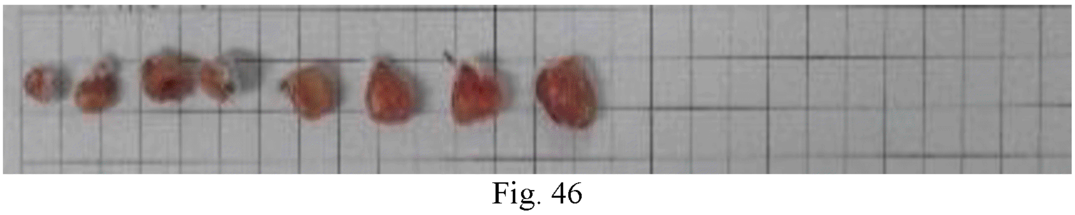

Fig. 46

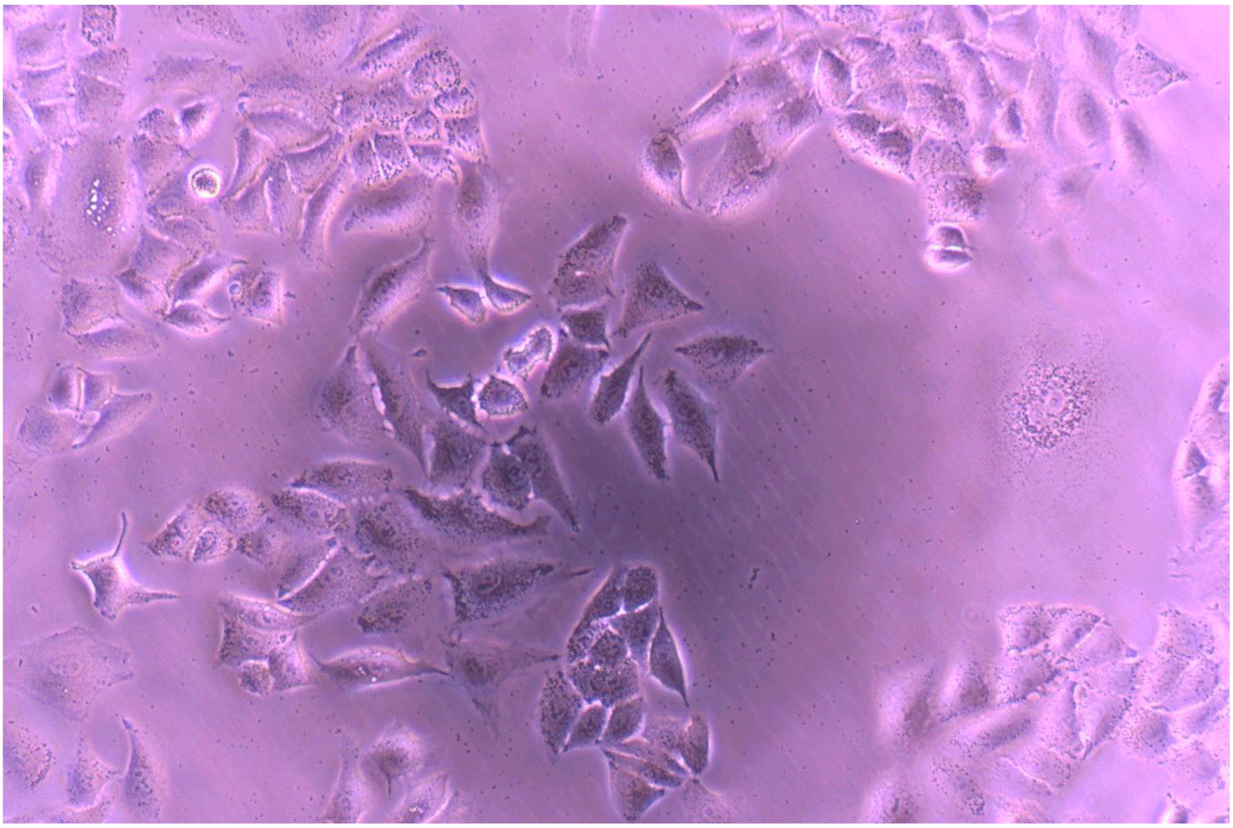

Fig. 47

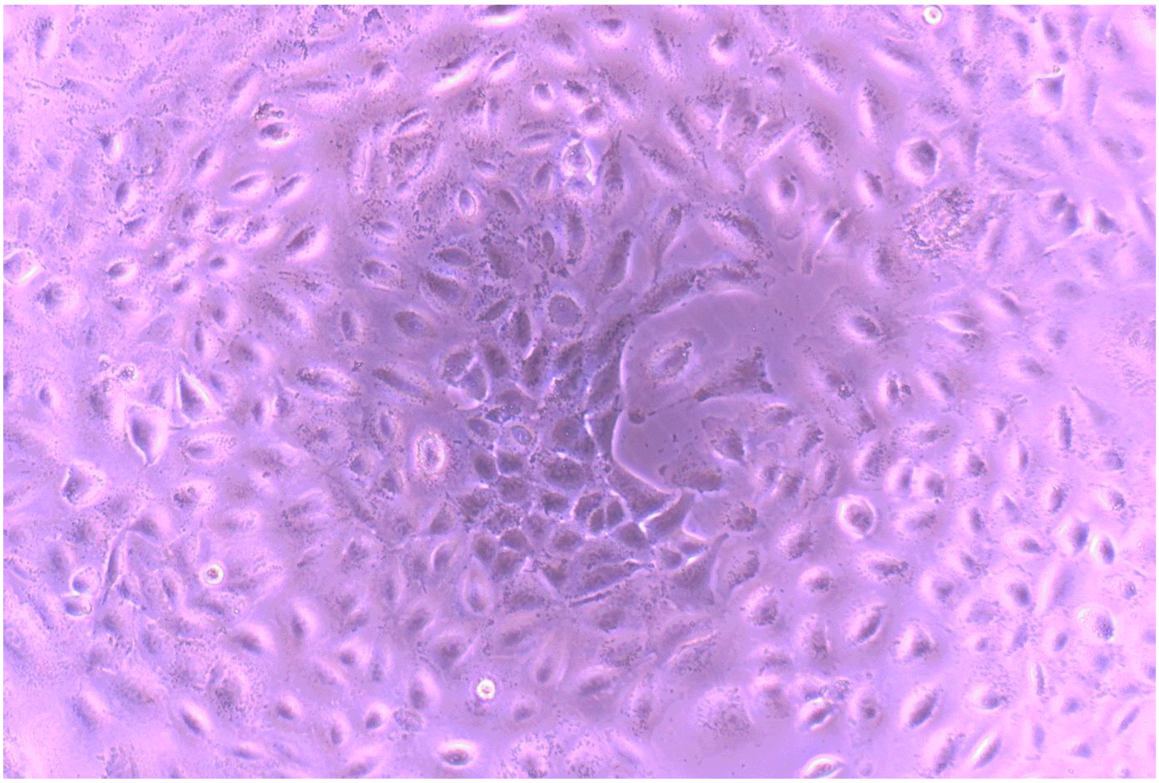

Fig. 48

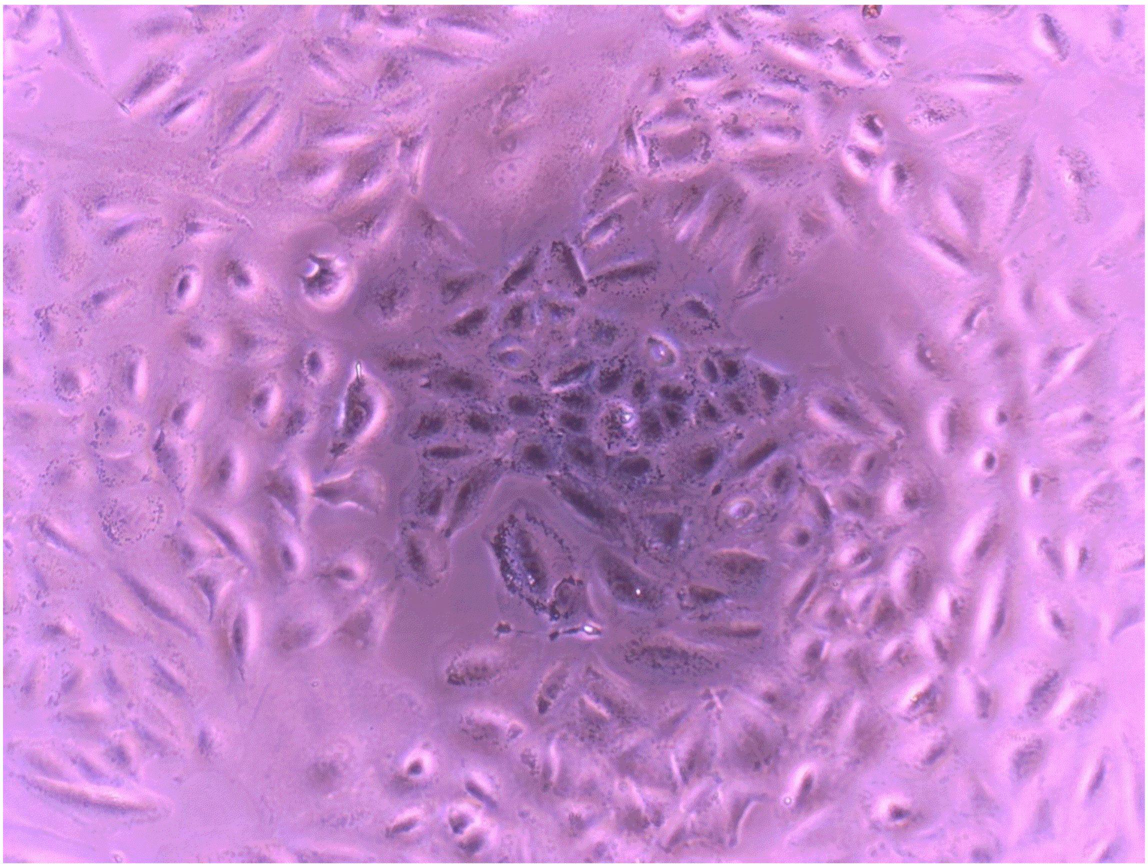

Fig. 49

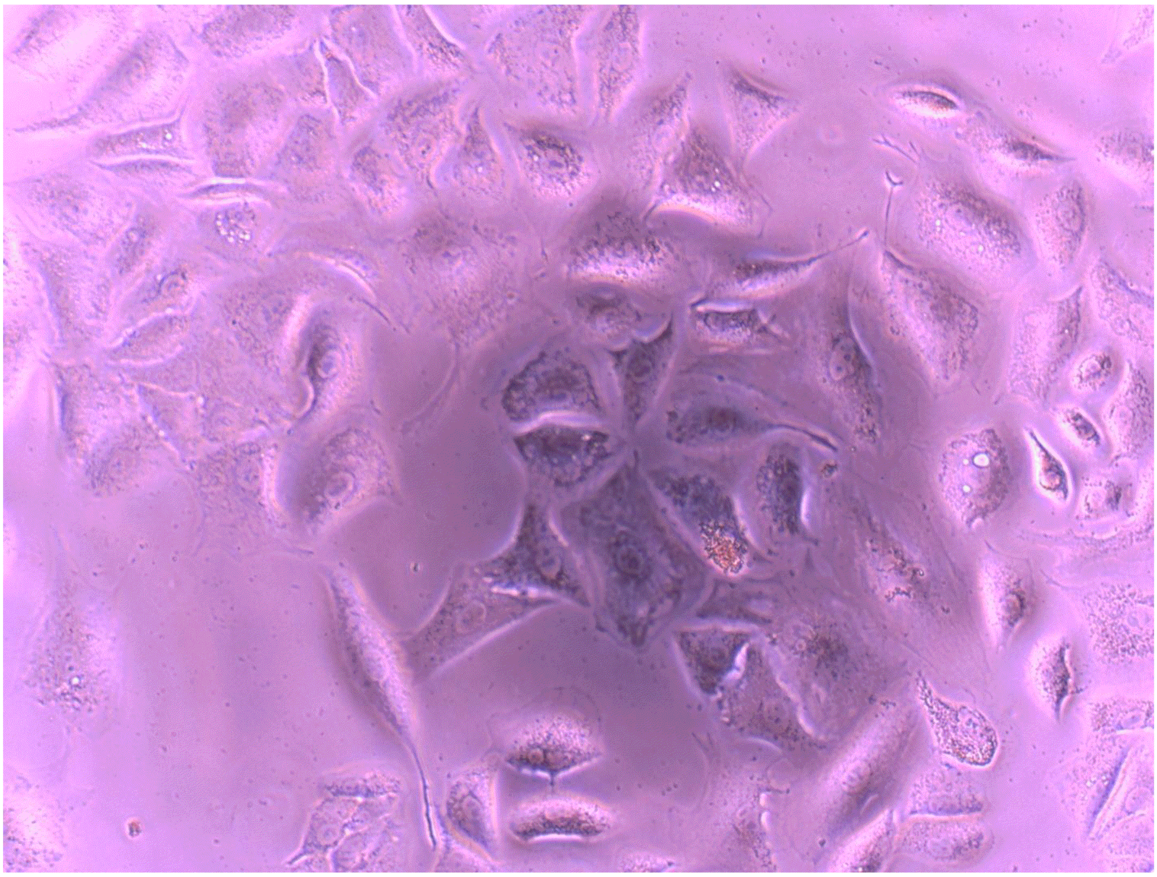

Fig. 50

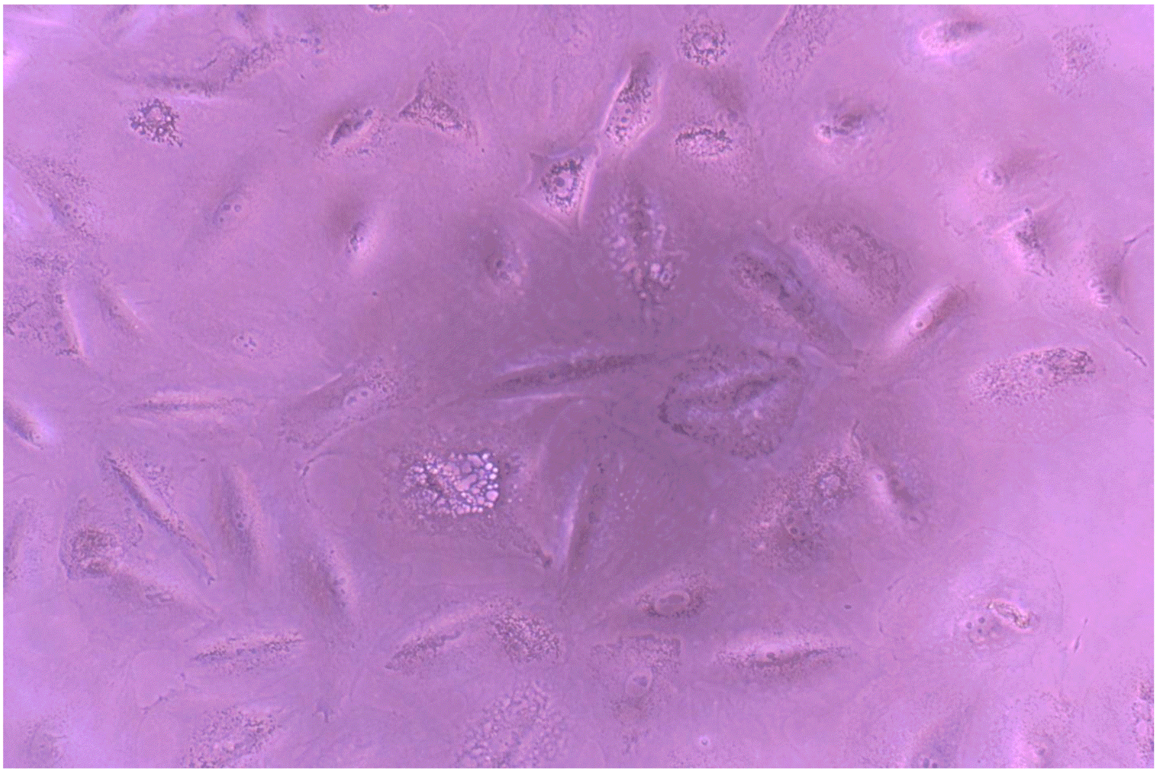

Fig. 51

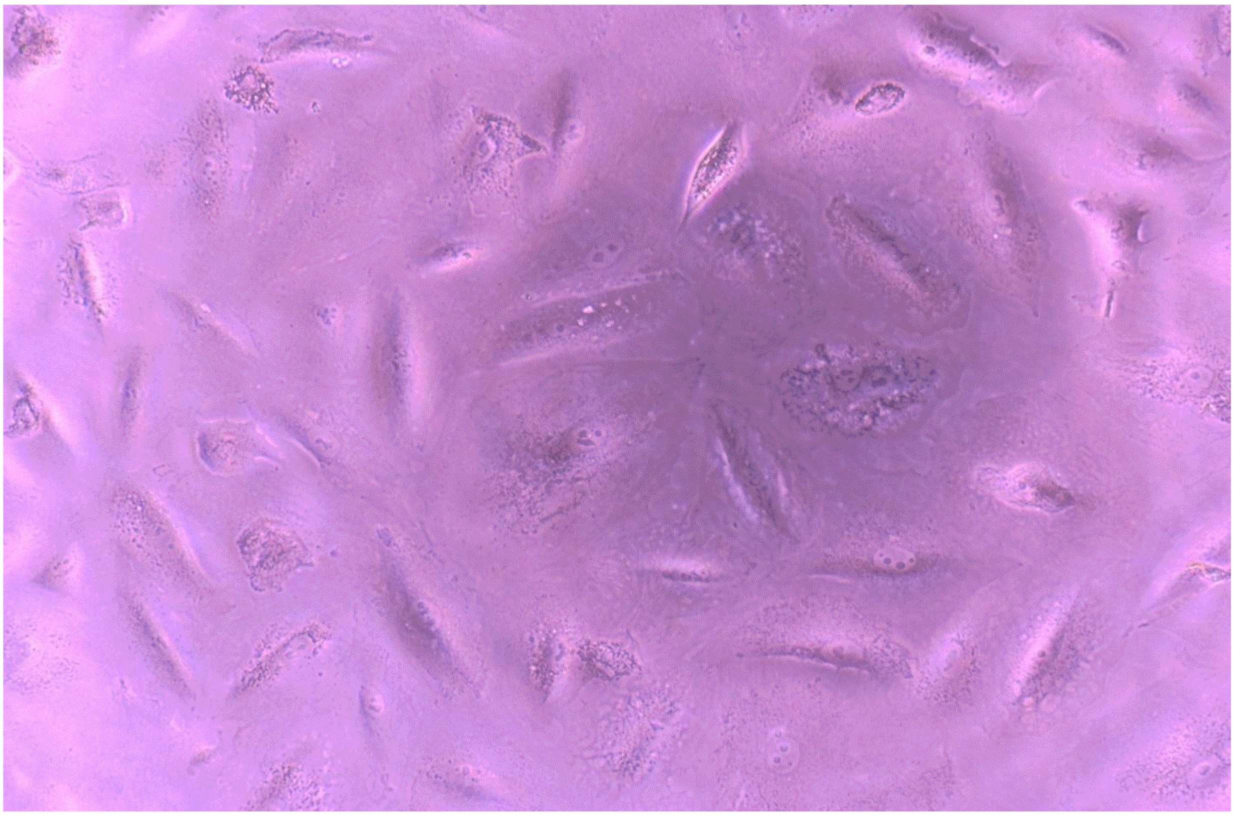

Fig. 52

**INTERNATIONAL SEARCH REPORT**

International application No. **PCT/CN2024/080443**

**A. CLASSIFICATION OF SUBJECT MATTER**

C08B37/00(2006.01)i； A61K31/715(2006.01)i； A61P35/00(2006.01)i

According to International Patent Classification (IPC) or to both national classification and IPC

**B. FIELDS SEARCHED**

Minimum documentation searched (classification system followed by classification symbols)

IPC:C08B A61K A61P

Documentation searched other than minimum documentation to the extent that such documents are included in the fields searched

Electronic data base consulted during the international search (name of data base and, where practicable, search terms used)

CNKI, 万方, WANFANG, CJFD, CNTXT, ENTXT, DWPI, STN, ISI Web of Science: 多糖, 高温, 高压, 灵芝, 柱层析, 色谱柱, 膜浓缩, 抗肿瘤, 癌, column, chromatography, ganoderma, lucidum, polysaccharide, pressure, high, temperature, membrane, concentration, cancer

**C. DOCUMENTS CONSIDERED TO BE RELEVANT**

| Category* | Citation of document, with indication, where appropriate, of the relevant passages | Relevant to claim No. |
|---|---|---|
| PX | CN 117003906 A (SHENZHEN YANDAI INVESTMENT CO., LTD.) 07 November 2023 (2023-11-07)<br>claims 1-10 | 1-10 |
| A | 操丽丽等 (CAO, Lili et al.). "高压热水提取灵芝多糖及对其抗氧化活性的影响 (Study on Extraction of Polysaccharides from Ganoderma Lucidum by Hot Compressed Water and Its Antioxidant Activities)"<br>*食品科学技术学报 (Journal of Food Science and Technology)*,<br>Vol. 36, No. 2, 17 April 2018 (2018-04-17), 58-62<br>section 1.5, and table 1 | 1-10 |
| A | CN 102617745 A (GUANGDONG INSTITUTE OF MICROBIOLOGY et al.) 01 August 2012 (2012-08-01)<br>entire document | 1-10 |
| A | CN 101367881 A (NANJING AGRICULTURAL UNIVERSITY) 18 February 2009 (2009-02-18)<br>entire document | 1-10 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **04 June 2024** | **07 June 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

INTERNATIONAL SEARCH REPORT

International application No.
PCT/CN2024/080443

C. DOCUMENTS CONSIDERED TO BE RELEVANT

| Category* | Citation of document, with indication, where appropriate, of the relevant passages | Relevant to claim No. |
|---|---|---|
| A | CN 101357951 A (NANFANG LEE KUM KEE CO., LTD. et al.) 04 February 2009 (2009-02-04)<br>entire document | 1-10 |
| A | CN 107857828 A (DAXINGANLING LINGOBERRY BOREAL BIOTECH CO., LTD.) 30 March 2018 (2018-03-30)<br>entire document | 1-10 |
| A | CN 111533823 A (GUANGZHOU YOROYAL BIOLOGICAL TECHNOLOGY CO., LTD.) 14 August 2020 (2020-08-14)<br>entire document | 1-10 |
| A | CN 108976314 A (OCEAN UNIVERSITY OF CHINA) 11 December 2018 (2018-12-11)<br>entire document | 1-10 |
| A | WO 2022062380 A1 (GUANGDONG INSTITUTE OF MICROBIOLOGY (GUANGDONG DETECTION CENTER OF MICROBIOLOGY) et al.) 31 March 2022 (2022-03-31)<br>entire document | 1-10 |

Form PCT/ISA/210 (second sheet) (July 2022)

**INTERNATIONAL SEARCH REPORT**
**Information on patent family members**

International application No.
**PCT/CN2024/080443**

| Patent document cited in search report | Publication date (day/month/year) | Patent family member(s) | Publication date (day/month/year) |
|---|---|---|---|
| CN 117003906 A | 07 November 2023 | None | |
| CN 102617745 A | 01 August 2012 | CN 102617745 B | 13 November 2023 |
| CN 101367881 A | 18 February 2009 | CN 101367881 B | 08 December 2010 |
| CN 101357951 A | 04 February 2009 | None | |
| CN 107857828 A | 30 March 2018 | None | |
| CN 111533823 A | 14 August 2020 | None | |
| CN 108976314 A | 11 December 2018 | None | |
| WO 2022062380 A1 | 31 March 2022 | CN 112094358 A | 18 December 2020 |
| | | CN 112094358 B | 27 May 2022 |

Form PCT/ISA/210 (patent family annex) (July 2022)